(19) Europäisches Patentamt
European Patent Office
Office européen des brevets

(11) EP 3 845 591 A1

(12) **EUROPEAN PATENT APPLICATION**
published in accordance with Art. 153(4) EPC

(43) Date of publication:
**07.07.2021 Bulletin 2021/27**

(21) Application number: **19854454.6**

(22) Date of filing: **29.08.2019**

(51) Int Cl.:
*C08L 21/00* (2006.01) *B60C 1/00* (2006.01)
*C08K 3/013* (2018.01) *C08K 3/04* (2006.01)
*C08K 5/3445* (2006.01) *C08L 9/00* (2006.01)

(86) International application number:
**PCT/JP2019/033950**

(87) International publication number:
**WO 2020/045575 (05.03.2020 Gazette 2020/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **30.08.2018 JP 2018160997**
**30.08.2018 JP 2018160999**
**25.12.2018 JP 2018240987**
**25.12.2018 JP 2018240988**
**28.02.2019 JP 2019035614**
**06.06.2019 JP 2019106156**
**08.08.2019 JP 2019146231**

(71) Applicant: **Otsuka Chemical Co., Ltd.**
**Osaka-shi, Osaka 540-0021 (JP)**

(72) Inventors:
- **AOYAGI, Seiichi**
**Tokushima-shi, Tokushima 771-0193 (JP)**
- **YOSHIDA, Akiyuki**
**Tokushima-shi, Tokushima 771-0193 (JP)**
- **NAKASHIMA, Shinya**
**Tokushima-shi, Tokushima 771-0193 (JP)**

(74) Representative: **Held, Stephan**
**Meissner Bolte Patentanwälte**
**Rechtsanwälte Partnerschaft mbB**
**Widenmayerstraße 47**
**80538 München (DE)**

(54) **RUBBER COMPOSITION, RUBBER MATERIAL, USE OF SAME AND ADDITIVE**

(57) The present invention provides a rubber composition that can exhibit excellent low heat build-up, tear strength, and durability; and a rubber composition having excellent low heat build-up, tear strength, and durability, by incorporating a compound represented by the following formula (1) or (2), or a salt of the compound.

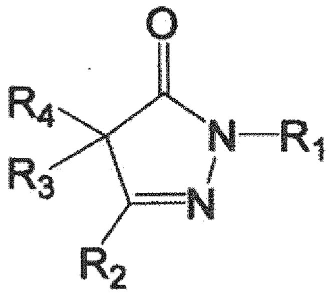 (1)

In the formula (1), $R_1$, $R_2$, $R_3$, and $R_4$ are the same or different, and each represents a hydrogen atom, an alkyl group, or the like; $R_3$ and $R_4$ may be taken together to form an alkylidene group, and any two of $R_2$, $R_3$, and $R_4$ may be taken together to form an alkylene group; and each of these groups may have one or more substituents.

EP 3 845 591 A1

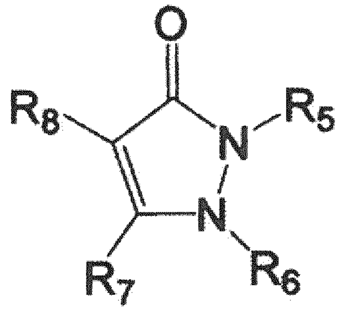

(2)

In the formula (2), $R_5$, $R_7$, and $R_8$ are the same or different, and each represents a hydrogen atom or an amino group; and $R_6$ represents an alkyl group or an aralkyl group; and each of these groups may have one or more substituents.

## Description

Technical Field

The present invention relates to a rubber composition, a rubber material, their use, and an additive.

Background Art

Recent perspectives on resource and energy conservation, as well as environmental protection, have led to strict regulations on emission gases, including carbon dioxide, and a highly increased demand for lower fuel consumption in automobiles. While the drive systems such as engines, as well as transmission systems, greatly contribute to lower fuel consumption, rolling resistance of tires is also largely involved in lower fuel consumption. Accordingly, for lower fuel consumption, improving rolling resistance of tires is required in addition to the drive systems and transmission systems.

In addition to the above, excellent tear strength, wet grip performance, durability, and processability of the rubber composition are desired.

Although Patent Literature 1 to 7 have been proposed to address the above objects, they are not sufficient.

In various types of vehicles, such as automobiles, vibration-proof rubber has been used for the components of the engine (e.g., engine mounts and torsion dampers), which is the main source of vibration and noise, to absorb vibration during engine operation, and to inhibit the intrusion of vibration and noise into the cabin.

Patent Literature 8 suggests a rubber composition for vibration-proof rubber, comprising an ethylene-α-olefin copolymer rubber as an essential rubber component, and a compound containing an organic peroxide and a hydrazine derivative. However, although the use of an organic peroxide greatly improves heat resistance, fatigue resistance is reduced, and so satisfactory vibration-proof performance required for vibration-proof rubber and satisfactory durability are not always attained.

When a compound is added for the purpose of improving the functionality of a rubber material, the processability of the rubber material is generally reduced.

Thus, there is a trade-off between low fuel consumption and processability, and a rubber material having excellent low fuel consumption and processability is desired.

Citation List

Patent Literature

PTL 1: JP2015-086318A

PTL 2: WO2012/031183

PTL 3: JP2018-062629A

PTL 4: JP2011-052146A

PTL 5: JP2017-75245A

PTL 6: JP2015-28113A

PTL 7: JP2015-17160A

PTL 8: JP2007-131806A

Summary of Invention

Technical Problem

An object of the present invention is to provide a rubber composition that can exhibit low heat build-up.

Another object of the present invention is to provide a rubber composition that can exhibit high tear strength.

Another object of the present invention is to provide a rubber composition that has excellent processability.

Another object of the present invention is to provide a tire that has excellent low heat build-up.

Another object of the present invention is to provide a tire that has excellent tear strength.

Another object of the present invention is to provide a hose that has excellent low heat build-up, tear strength, and durability.

Another object of the present invention is to provide an additive for imparting low heat build-up to a rubber component.

Another object of the present invention is to provide an additive for imparting tear strength to a rubber component.

Another object of the present invention is to provide a rubber material that has sufficient processability and excellent low heat built-up although it includes an additive.

Another object of the present invention is to provide a rubber composition that contains carbon black and/or an inorganic filler, and that has sufficient processability and excellent low heat build-up although it includes an additive.

Another object of the present invention is to provide a tire that has sufficient processability and excellent low heat build-up although it includes an additive.

Another object of the present invention is to provide a hose, a belt (conveyor belt), and vibration-proof rubber, each having excellent processability.

Solution to Problem

To achieve the above objects, the present inventors carried out extensive research. As a result, the inventors found that a specific pyrazolone-based compound can impart low heat build-up to a rubber component. Based on this finding, the inventors continued further research and have accomplished the present invention.

To achieve the above objects, the present inventors further carried out extensive research. As a result, the inventors found that a specific pyrazolone-based compound can impart excellent tear strength to a rubber component. Based on this finding, the inventors continued further research and have accomplished the present invention.

To achieve the above objects, the present inventors further carried out extensive research. As a result, the inventors found that the wet grip performance of tires to be obtained is improved by incorporating a specific pyrazolone-based compound into a silica-containing rubber composition.

To achieve the above objects, the present inventors further carried out extensive research. As a result, the inventors found that incorporating a specific pyrazolone-based compound into a rubber composition containing sulfur in a specific amount can improve the wet grip performance of tires to be obtained. The inventors also found that the durability is also improved.

To achieve the above objects, the present inventors further carried out extensive research. As a result, the inventors found that a tire obtained from a second rubber composition that is obtained by further adding a vulcanizing agent to a first rubber composition comprising a specific pyrazolone-based compound, an antioxidant, and a vulcanizing agent in an amount of 0 to 1 part by mass, per 100 parts by mass of the rubber component, has improved tear strength performance, and that such a rubber composition has improved processability.

To achieve the above objects, the present inventors further carried out extensive research. As a result, the inventors found that the durability of the rubber composition is maintained and vibration-proof performance can be obtained by incorporating diene rubber, a pyrazolone-based compound, carbon black, and a specific cross-linking agent. Based on this finding, the inventors continued further research and have accomplished the present invention.

To achieve the above objects, the present inventors further carried out extensive research. As a result, the inventors found that a rubber material that can be obtained by incorporating a compound having a predetermined structure to a rubber component has not only sufficient processability and excellent low heat build-up. Based on this finding, the inventors continued further research and have accomplished the present invention.

Specifically, the present invention provides the following rubber compositions, rubber materials, methods for producing rubber compositions, tires, vibration-proof rubber, belts (conveyor belts), or additives for imparting low heat built-up to hose rubber components, and additives for imparting high tear strength to rubber components.

Item 1 A rubber composition comprising the following components (a1), (b1), and (c1) :

component (a1): a rubber component;
component (b1): a compound represented by the following formula (1) or (2) , or a salt of the compound; and
component (c1): carbon black and/or an inorganic filler;

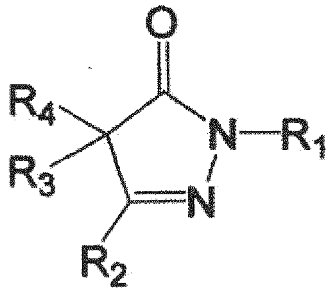

(1)

wherein
$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

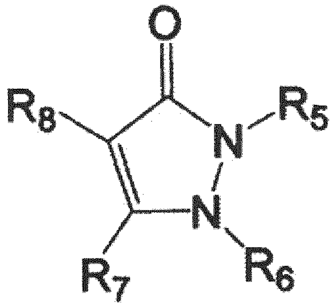

(2)

wherein
$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Item 2. The rubber composition according to Item 1, which is a compound in which in formula (1) above, $R_1$, $R_3$, and $R_4$ are the same or different, and each represents a hydrogen atom, a $C_{1-4}$ linear or branched alkyl group, an aralkyl group, an aryl group, or a heterocyclic group; $R_2$ represents a hydrogen atom, a $C_{1-18}$ linear or branched alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or a compound in which in formula (2) , $R_5$ represents a hydrogen atom; $R_6$ represents a $C_{1-4}$ linear alkyl group, an aralkyl group, or an aryl group; $R_7$ and $R_8$ are the same or different, and each represents a hydrogen atom, an amino group, an alkyl group, an aralkyl group, an aryl group, or a heterocyclic group; and each of these groups may have one or more substituents.

Item 3. The rubber composition according to Item 1 or 2, which is a compound in which in the formula (1), $R_1$, $R_3$, and $R_4$ all represent hydrogen atoms; and $R_2$ represents a hydrogen atom, a $C_{1-4}$ linear alkyl group, a phenyl group, a naphthyl group, or a furyl group.

Item 4. The rubber composition according to any one of Items 1 to 3, wherein the compound represented by the formula (1) is 5-pyrazolone, 3-methyl-5-pyrazolone, 3-(naphthalene-2-yl)-1H-pyrazol-5 (4H) -one, 3- (furan-2-yl)-1H-pyrazol-5(4H)-one, 3-phenyl-1H-pyrazol-5(4H)-one, or 3-propyl-1H-pyrazol-5(4H)-one.

Item 5. The rubber composition according to any one of Items 1 to 4, wherein the component (a1) is diene rubber.

Item 6. The rubber composition according to any one of Items 1 to 5, wherein the component (a1) is at least one member selected from the group consisting of natural rubber, isoprene rubber, styrene-butadiene copolymer rubber, and butadiene rubber.

Item 7. The rubber composition according to any one of Items 1 to 6, wherein the component (b1) is present in an

amount of 0.1 to 50 parts by mass, and the carbon black and/or the inorganic filler is present in an amount of 2 to 200 parts by mass, per 100 parts by mass of the component (a1).

Item 8. The rubber composition according to Items 1 to 7, which is used for tires.

Item 9. The rubber composition for tires according to Item 8, which is used for at least one member selected from the group consisting of tread, sidewall, bead area, belt, carcass, and shoulder portions.

Item 10. A tire produced using the rubber composition for tires according to Item 8 or 9.

Item 11. A method for producing a rubber composition, comprising step (A1) of mixing raw material ingredients including a rubber component, a compound represented by the formula (1) or (2) or a salt of the compound, and carbon black and/or an inorganic filler; and step (B1) of mixing the mixture obtained in step (A1) and a vulcanizing agent.

Item 12. The production method according to Item 11, wherein step (A1) comprises step (A1-1) of mixing the rubber component, and the compound represented by the formula (1) or (2) or a salt of the compound, and step (A1-2) of mixing the mixture obtained in step (A1-1) and the carbon black and/or the inorganic filler.

Item 13. A rubber composition comprising the following components (a2), (b2), and (c2):

component (a2): a rubber component;
component (b2): at least one compound selected from the group consisting of a compound represented by the formula (1), a compound represented by the formula (2), and salts of these compounds; and
component (c2): silica;

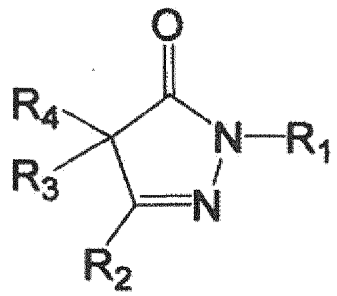

(1)

wherein
$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group; $R_2$, $R_3$, and $R_4$ may be taken together to form an alkylene group; and each of these groups may have one or more substituents; and

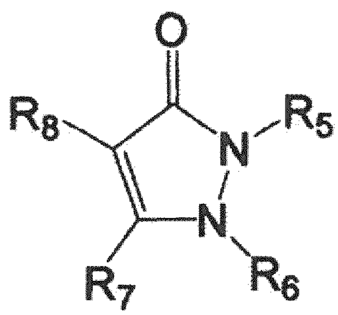

(2)

wherein
$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Item 14. The rubber composition according to Item 13, wherein the component (b2) is a compound represented by the formula (1).

Item 15. The rubber composition according to Item 13 or 14, wherein the component (c2) is present in an amount of 5 to 120 parts by mass, per 100 parts by mass of the component (a2).

Item 16. The rubber composition according to any one of Items 13 to 15, further comprising component (d2), a hydrocarbon polymer.

Item 17. A tire produced using the rubber composition according to any one of Items 13 to 16.

Item 18. A method for producing a rubber composition, comprising step (A2) of mixing the raw material ingredients including the components (a2), (b2), and (c2).

Item 19. The method for producing a rubber composition according to Item 18, further comprising mixing the component (d2) in step (A2) .

Item 20. The production method according to Item 18, wherein step (A2) comprises step (A2-1) of mixing the components (a2) and (b2), and step (A2-2) of mixing the mixture obtained in step (A2-1) and the component (c2).

Item 21. The method for producing a rubber composition according to Item 20, comprising further mixing the component (d2) in step (A2-1).

Item 22. A rubber composition for tires, comprising the following components (a3), (b3), (c3), and (d3):

component (a3): a rubber component;
component (b3): at least one compound selected from the group consisting of a compound represented by the formula (1), a compound represented by the formula (2), and salts of these compounds;
component (c3): carbon black; and
component (d3): sulfur;

wherein
the component (d3) is present in an amount of 0.8 to 2.2 parts by mass, per 100 parts by mass of the component (a3),

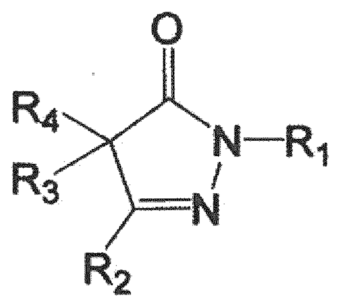 (1)

wherein
$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

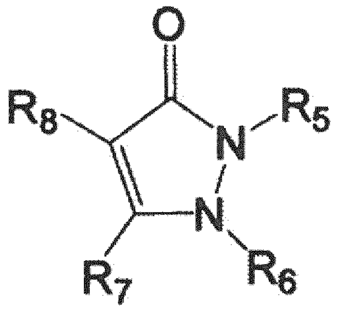

(2)

wherein
$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Item 23. The rubber composition for tires according to Item 22, wherein the component (b3) is the compound represented by the formula (1).

Item 24. The rubber composition for tires according to Item 22 or 23, wherein the component (c3) is present in an amount of 20 to 100 parts by mass, per 100 parts by mass of the component (a3) .

Item 25. The rubber composition for tires according to any one of Items 22 to 24, wherein in the component (a3), the content of natural rubber in 100 mass% of the rubber component is 65 to 100 mass%.

Item 26. The rubber composition for tires according to any one of Items 22 to 25, further comprising component (e3), a vulcanization accelerator in an amount of 1.0 to 3.0 parts by mass, per 100 parts by mass of the component (a3).

Item 27. The rubber composition for tires according to any of Items 22 to 26, wherein the component (e3) is N-cyclohexyl-2-benzothiazolysulfenamide.

Item 28. A tire produced using the rubber composition for tires according to any one of Items 22 to 27.

Item 29. A method for producing a rubber composition, comprising step (A3) of mixing the raw material ingredients including the components (a3), (b3), and (c3), and step (B3) of mixing the mixture obtained in step (A3) and the component (d3).

Item 30. The method for producing a rubber composition according to Item 29, further comprising mixing the component (e3) in step (B3) .

Item 31. The production method according to Item 29, wherein step (A3) includes step (A3-1) of mixing the components (a3) and (b3), and step (A3-2) of mixing the mixture obtained in step (A3-1) and the component (c3).

Item 32. A first rubber composition comprising the following components (a4), (b4), and (c4), wherein a vulcanizing agent is present in an amount of 0 to 1 part by mass, per 100 parts by mass of the component (a4);
(a4): a rubber component;
(b4): at least one compound selected from the group consisting of a compound represented by the formula (1), a compound represented by the formula (2), and salts of the compounds; and (c4) an antioxidant;

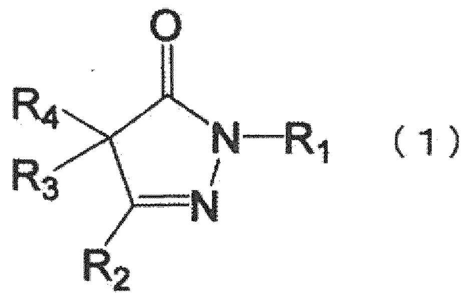

wherein

$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

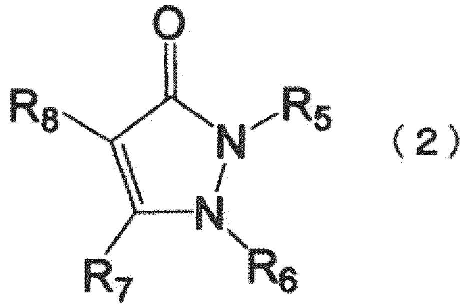

wherein

Rs, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents. Item 33. The first rubber composition according to Item 32, wherein the component (b4) is the compound represented by the formula (1).

Item 34. The first rubber composition according to Item 32 or 33, wherein the component (c4) is present in an amount of 0.1 to 10 parts by mass, per 100 parts by mass of the component (a4).

Item 35. A second rubber composition obtained by adding a vulcanizing agent to the first rubber composition according to any one of Items 32 to 34.

Item 36. A tire produced using the second rubber composition according to Item 35.

Item 37. A method for producing the first rubber composition according to any one of Items 32 to 34, comprising step (A4) of mixing the components (a4), (b4), and (c4).

Item 38. A method for producing a second rubber composition, comprising step (B4) of mixing a vulcanizing agent and the first rubber composition produced by the method according to Item 37.

Item 39. A method for producing a second rubber composition, comprising step (A4) of mixing the components (a4), (b4), and (c4), and step (B4) of mixing the first rubber composition obtained from step (A4) and a vulcanizing agent.

Item 40. A rubber composition for vibration-proof rubber, comprising the following components (a5), (b5), (c5), and (d5): component (a5): diene rubber;

component (b5): a pyrazolone compound represented by the following formula (1) or (2);

component (c5): carbon black; and

components (d5): a cross-linking agent;

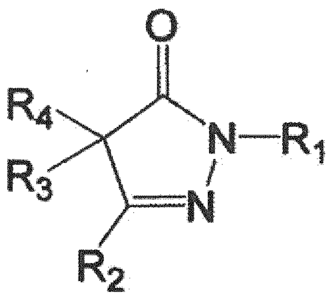

(1)

wherein

$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group; any two of $R_2$, $R_3$, and $R_4$ may be taken together to form an alkylene group; and each of these groups may have one or more substituents; or

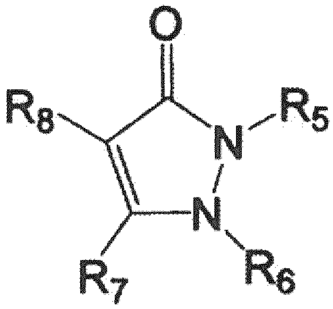

(2)

wherein

$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Item 41. The composition according to Item 40, wherein the component (b5) is present in an amount of 0.1 to 50 parts by mass, per 100 parts by mass of the component (a5).

Item 42. The composition according to Item 40 or 41, wherein the component (c5) is present in an amount of 2 to 100 parts by mass, per 100 parts by mass of the component (a5).

Item 43. The composition according to any one of Items 40 to 42, wherein the component (d5) is at least one cross-linking agent selected from the group consisting of sulfur cross-linking agents, sulfur compound cross-linking agents, quinoid cross-linking agents, and maleimide cross-linking agents.

Item 44. Vibration-proof rubber produced using the composition according to any one of Items 40 to 43.

Item 45. An additive for imparting low heat build-up to a rubber component, comprising a compound represented by the following formula (1) or (2), or a salt of the compound,

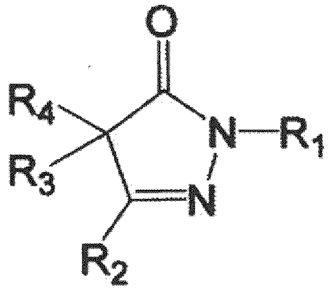

(1)

wherein
$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group; any two of $R_2$, $R_3$, and $R_4$ may be taken together to form an alkylene group; and each of these groups may have one or more substituents; or

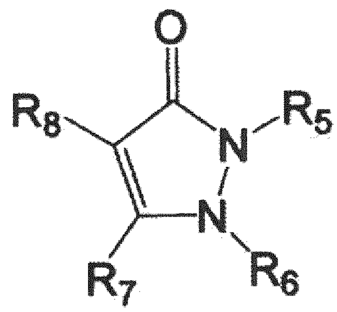

(2)

wherein
$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Item 46. A low heat built-up agent for rubber components, comprising a compound represented by the following formula (1) or (2), or a salt of the compound,

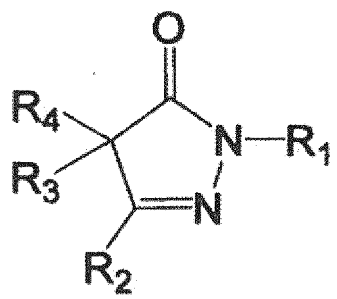

(1)

wherein
$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

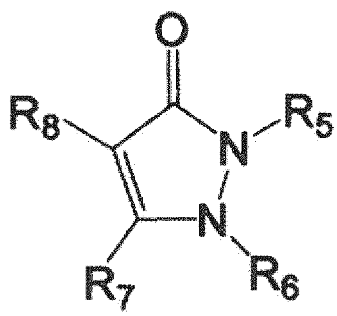

(2)

wherein
$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Item 47. An additive for imparting tear strength to a rubber component, comprising a compound represented by the formula (1) or (2), or a salt of the compound,

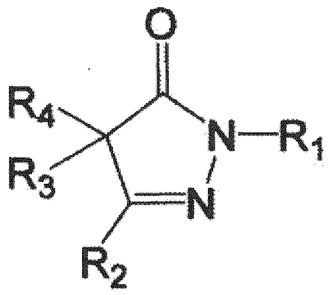
(1)

wherein

$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

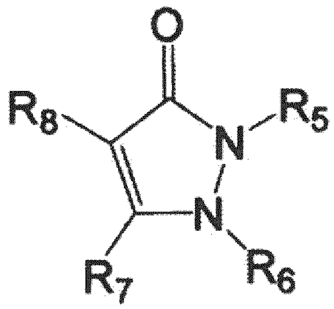
(2)

wherein

$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Item 48. A tear strength imparting agent for rubber, comprising a compound represented by the following formula (1) or (2), or a salt of the compound,

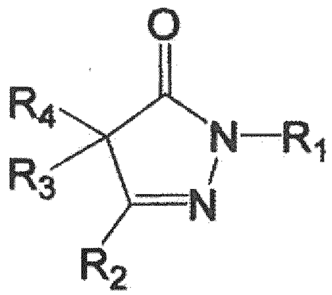
(1)

wherein

$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

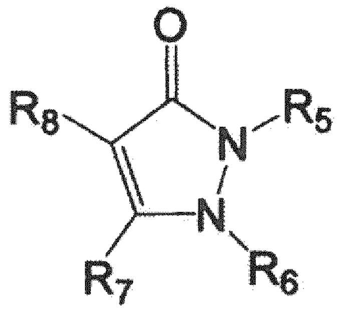

(2)

wherein

$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Item 49. A rubber material obtained by incorporating, into the rubber component, at least one member selected from the group consisting of compounds represented by the following formulas (1) and (2), and salts of the compounds,

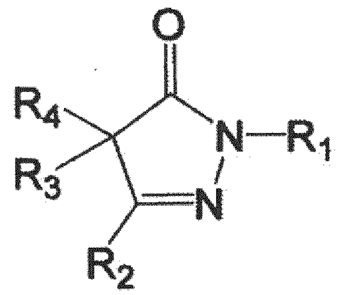

(1)

wherein

$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

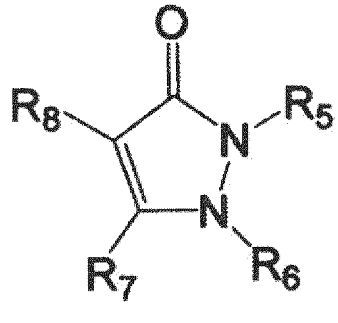

(2)

wherein

$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Item 50. The material according to Item 49, which is a compound in which in the formula (1), $R_1$, $R_3$, and $R_4$ are the same or different, and each represents a hydrogen atom, a $C_{1-4}$ linear or branched alkyl group, an aralkyl group, an aryl group, or a heterocyclic group; $R_2$ represents a hydrogen atom, a $C_{1-18}$ linear or branched alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; each of these groups may have one or more substituents; or a compound in which in the formula (2) , $R_5$ represents a hydrogen atom; $R_6$ represents

a $C_{1-4}$ linear alkyl group, aralkyl group, or an aryl group; $R_7$ and $R_8$ are the same or different, and each represents a hydrogen atom, an amino group, an alkyl group, an aralkyl group, an aryl group, or a heterocyclic group; and each of these groups may have one or more substituents.

Item 51. The material according to Item 49 or 50, wherein the rubber component is diene rubber.

Item 52. The material according to Item 49 or 50, wherein the rubber component is natural rubber.

Item 53. A master batch comprising the material according to any one of Items 49 to 52.

Item 54. A rubber composition obtained by adding carbon black and/or an inorganic filler to the material according to any of Items 49 to 52 or the master batch according to Item 5.

Item 55. The rubber composition according to Item 54, wherein the inorganic filler is silica.

Item 56. A tire, a hose, vibration-proof rubber, or a belt (a conveyor belt) produced using the rubber composition according to Item 54 or 55.

Item 57. A method for producing a rubber material, comprising incorporating, into a rubber component, at least one member selected from the group consisting of the compounds represented by the following formulas (1) and (2), and the salts of the compounds,

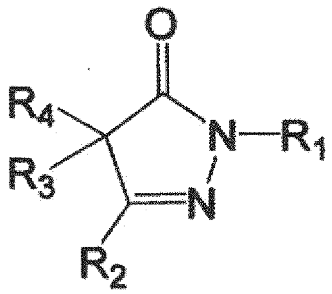
(1)

wherein
$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

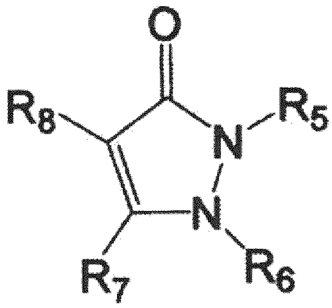
(2)

wherein
$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

Advantageous Effects of Invention

The present invention can provide a rubber composition capable of exhibiting low heat build-up.

The present invention produces a tire using a rubber composition capable of exhibiting low heat build-up, and can thereby reduce rolling resistance of the tire and lower the heat build-up of the tire, thus providing a fuel-efficient tire.
The present invention can provide an additive for imparting low heat built-up to a rubber component. By using the additive, the carbon black and/or the inorganic filler can be dispersed in the rubber component.
The present invention can provide an additive for imparting tear strength to a rubber component.
The present invention can provide a rubber composition capable of exhibiting excellent tear strength.
The present invention produces a tire using a rubber composition capable of exhibiting excellent tear strength, thus providing a tire having excellent tear strength.
The present invention provides a rubber composition having excellent processability, and a tire having excellent tear strength performance that is produced from the rubber composition.
The present invention can provide a hose having excellent low heat build-up, tear strength, and durability.
The present invention provides a rubber material having sufficient processability and excellent low heat build-up even though it contains an additive.
The present invention can provide a rubber composition that contains black and/or inorganic filler and that has sufficient processability and excellent low heat build-up even though it contains an additive.
The present invention can provide a tire having sufficient processability and excellent low heat build-up even though it contains an additive.
According to the present invention, it is possible to provide a belt (conveyor belt), a hose, and vibration-proof rubber each having excellent processability.
The present invention can provide a method for producing a rubber material having sufficient processability and excellent low heat build-up although it contains an additive.

Description of Embodiments

The present invention will be described in detail below.

Rubber Composition

The rubber composition of the present invention includes the following components (a1), (b1), and (c1): component (a1): a rubber component,
component (b1): a compound represented by the following formula (1) or a salt thereof, and
component (c1): carbon black and/or an inorganic filler.
The compound represented by the formula (1) or the salt thereof may be referred to below in this specification as "compound (1)." The compound represented by the formula (2) or the salt thereof may be referred to as "compound (2)."
The rubber composition of the present invention can be used for tires, belts (conveyor belts), suspension bushings, strut mounts, vibration-proof rubber, seismic isolation rubber, fuel hoses, concrete pouring hose ends, and the like. Among these, the rubber composition is preferably used for tires, belts (conveyer belts), vibration-proof rubber, and hoses; and is more preferably used for tires.

Compound (1) or Compound (2)

The rubber composition of the present invention includes a compound represented by the following formula (1) or a salt thereof (compound (1)) or a compound represented by the following formula (2), or a salt thereof (compound (2)).
By adding the compound (1) or the compound (2) to the rubber component, low heat build-up can be imparted to the rubber component. A tire produced (manufactured) from the rubber composition containing the compound (1) or (2) can impart low heat build-up, and can thereby reduce rolling resistance, thus exhibiting low fuel consumption performance.

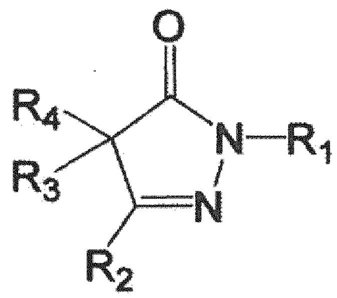

(1)

In the formula (1), $R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and $R_2$, $R_3$, and $R_4$ may be taken together to form an alkylene group; and each of these groups may have one or more substituents.

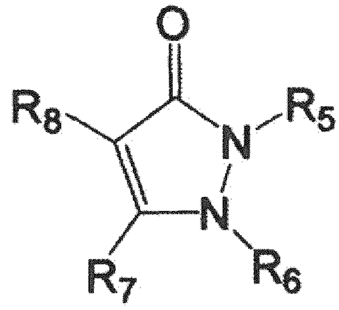

(2)

In the formula (2), $R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

The addition of the compound (1) or compound (2) to the rubber component can impart excellent tear strength to the rubber component. The tire produced (manufactured) from the rubber composition containing the compound (1) or the compound (2) exhibits excellent tear resistance.

The "alkyl" as used herein is not particularly limited. Examples include linear, branched, or cyclic alkyl groups. Specific examples include $C_{1-4}$ linear or branched alkyl groups, such as methyl, ethyl, n-propyl, isopropyl, n-butyl, isobutyl, s-butyl, t-butyl, and 1-ethylpropyl; $C_{1-18}$ linear or branched alkyl groups, such as n-pentyl, isopentyl, neopentyl, n-hexyl, isohexyl, 3-methylpentyl, n-heptyl, n-octyl, n-nonyl, n-decyl, n-undecyl, n-dodecyl, 5-propylnonyl, n-tridecyl, n-tetradecyl, n-pentadecyl, hexadecyl, heptadecyl, and octadecyl; $C_{3-8}$ cyclic alkyl groups, such as cyclopropyl, cyclobutyl, cyclopentyl, cyclohexyl, cycloheptyl, and cyclooctyl; and the like.

The "aralkyl" as used herein is not particularly limited. Examples include benzyl, phenethyl, trityl, 1-naphthylmethyl, 2-(1-naphthyl)ethyl, 2-(2-naphthyl)ethyl, and the like.

The "aryl" as used herein is not particularly limited. Examples include phenyl, biphenyl, naphthyl, dihydroindenyl, 9H-fluorenyl, and the like.

The "amino" as used herein includes an amino group represented by $-NH_2$ and substituted amino groups. Examples of substituted amino groups include linear or branched monoalkylamino groups, such as methylamino, ethylamino, n-propylamino, isopropylamino, n-butylamino, isobutylamino, s-butylamino, t-butylamino, 1-ethylpropylamino, n-pentylamino, neopentylamino, n-hexylamino, isohexylamino, and 3-methylpentylamino; and dialkylamino groups having two linear or branched alkyl groups, such as dimethylamino, ethylmethylamino, and diethylamino.

The "heterocyclic group" as used herein is not particularly limited. Examples include 2-pyridyl, 3-pyridyl, 4-pyridyl, 2-pyrazinyl, 2-pyrimidyl, 4-pyrimidyl, 5-pyrimidyl, 3-pyridazyl, 4-pyridazyl, 4-(1,2,3-triazyl), 5-(1,2,3-triazyl), 2-(1,3,5-triazyl), 3-(1,2,4-triazyl), 5-(1,2,4-triazyl), 6-(1,2,4-triazyl), 2-quinolyl, 3-quinolyl, 4-quinolyl, 5-quinolyl, 6-quinolyl, 7-quinolyl, 8-quinolyl, 1-isoquinolyl, 3-isoquinolyl, 4-isoquinolyl, 5-isoquinolyl, 6-isoquinolyl, 7-isoquinolyl, 8-isoquinolyl, 2-quinoxalyl, 3-quinoxalyl, 5-quinoxalyl, 6-quinoxalyl, 7-quinoxalyl, 8-quinoxalyl, 3-cinnolyl, 4-cinnolyl, 5-cinnolyl, 6-cinnolyl, 7-cinnolyl, 8-cinnolyl, 2-quinazolyl, 4-quinazolyl, 5-quinazolyl, 6-quinazolyl, 7-quinazolyl, 8-quinazolyl, 1-phthalazyl, 4-phthalazyl, 5-phthalazyl, 6-phthalazyl, 7-phthalazyl, 8-phthalazyl, 1-tetrahydroquinolyl, 2-tetrahydroquinolyl, 3-tetrahydroquinolyl, 4-tetrahydroquinolyl, 5-tetrahydroquinolyl, 6-tetrahydroquinolyl, 7-tetrahydroquinolyl, 8-tetrahydroquinolyl, 1-pyrrolyl, 2-pyrrolyl, 3-pyrrolyl, 2-furyl, 3-furyl, 2-thienyl, 3-thienyl, 1-imidazolyl, 2-imidazolyl, 4-imidazolyl, 5-imidazolyl, 1-pyrazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl,

3-isoxazolyl, 4-isoxazolyl, 5-isoxazolyl, 3-isothiazolyl, 4-isothiazolyl, 5-isothiazolyl, 4-(1,2,3-thiadiazolyl), 5-(1,2,3-thiadiazolyl), 3-(1,2,5-thiadiazolyl), 2-(1,3,4-thiadiazolyl), 4-(1,2,3-oxadiazolyl), 5-(1,2,3-oxadiazolyl), 3-(1,2,4-oxadiazolyl), 5-(1,2,4-oxadiazolyl), 3-(1,2,5-oxadiazolyl), 2-(1,3,4-oxadiazolyl), 1-(1,2,3-triazolyl), 4-(1,2,3-triazolyl), 5-(1,2,3-triazolyl), 1-(1,2,4-triazolyl), 3-(1,2,4-triazolyl), 5-(1,2,4-triazolyl), 1-tetrazolyl, 5-tetrazolyl, 1-indolyl, 2-indolyl, 3-indolyl, 4-indolyl, 5-indolyl, 6-indolyl, 7-indolyl, 1-isoindolyl, 2-isoindolyl, 3-isoindolyl, 4-isoindolyl, 5-isoindolyl, 6-isoindolyl, 7-isoindolyl, 1-benzimidazolyl, 2-benzimidazolyl, 4-benzimidazolyl, 5-benzimidazolyl, 6-benzimidazolyl, 7-benzimidazolyl, 2-benzofuranyl, 3-benzofuranyl, 4-benzofuranyl, 5-benzofuranyl, 6-benzofuranyl, 7-benzofuranyl, 1-isobenzofuranyl, 3-isobenzofuranyl, 4-isobenzofuranyl, 5-isobenzofuranyl, 6-isobenzofuranyl, 7-isobenzofuranyl, 2-benzothienyl, 3-benzothienyl, 4-benzothienyl, 5-benzothienyl, 6-benzothienyl, 7-benzothienyl, 2-benzoxazolyl, 4-benzoxazolyl, 5-benzoxazolyl, 6-benzoxazolyl, 7-benzoxazolyl, 2-benzothiazolyl, 4-benzothiazolyl, 5-benzothiazolyl, 6-benzothiazolyl, 7-benzothiazolyl, 1-indazolyl, 3-indazolyl, 4-indazolyl, 5-indazolyl, 6-indazolyl, 7-indazolyl, 2-morpholyl, 3-morpholyl, 4-morpholyl, 1-piperazyl, 2-piperazyl, 1-piperidyl, 2-piperidyl, 3-piperidyl, 4-piperidyl, 2-tetrahydropyranyl, 3-tetrahydropyranyl, 4-tetrahydropyranyl, 2-tetrahydrothiopyranyl, 3-tetrahydrothiopyranyl, 4-tetrahydrothiopyranyl, 1-pyrrolidyl, 2-pyrrolidyl, 3-pyrrolidyl, furanyl, 2-tetrahydrofuranyl, 3-tetrahydrofuranyl, 2-tetrahydrothienyl, 3-tetrahydrothienyl, 5-methyl-3-oxo-2,3-dihydro-1H-pyrazol-4-yl, and the like.

The "alkylidene group" as used herein is not particularly limited. Examples include methylidene, ethylidene, propylidene, isopropylidene, butylidene, and the like.

The "alkylene" as used herein is not particularly limited. Examples include ethylene, trimethylene, tetramethylene, pentamethylene, hexamethylene, heptamethylene, and the like. These alkylene groups may contain nitrogen, oxygen, or sulfur, or may be via phenylene. Such alkylene groups include $-CH_2NHCH_2-$, $-CH_2NHCH_2CH_2-$, $-CH_2NHNHCH_2-$, $-CH_2CH_2NHCH_2CH_2-$, $-CH_2NHNHCH_2CH_2-$, $-CH_2NHCH_2NHCH_2-$, $-CH_2CH_2CH_2NHCH_2CH_2CH_2-$, $-CH_2OCH_2CH_2-$, $-CH_2CH_2OCH_2CH_2-$, $-CH_2SCH_2CH_2-$, $-CH_2CH_2SCH_2CH_2-$,

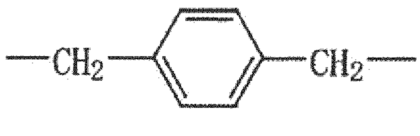 、 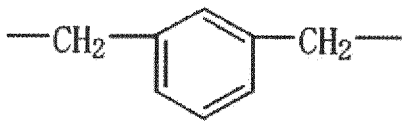 、

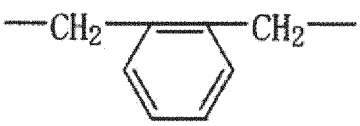 、 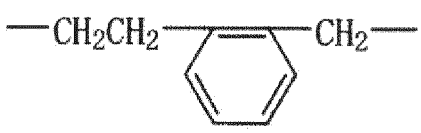

and the like.

The alkyl, aralkyl, aryl, heterocyclic, alkylidene, and alkylene groups may each have one or more substituents at a replaceable position. The "substituent" is not particularly limited. Examples of substituents include halogen atoms, amino, aminoalkyl, alkoxycarbonyl, acyl, acyloxy, amide, carboxyl, carboxyalkyl, formyl, nitrile, nitro, alkyl, hydroxyalkyl, hydroxy, alkoxy, aryl, aryloxy, heterocyclic, thiol, alkylthio, arylthio, and like groups. The number of substituents is preferably 1 to 5, and more preferably 1 to 3.

The "halogen atom" as used herein includes fluorine, chlorine, bromine, and iodine atoms. Preferable halogen atoms are chlorine, bromine, and iodine atoms.

The "aminoalkyl" as used herein is not particularly limited. Examples include amino alkyl groups, such as aminomethyl, methylaminomethyl, ethylaminomethyl, dimethylaminomethyl, ethylmethylaminomethyl, diethylaminomethyl, 2-aminoethyl, 2-(methylamino)ethyl, 2-(ethylamino)ethyl, 2-(dimethylamino)ethyl, 2-(ethylmethylamino)ethyl, 2-(diethylamino)ethyl, 3-aminopropyl, 3-(methylamino)propyl, 3-(ethylamino)propyl, 3-(dimethylamino)propyl, 3-(ethylmethylamino)propyl, and 3-(diethylamino)propyl; aminoalkyl groups substituted with monoalkyl or aminoalkyl groups substituted with dialkyl; and the like.

The "alkoxycarbonyl" as used herein is not particularly limited. Examples include methoxycarbonyl, ethoxycarbonyl, and the like.

The "acyl" as used herein is not particularly limited. Examples include $C_{1-4}$ linear or branched alkylcarbonyl groups, such as acetyl, propionyl, and pivaloyl.

The "acyloxy" as used herein is not particularly limited. Examples include acetyloxy, propionyloxy, n-butyryloxy, and the like.

The "amide" as used herein is not particularly limited. Examples include carboxylic acid amide groups, such as acetamide and benzamide; thioamide groups such as thioacetamide and thiobenzamide; N-substituted amide groups such as N-methylacetamide and N-benzylacetamide; and the like.

The "carboxyalkyl" as used herein is not particularly limited. Examples include carboxyalkyl groups, such as carboxymethyl, carboxyethyl, carboxy-n-propyl, carboxy-n-butyl, carboxy-n-pentyl, and carboxy-n-hexyl.

The "hydroxyalkyl" as used herein is not particularly limited. Examples include hydroxyalkyl groups, such as hydroxymethyl, hydroxyethyl, hydroxy-n-propyl, and hydroxy-n-butyl.

The "alkoxy" as used herein is not particularly limited. Examples include linear, branched, or cyclic alkoxy groups. Specific examples include linear or branched alkoxy groups, such as methoxy, ethoxy, n-propoxy, isopropoxy, n-butoxy, t-butoxy, n-pentyloxy, neopentyloxy, and n-hexyloxy; cyclic alkoxy groups, such as cyclopropyloxy, cyclobutyloxy, cyclopenthyloxy, cyclohexyloxy, cycloheptyloxy, and cyclooctyloxy; and the like.

The "aryloxy" as used herein is not particularly limited. Examples include phenoxy, biphenyloxy, naphthoxy, and the like.

Among the compounds (1) , a compound in which $R_1$, $R_3$, and $R_4$ are the same or different, and each represents a hydrogen atom, a $C_{1-4}$ linear or branched alkyl group, an aralkyl group, an aryl group, or a heterocyclic group is preferable.

Among the compounds (1), a compound in which $R_1$ represents a hydrogen atom is preferable.

Among the compounds (1), a compound in which $R_2$ represents a hydrogen atom, a $C_{1-4}$ linear or branched alkyl group, an aralkyl group, an aryl group, or a heterocyclic group is preferable, and a compound in which $R_2$ represents a hydrogen atom, a $C_{1-4}$ linear or branched alkyl group, a benzyl group, a phenyl group, a naphthyl group, or a furyl group is more preferable, and a compound in which $R_2$ represents a hydrogen atom, or a $C_{1-4}$ linear alkyl group is particularly preferable.

Among the compounds (1), a compound in which at least one of $R_3$ and $R_4$ represents a hydrogen atom is preferable, and a compound in which both $R_3$ and $R_4$ are hydrogen atoms is more preferable.

Among the compounds (1), a compound in which $R_1$ represents a hydrogen atom, $R_2$ represents a hydrogen atom, a $C_{1-4}$ linear or branched alkyl group, an aralkyl group, an aryl group, or a heterocyclic group; and $R_3$ and $R_4$ both represent hydrogen atoms, and a compound in which $R_1$ represents a hydrogen atom, $R_2$ represents a hydrogen atom, a $C_{1-4}$ linear or branched alkyl group, an aralkyl group, an aryl group, or a heterocyclic group; and $R_3$ and $R_4$ are taken together to form an alkylidene group are more preferable; and a compound in which $R_1$ represents a hydrogen atom, $R_2$ represents a hydrogen atom or a $C_{1-4}$ linear alkyl group, and $R_3$ and $R_4$ are both hydrogen atoms is particularly preferable.

Of the compounds (2) , a compound in which $R_5$ represents a hydrogen atom; $R_6$ is a $C_{1-4}$ linear or branched alkyl group, an aralkyl group, or an aryl group; and $R_7$ and $R_8$ are the same or different, and each represents a hydrogen atom, a $C_{1-4}$ linear or branched alkyl group, an aralkyl group, an aryl group, an amino group, or a heterocyclic group is preferable.

Among the compounds (2) , a compound in which $R_6$ represents a $C_{1-4}$ linear alkyl group, an aralkyl group, or an aryl group is preferable, and a compound in which $R_6$ represents a $C_{1-4}$ linear alkyl group or an aryl group is more preferable.

Among the compounds (2), a compound in which $R_7$ and $R_8$ are the same or different, and each represents a hydrogen atom, a $C_{1-4}$ linear alkyl group, or an amino group is preferable.

Among the compounds (2) , a compound in which $R_5$ represents a hydrogen atom; $R_6$ represents a $C_{1-4}$ linear alkyl or an aryl group; $R_7$ and $R_8$ are the same or different, and each represents a hydrogen atom, a $C_{1-4}$ linear alkyl group, or an amino group is preferable.

Of the compounds (1) and (2), the compound (1) is particularly preferable.

Specific examples of the compound (1) or (2) include 5-pyrazolone, 3-methyl-5-pyrazolone, 3-(naphthalene-2-yl)-1H-pyrazol-5(4H)-one, 3-(furan-2-yl)-1H-pyrazol-5(4H)-one, 3-phenyl-1H-pyrazol-5(4H)-one, 3-propyl-1H-pyrazol-5(4H)-one, 3-undecyl-1H-pyrazol-5(4H)-one, 4-(2-hydroxyethyl)-3-methyl-1H-pyrazol-5(4H)-one, 4-benzyl-3-methyl-1H-pyrazol-5(4H)-one, 4,4'-(phenylmethylene)bis(5-methyl-1H-pyrazol-3(2H)-one), 4-[(dimethylamino)methylidenel-3-methyl-1H-pyrazol-5(4H)-one, 4-methyl-2,3-diazospiro[4.4]non-3-en-1-one, 5-methyl-2-(4-nitrophenyl)-1H-pyrazol-3(2H)-one, 5-methyl-2-phenyl-2,4-dihydro-3H-pyrazol-3-one, 1,5-dimethyl-2-phenyl-1H-pyrazol-3(2H)-one, 4,5,6,7-tetrahydro-2H-indazol-3(3aH)-one, 4-{[4-dimethylamino]phenyl}methylidene}-3-methyl-1H-pyrazol-5(4H)-one, 1-phenyl-1H-pyrazol-3(2H)-one, 4,4'-(4-hydroxyphenylmethylene)bis(5-methyl-1H-pyrazol-3(2H)-one), 1,3-diphenyl-1H-pyrazol-5(4H)-one, 4,4'-(4-nitrophenylmethylene)bis(5-methyl-1H-pyrazol-3(2H)-one), 4-amino-1,5-dimethyl-2-phenyl-1H-pyrazol-3(2H)-one, and the like.

Of these, the compound (1) is a preferable compound, and 5-pyrazolone, 3-methyl-5-pyrazolone, 3-(naphthalene-2-yl)-1H-pyrazol-5(4H)-one, 3-(furan-2-yl)-1H-pyrazol-5(4H)-one, 3-phenyl-1H-pyrazol-5(4H)-one, and 3-propyl-1H-pyrazol-5(4H)-one are more preferable.

The rubber composition of the present invention may include one kind of compound or a mixture of two or more compounds.

Some of the compounds (1) or (2) may have tautomeric forms. When tautomerization is possible (e.g., in a solution), the compounds can attain the tautomeric chemical equilibrium state. The compounds (1) or (2) can be present

as tautomers represented by the following formulas (3) to (9).

The compound (compound (1)-A) in which $R_1$ and $R_3$ are hydrogen atoms in the formula (1) described above includes tautomers represented by the following formulas (3) to (5),

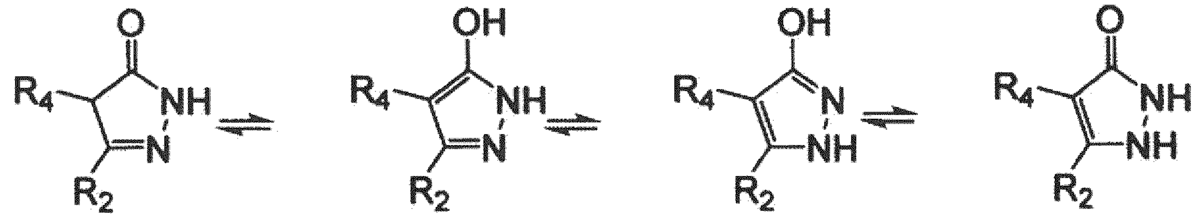

(1) -A (3) (4) (5)

wherein $R_2$ and $R_4$ are as defined above.

The compound (compound (1)-B) in which $R_3$ represents a hydrogen atom in the formula (1) described above includes tautomers represented by the following formulas (6) and (7),

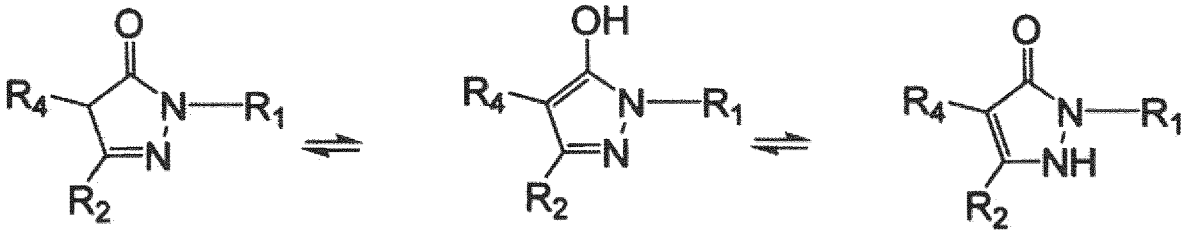

(1) -B (6) (7)

wherein $R_1$, $R_2$, and $R_4$ are as defined above.

The compound (compound (1)-C) in which $R_1$ represents a hydrogen atom in the formula (1) described above includes a tautomer represented by the following formula (8),

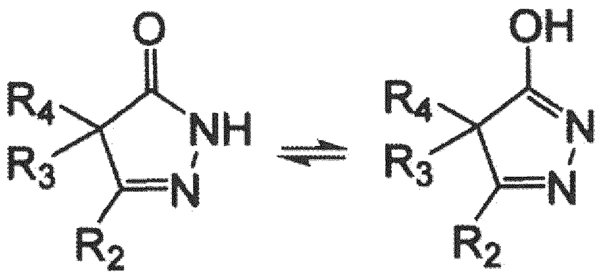

(1) -C (8)

wherein $R_2$, $R_3$, and $R_4$ are as defined above.

The compound (compound (2) -A) in which $R_5$ represents a hydrogen atom in the formula (2) described above includes a tautomer represented by the following formula (9),

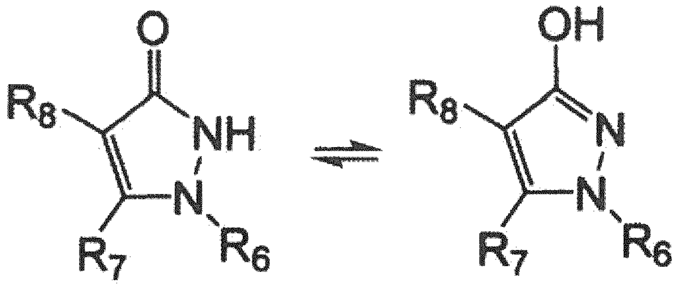

(2) −A

(9)

wherein $R_6$, $R_7$, and $R_8$ are as defined above.

The tautomers represented by the formulas (3) to (9) above and the compounds (1) or (2) attain an equilibrium state in which both isomers are present. Therefore, unless otherwise specified, all tautomeric forms of the compounds (1) or (2) are within the scope of the present invention.

Accordingly, the additives of the present invention also include tautomers of compound (1) or (2).

The salts of the compounds represented by the formula (1) or (2) are not particularly limited, and include various types of salts. Examples of such salts include inorganic acid salts, such as hydrochloride, sulfate, and nitrate; organic acid salts, such as acetate and methanesulfonate; alkali metal salts, such as sodium salts and potassium salts; alkaline earth metal salts, such as magnesium salts and calcium salts; ammonium salts, such as dimethylammonium and trietaylammonium; and the like.

The rubber composition of the present invention may include a mixture containing the compound (1) or the compound (2) in any proportion.

Rubber Component

In this specification, the rubber component is not particularly limited. Examples include natural rubber (NR), synthetic diene rubber, and a mixture of natural rubber and synthetic diene rubber; and non-diene rubber other than these types of rubber.

Examples of natural rubber include natural rubber latex, technically specified rubber (TSR), ribbed smoked sheet (RSS) , gutta-percha, Chinese gutta-percha *(Eucommia ulmoides)*-derived natural rubber, guayule-derived natural rubber, Russian dandelion (*Taraxacum kok-saghyz*)-derived natural rubber, and the like. Examples of natural rubber according to the present invention further include rubber obtained by modifying these types of natural rubber, such as epoxidated natural rubber, methacrylic acid modified natural rubber, and styrene modified natural rubber.

Examples of synthetic diene rubber include styrene-butadiene copolymer rubber (SBR), butadiene rubber (BR), isoprene rubber (IR), nitrile rubber (NBR), chloroprene rubber (CR), ethylene-propylene-diene terpolymer rubber (EPDM), styreneisoprene-styrene triblock copolymer (SIS), styrene-butadienestyrene triblock copolymer (SBS), and the like; and modified synthetic diene rubber thereof. Examples of modified synthetic diene rubber include main-chain-modified, one-terminal-modified, both-terminal-modified, or like modified diene rubber. Examples of functional groups of modified synthetic diene rubber include functional groups, such as epoxy, amino, alkoxysilyl, and hydroxyl groups. One or two or more kinds of these functional groups may be included in modified synthetic diene rubber.

The method for producing a synthetic diene rubber is not particularly limited. Examples of the production method include emulsion polymerization, solution polymerization, radical polymerization, anionic polymerization, cationic polymerization, and the like.

The glass transition point of the synthetic diene rubber is also not particularly limited.

The cis/trans/vinyl ratio of the double-bond portion of the natural rubber or synthetic diene rubber is not particularly limited, and any ratio is suitable. The number average molecular weight and molecular weight distribution of the diene rubber are not particularly limited. The diene rubber preferably has a number average molecular weight of 500 to 3000000, and a molecular weight distribution of 1.5 to 15.

A wide variety of non-diene rubber can be used as the non-diene rubber.

The rubber component can be used singly, or as a mixture (blend) of two or more. Among these, the rubber component is preferably natural rubber, IR, SBR, BR, or a mixture of two or more of these types of rubber. More preferably, the rubber component is natural rubber, SBR, BR, or a mixture of two or more of these types of rubber.

Carbon Black

Carbon black is usually used to improve rubber reinforcement. In this specification, carbon black is not included

in the inorganic filler.

The carbon black for use is not particularly limited. For example, commercially available carbon black, carbon-silica dual phase fillers, and the like can be used. Incorporating carbon black into a rubber component reduces electric resistance of rubber, thus providing an effect of suppressing electrical charge and an effect of enhancing rubber strength.

Specific examples of carbon black include high-, middle-, or low-structure SAF, ISAF, IISAF, N110, N134, N220, N234, N330, N339, N375, N550, HAF, FEF, GPF, or SRF-grade carbon black, and the like. Among these, SAF, ISAF, IISAF, N134, N234, N330, N339, N375, HAF, or FEF-grade carbon black is preferable.

There is no particular limitation on the DBP absorption of the carbon black. The carbon black preferably has a DBP absorption of 60 to 200 $cm^3$/100 g, more preferably 70 to 180 $cm^3$/100 g, and particularly preferably 80 to 160 $cm^3$/100 g.

The carbon black preferably has a nitrogen adsorption specific surface area (N2SA, measured according to JIS K6217-2: 2001) of 30 to 200 $m^2$/g, more preferably 40 to 180 $m^2$/g, and particularly preferably 50 to 160 $m^2$/g.

In the rubber composition containing carbon black, the compound (1) or a reaction product of the rubber component and the compound (1) is believed to strongly interact with carbon black. Therefore, when the rubber composition of the present invention is used, dispersibility of carbon black, in particular, is increased significantly, and low heat build-up of the rubber composition can be significantly improved.

In the rubber composition containing carbon black, the compound (1), the compound (2), a reaction product of the rubber component and compound (1) or a reaction product of the rubber component and compound (2) is believed to strongly interact with carbon black. Therefore, when the rubber composition of the present invention is used, dispersibility of carbon black, in particular, is increased significantly, and tear strength of the rubber composition can be significantly improved.

Inorganic Filler

The inorganic filler is not particularly limited as long as it is an inorganic compound usually used in the rubber industry. Examples of usable inorganic compounds include silica; aluminas ($Al_2O_3$) such as γ-alumina and α-alumina; alumina monohydrates ($Al_2O_3 \cdot H_2O$) such as boehmite and diaspore; aluminum hydroxides [$Al(OH)_3$] such as gibbsite and bayerite; aluminum carbonate [$Al_2(CO_3)_3$], magnesium hydroxide [$Mg(OH)_2$], magnesium oxide (MgO), magnesium carbonate ($MgCO_3$), talc ($3MgO \cdot 4SiO_2 \cdot H_2O$), attapulgite ($5MgO \cdot 8SiO_2 \cdot 9H_2O$), titanium white ($TiO_2$), titanium black ($TiO_{2n-1}$), calcium oxide (CaO), calcium hydroxide [$Ca(OH)_2$], magnesium aluminum oxide ($MgO \cdot Al_2O_3$), clay ($Al_2O_3 \cdot 2SiO_2$), kaolin ($Al_2O_3 \cdot 2SiO_2 \cdot 2H_2O$), pyrophyllite ($Al_2O_3 \cdot 4SiO_2 \cdot H_2O$), bentonite ($Al_2O_3 \cdot 4SiO_2 \cdot 2H_2O$), aluminum silicates ($Al_2SiO_5$, $Al_4 \cdot 3SiO_4 \cdot 5H_2O$, etc.), magnesium silicates ($Mg_2SiO_4$, $MgSiO_3$, etc.), calcium silicates ($Ca_2 \cdot SiO_4$ etc.), aluminum calcium silicates ($Al_2O3 \cdot CaO \cdot 2SiO_2$ etc.), magnesium calcium silicate ($CaMgSiO_4$), calcium carbonate ($CaCO_3$), zirconium oxide ($ZrO_2$), zirconium hydroxide [$ZrO(OH)_2 \cdot nH_2O$], zirconium carbonate [$Zr(CO_3)_2$], zinc acrylate, zinc methacrylate, and crystalline aluminosilicates containing hydrogen, alkali metal, or alkaline earth metal that compensate charge, such as various types of zeolites. To enhance affinity to the rubber component, the surface of these inorganic fillers may be treated with an organic compound.

From the viewpoint of imparting rubber strength, silica is preferably used as the inorganic filler. Using silica alone or a combination of silica with one or more inorganic compounds usually used in the rubber industry is more preferable. When the inorganic filler is a combination of silica with one or more inorganic compounds other than silica, their amounts may be appropriately adjusted so that the total amount of the inorganic filler components falls within the range mentioned below.

Adding silica is preferable because it can impart rubber strength.

As silica, any type of commercially available products can be used. Among these, wet silica, dry silica, or colloidal silica is preferable, and wet silica is more preferable. To enhance affinity to the rubber component, the surface of silica may be treated with an organic compound.

The BET specific surface area of silica is not particularly limited and may be, for example, in the range of 40 to 350 $m^2$/g. Silica that has a BET specific surface area within this range is advantageous in that rubber reinforcement and dispersibility in the rubber component can both be achieved. The BET specific surface area is measured according to ISO 5794/1.

From this viewpoint, preferred is silica having a BET specific surface area of 80 to 300 $m^2$/g, more preferred is silica having a BET specific surface area of 100 to 270 $m^2$/g, and particularly preferred is silica having a BET specific surface area of 110 to 270 $m^2$/g.

Examples of commercially available products of such silica include products under the following trade names: HD165MP (BET specific surface area: 165 $m^2$/g), HD115MP (BET specific surface area: 115 $m^2$/g), HD200MP (BET specific surface area: 200 $m^2$/g), and HD250MP (BET specific surface area: 250 $m^2$/g), all produced by Quechen Silicon Chemical Co., Ltd.; Nipsil AQ (BET specific surface area: 205 $m^2$/g) and Nipsil KQ (BET specific surface area: 240 $m^2$/g), both produced by Tosoh Silica Corporation; Ultrasil VN3 (BET specific surface area: 175 $m^2$/g) produced by Degussa

AG; and the like.

When the compound (1) or compound (2) is incorporated into a rubber composition containing an inorganic filler, in particular, silica, dispersibility of silica can be significantly improved, thus remarkably improving low heat build-up of the rubber composition. Specifically, the compound (1) or compound (2) can be used as a dispersant for carbon black and/or inorganic fillers, a low heat build-up agent, a heat build-up inhibitor, or a heat build-up suppressor. Preferably, the compound (1) or compound (2) can be used as a dispersant for rubber, a low heat build-up agent for rubber, a heat build-up inhibitor for rubber, or a heat build-up suppressor for rubber.

When the compound (1) or compound (2) is incorporated into a rubber composition containing an inorganic filler, in particular, silica, dispersibility of silica can be significantly improved, thus remarkably improving tear strength of the rubber composition.

The amount of the compound (1) or compound (2) is usually 0.1 to 50 parts by mass, preferably 0.1 to 20 parts by mass, and more preferably 0.2 to 10 parts by mass, per 100 parts by mass of the rubber component in the rubber composition.

The amount of the carbon black and/or inorganic filler is not particularly limited. For example, the amount of the carbon black and/or inorganic filler is usually 2 to 200 parts by mass, preferably 30 to 130 parts by mass, and more preferably 35 to 110 parts by mass, per 100 parts by mass of the rubber component.

When the carbon black and inorganic filler are both used, their amounts are appropriately adjusted so that the total amount of these components falls within the range mentioned above.

Incorporating 2 parts by mass or more of the carbon black and/or inorganic filler is preferable from the viewpoint of improving rubber composition reinforcement, whereas incorporating 200 parts by mass or less of the carbon black and/or inorganic filler is preferable from the viewpoint of reducing rolling resistance.

The amount of the carbon black is usually 20 to 200 parts by mass, preferably 30 to 130 parts by mass, and more preferably 35 to 100 parts by mass, per 100 parts by mass of the rubber component.

Two parts by mass or more of the carbon black is preferable in terms of ensuring antistatic performance and rubber strength performance, whereas 200 parts by mass or less of the carbon black is preferable in terms of reducing rolling resistance.

The amount of the inorganic filler is usually 10 to 200 parts by mass per 100 parts by mass of the rubber component.

The amount of silica is usually 2 to 1200 parts by mass, preferably 30 to 130 parts by mass, and more preferably 35 to 130 parts by mass, per 100 parts by mass of the rubber component.

In particular, to achieve both kinematic performance and low fuel consumption performance, the amount of silica is usually 20 to 200 parts by mass, preferably 30 to 130 parts by mass, and more preferably 35 to 130 parts by mass, per 100 parts by mass of the rubber component.

## Other Ingredients

In addition to the compound (1) or compound (2), and the carbon black and/or inorganic filler, the rubber composition of the present invention may comprise ingredients usually used in the rubber industry, such as antioxidants, ozone protectants, softeners, processing aids, waxes, resins, foaming agents, oils, stearic acid, zinc oxide (ZnO), vulcanization accelerators, vulcanization retarders, and vulcanizing agents (sulfur) as long as the objects of the present invention are not impaired. As such ingredients, commercially available products can be suitably used.

It is preferable that the rubber composition of the present invention comprises no phenolic resins.

Further, a silane coupling agent, a titanate coupling agent, an aluminate coupling agent, or a zirconate coupling agent may be incorporated into the rubber composition that comprises carbon black and/or an inorganic filler, such as silica, for the purpose of enhancing the rubber composition reinforcement by carbon black and/or silica, or enhancing abrasion resistance and tear strength of the rubber composition.

The silane coupling agent that can be used with carbon black and/or an inorganic filler is not particularly limited, and commercially available products can be suitably used. Examples of such silane coupling agents include sulfide, polysulfide, thioester, thiol, olefin, epoxy, amino, or alkyl silane coupling agents.

Examples of sulfide silane coupling agents include bis(3-triethoxysilylpropyl)tetrasulfide, bis(3-trimethoxysilylpropyl)tetrasulfide, bis(3 - methyldimethoxysilylpropyl)tetrasulfide, bis(2-triethoxysilylethyl)tetrasulfide, bis(3-triethoxysilylpropyl) disulfide, bis(3-trimethoxysilylpropyl)disulfide, bis(3-methyldimethoxysilylpropyl)disulfide, bis(2 - triethoxysilylethyl)disulfide, bis(3-triethoxysilylpropyl)trisulfide, bis(3-trimethoxysilylpropyl)trisulfide, bis(3-methyldimethoxysilylpropyl)trisulfide, bis(2-triethoxysilylethyl)trisulfide, bis(3-monoethoxydimethylsilylpropyl)tetrasulfide, bis(3-monoethoxydimethylsilylpropyl)trisulfide, bis(3-monoethoxydimethylsilylpropyl)disulfide, bis(3-monomethoxydimethylsilylpropyl)tetrasulfide, bis(3 - monomethoxydimethylsilylpropyl)trisulfide, bis(3-monomethoxydimethylsilylpropyl)disulfide, bis(2-monoethoxydimethylsilylethyl)tetrasulfide, bis(2-monoethoxydimethylsilylethyl)trisulfide, bis(2-monoethoxydimethylsilylethyl)disulfide, and the like. Among these, bis(3-triethoxysilylpropyl)tetrasulfide is particularly preferable.

Examples of thioester silane coupling agents include 3-hexanoylthiopropyltriethoxysilane, 3-octanoylthiopropyltriethoxysilane, 3-decanoylthiopropyltriethoxysilane, 3-lauroylthiopropyltriethoxysilane, 2-hexanoylthioethyltriethoxysilane, 2-octanoylthioethyltriethoxysilane, 2-decanoylthioethyltriethoxysilane, 2-lauroylthioethyltriethoxysilane, 3-hexanoylthiopropyltrimethoxysilane, 3-octanoylthiopropyltrimethoxysilane, 3-decanoylthiopropyltrimethoxysilane, 3-lauroylthiopropyltrimethoxysilane, 2-hexanoylthioethyltrimethoxysilane, 2-octanoylthioethyltrimethoxysilane, 2-decanoylthioethyltrimethoxysilane, 2-lauroylthioethyltrimethoxysilane, and the like.

Examples of thiol silane coupling agents include 3-mercaptopropyltrimethoxysilane, 3-mercaptopropyltriethoxysilane, 3-mercaptopropylmethyldimethoxysilane, 3-[ethoxybis(3,6,9,12,15-pentaoxaoctacosan-1-yloxy)silyl]-1-propanethiol, and the like.

Examples of olefin silane coupling agents include dimethoxymethylvinylsilane, vinyltrimethoxysilane, dimethylethoxyvinylsilane, diethoxymethylvinylsilane, triethoxyvinylsilane, vinyltris(2-methoxyethoxy)silane, allyltrimethoxysilane, allyltriethoxysilane, p-styryltrimethoxysilane, 3-(methoxydimethoxydimethylsilyl)propyl acrylate, 3-(trimethoxysilyl)propyl acrylate, 3-[dimethoxy(methyl)silyl]propyl methacrylate, 3-(trimethoxysilyl)propyl methacrylate, 3-[dimethoxy(methyl)silyl]propyl methacrylate, 3-(triethoxysilyl)propyl methacrylate, 3-[tris(trimethylsiloxy)silyl]propyl methacrylate, and the like.

Examples of epoxy silane coupling agents include 3-glycidyloxypropyl(dimethoxy)methylsilane, 3-glycidyloxypropyltrimethoxysilane, diethoxy(3-glycidyloxypropyl)methylsilane, triethoxy(3-glycidyloxypropyl)silane, 2-(3,4-epoxycyclohexyl)ethyltrimethoxysilane, and the like. Among these, 3-glycidyloxypropyltrimethoxysilane is preferable.

Examples of amino silane coupling agents include N-2-(aminoethyl)-3-aminopropylmethyldimethoxysilane, N-2-(aminoethyl)-3-aminopropyltrimethoxysilane, 3-aminopropyltrimethoxysilane, 3-aminopropyltriethoxysilane, 3-ethoxysilyl-N-(1,3-dimethylbutylidene)propylamine, N-phenyl-3-aminopropyltrimethoxysilane, N-(vinylbenzyl)-2-aminoethyl-3-aminopropyltrimethoxysilane, and the like. Among these, 3-aminopropyltriethoxysilane is preferable.

Examples of alkyl silane coupling agents include methyltrimethoxysilane, dimethyldimethoxysilane, trimethylmethoxysilane, methyltriethoxysilane, ethyltrimethoxysilane, n-propyltrimethoxysilane, isobutyltrimethoxysilane, isobutyltriethoxysilane, n-hexyltrimethoxysilane, n-hexyltriethoxysilane, cyclohexylmethyldimethoxysilane, n-octyltriethoxysilane, n-decyltrimethoxysilane, and the like. Among these, methyltriethoxysilane is preferable.

Among these silane coupling agents, bis(3-triethoxysilylpropyl)tetrasulfide can be particularly preferably used.

The titanate coupling agent that can be used with carbon black and/or an inorganic filler is not particularly limited, and commercially available products can be suitably used. Examples of such titanate coupling agents include alkoxide, chelate, or acylate titanate coupling agents.

Examples of alkoxide titanate coupling agents include tetraisopropyl titanate, tetranormalbutyl titanate, butyl titanate dimer, tetraoctyl titanate, tetra tertiary butyl titanate, tetrastearyl titanate, and the like. Among these, tetraisopropyl titanate is preferable.

Examples of chelate titanate coupling agents include titanium acetylacetonate, titanium tetraacetyl acetonate, titanium ethyl acetoacetate, titanium dodecylbenzenesulfonate compounds, titanium phosphate compounds, titanium octyleneglycolate, titanium ethyl acetoacetate, titanium lactate ammonium salt, titanium lactate, titanium ethanol aminate, titanium octyleneglycolate, titanium aminoethylaminoethanolate, and the like. Among these, titanium acetylacetonate is preferable.

Examples of acylate titanate coupling agents include titanium isostearate and the like.

The aluminate coupling agent that can be used with carbon black and/or an inorganic filler is not particularly limited, and commercially available products can be suitably used. Examples of such aluminate coupling agents include 9-octadecenylacetoacetate aluminum diisopropylate, aluminum sec-butoxide, aluminum trisacetylacetonate, aluminum bisethyl acetoacetate monoacetylacetonate, aluminum trisethyl acetoacetate, and the like. Among these, 9-octadecenylacetoacetate aluminum diisopropylate is preferable. The zirconate coupling agent that can be used with carbon black and/or an inorganic filler is not particularly limited, and commercially available products can be suitably used. Examples of such zirconate coupling agents include alkoxide, chelate, or acylate zirconate coupling agents.

Examples of alkoxide zirconate coupling agents include normal propyl zirconate, normal butyl zirconate, and the like. Among these, normal butyl zirconate is preferable.

Examples of chelate zirconate coupling agents include zirconium tetraacetylacetonate, zirconium monoacetylacetonate, zirconium ethylacetoacetate, zirconium lactate ammonium salt, and the like. Among these, zirconium tetraacetylacetonate is preferable.

Examples of acylate zirconate coupling agents include zirconium stearate, zirconium octylate, and the like. Among these, zirconium stearate is preferable.

In the present invention, silane coupling agents, titanate coupling agents, aluminate coupling agents, and zirconate coupling agents can be used singly, or in a combination of two or more.

The amount of silane coupling agent in the rubber composition of the present invention is preferably 0.1 to 20 parts by mass, and particularly preferably 3 to 15 parts by mass, per 100 parts by mass of the carbon black and/or inorganic filler. This is because 0.1 parts by mass or more of a silane coupling agent can more advantageously achieve

improved tear strength of the rubber composition, whereas 20 parts by mass or less of a silane coupling agent can reduce the cost of the rubber composition and increase economic efficiency.

It is also preferable that the rubber composition of the present invention is a rubber composition comprising the following components (a2), (b2), and (c2). This rubber composition is excellent in wet grip performance. This rubber composition can be used for tires, belts (conveyor belts), vibration-proof rubber, seismic isolation rubber, hoses, and the like. In particular, the rubber composition is preferably used for tires, vibration-proof rubber, or hoses.

Component (a2): a rubber component;

Component (b2): at least one compound selected from the group consisting of a compound represented by the following formula (1), a compound represented by the following formula (2), and salts of the compounds; and

Component (c2): silica;

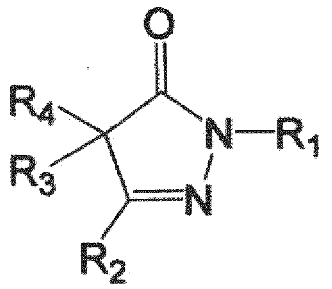

(1)

wherein $R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

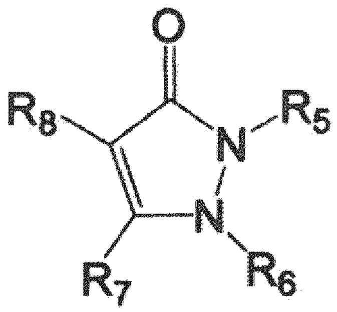

(2)

wherein $R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

The definitions of the components (a2), (b2), and (c2) are the same as those of the rubber component, compound (1) or compound (2), and silica described above.

The amount of the component (b2) is preferably 0.01 to 50 parts by mass, more preferably 0.05 to 30 parts by mass, and even more preferably 0.1 to 10 parts by mass, per 100 parts by mass of the component (a2) in the rubber composition.

The amount of the component (c2) is preferably 10 to 100 parts by mass, more preferably 20 to 80 parts by mass, and even more preferably 40 to 60 parts by mass, per 100 parts by mass of the component (a2) in the rubber composition.

The rubber composition of the present invention preferably further comprises a hydrocarbon polymer as component (d2) .

In the present specification, the term "hydrocarbon polymer" is defined as a polymer that has no heteroatom-containing polar groups (e.g., hydroxy, amino, carboxyl, nitro, cyano, mercapto, and ketone groups) in its structure.

Examples of hydrocarbon polymers include dicyclopentadiene resins, styrene polymers, coumarone-indene resins, hydrogenated dicyclopentadiene resins, and the like. Among these, dicyclopentadiene resins, hydrogenated dicyclopentadiene resins, and the like are preferable.

It is preferable that the rubber composition of the present invention comprises no phenolic resins.

The amount of the component (d2) is preferably 5 to 40 parts by mass, more preferably 10 to 30 parts by mass,

and even more preferably 15 to 25 parts by mass, per 100 parts by mass of the component (a2) in the rubber composition.

In another preferred embodiment, the rubber composition of the present invention is a rubber composition comprising the following components (a3), (b3), (c3), and (d3), wherein the composition comprises the component (d3) in an amount of 0.8 to 2.2 parts by mass per 100 parts by mass of the component (a3). This rubber composition is excellent in wet grip performance and durability. This rubber composition 2 can be used for tires, belts (conveyor belts), vibration-proof rubber, seismic isolation rubber, hoses, and the like. In particular, the rubber composition is preferably used for tires.

Component (a3): a rubber component;

Component (b3): at least one compound selected from the group consisting of a compound represented by the following formula (1), a compound represented by the following formula (2), and salts of the compounds;

Component (c3): carbon black; and

Component (d3): sulfur;

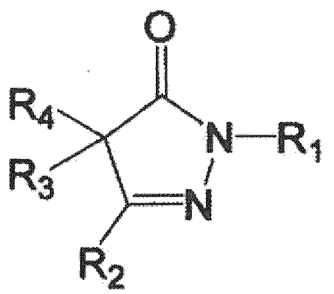

(1)

wherein $R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

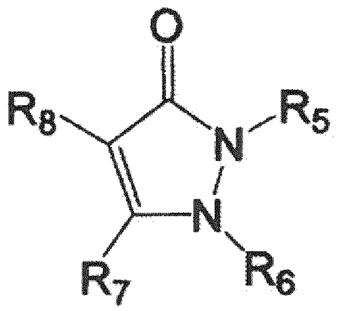

(2)

wherein $R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

The definitions of the components (a3), (b3), and (c3) are the same as those of the rubber component, compound (1) or compound (2), and carbon black described above.

The amount of the component (b3) is preferably 0.01 to 50 parts by mass, more preferably 0.05 to 30 parts by mass, and even more preferably 0.1 to 10 parts by mass, per 100 parts by mass of the component (a3) in the rubber composition.

The amount of the component (c3) is preferably 20 to 100 parts by mass, more preferably 30 to 80 parts by mass, and even more preferably 35 to 60 parts by mass, per 100 parts by mass of the component (a3) in the rubber composition.

The rubber composition of the present invention comprises sulfur in an amount of 0.8 to 2.2 parts by mass per 100 parts by mass of the rubber component. As sulfur, an oil-treated product or the like obtained by incorporating 2 to 10 mass% of oil commonly used in the rubber industry, such as naphthenic oil, into sulfur may be used. A sulfur content of less than 0.8 parts by mass per 100 parts by mass of the rubber component decreases hardness, whereas a sulfur content of more than 2.2 parts by mass per 100 parts by mass of the rubber component deteriorates abrasion resistance.

The amount of the component (d3) is preferably 0.5 to 2.2 parts by mass, and more preferably 1.0 to 2.0 parts by mass, per 100 parts by mass of the component (a3) in the rubber composition.

The rubber composition of the present invention preferably further comprises a vulcanization accelerator as component (e3).

The component (e3) is not particularly limited, and those usually used in the tire industry can be used. Examples include guanidine compounds, sulfenamide compounds, thiazole compounds, thiuram compounds, dithiocarbamic acid salt compounds, and the like. These may be used singly, or in a combination of two or more. Among these, sulfenamide compounds are preferable, and N-cyclohexyl-2-benzothiazolylsulfenamide (CBS) is particularly preferable.

The amount of the component (e3) is preferably 1.0 to 3.0 parts by mass, more preferably 1.5 to 2.5 parts by mass, and even more preferably 1.8 to 2.2 parts by mass, per 100 parts by mass of the component (a3) in the rubber composition.

It is also preferable that the rubber composition of the present invention is provided as a first rubber composition in addition to the rubber compositions described above, the first rubber composition comprising the following components (a4), (b4), and (c4), wherein the composition comprises a vulcanizing agent in an amount of 0 to 1 part by mass per 100 parts by mass of the component (a4). Further, the rubber composition of the present invention is also preferably provided as a second rubber composition obtained by adding a vulcanizing agent to the first rubber composition. The second rubber composition is preferable because it has excellent processability, and a tire obtained using the second rubber composition has improved tear strength. The second rubber composition can be used for tires, belts (conveyor belts), vibration-proof rubber, seismic isolation rubber, hoses, and the like. In particular, the second rubber composition is preferably used for tires, vibration-proof rubber, or hoses.

The first rubber composition of the present invention is a rubber composition that comprises the following components (a4), (b4), and (c4) and that comprises a vulcanizing agent in an amount of 0 to 1 part by mass per 100 parts by mass of the component (a4):

(a4) a rubber component;

(b4) at least one compound selected from the group consisting of a compound represented by the following formula (1), a compound represented by the following formula (2), and salts of the compounds; and

(c4) an antioxidant;

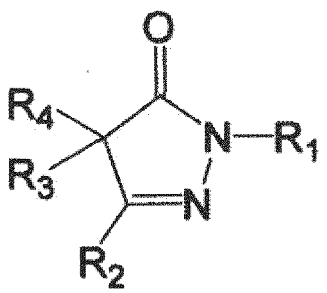
(1)

wherein $R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

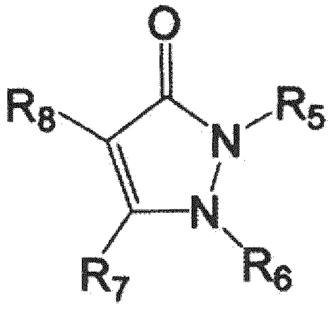
(2)

wherein $R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

The definitions of the components (a4) and (b4) are the same as those of the rubber component and compound (1) or compound (2) described above.

The amount of the component (b4) is preferably 0.01 to 50 parts by mass, more preferably 0.05 to 30 parts by mass, and even more preferably 0.1 to 10 parts by mass, per 100 parts by mass of the component (a4) in the first rubber composition.

The component (c4) in the first rubber composition of the present invention is an antioxidant. The antioxidant is not particularly limited. Examples include aromatic secondary amine antioxidants, amine-ketone antioxidants, monophenolic antioxidants, bisphenolic antioxidants, polyphenolic antioxidants, benzimidazole antioxidants, dithiocarbamic acid salt antioxidants, thiourea antioxidants, phosphorous acid antioxidants, organic thio acid antioxidants, sulfide antioxidants, special wax antioxidants, and the like. Among these, at least one member selected from aromatic secondary amine antioxidants, benzimidazole antioxidants, and special wax antioxidants is preferable. As such antioxidants, commercially available products can be suitably used.

Examples of aromatic secondary amine antioxidants include N-phenyl-1-naphthylamine, alkylated diphenylamine, octylated diphenylamine, 4,4'-bis(α,α-dimethylbenzyl)diphenylamine, p-(p-toluenesulfonylamide)diphenylamine, N,N'-di-2-naphthyl-p-phenylenediamine, N,N'-diphenyl-p-phenylenediamine, N-phenyl-N'-isopropyl-p-phenylenediamine, N-phenyl-N'-(1,3-dimethylbutyl)-p-phenylenediamine, N-phenyl-N'-(3-methacryloyloxy-2-hydroxypropyl)-p-phenylenediamine, and the like. Among these, N-phenyl-N'-(1,3-dimethylbutyl)-p-phenylenediamine is preferable.

Examples of amine-ketone antioxidants include 2,2,4-trimethyl-1,2-dihydroquincline polymers, 6-ethoxy-1,2-dihydro-2,2,4-trimethylquinoline, reaction products of diphenylamine and acetone, and the like. Among these, 6-ethoxy-1,2-dihydro-2,2,4-trimethylquinoline is preferable.

Examples of monophenolic antioxidants include 2,6-di-tert-butyl-4-methylphenol, mono(α-methylbenzyl)phenol, di(α-methylbenzyl)phenol, tri(α-methylbenzyl)phenol, and the like. Among these, 2,6-di-tert-butyl-4-methylphenol is preferable.

Examples of bisphenolic antioxidants include 2,2'-methylenebis(4-ethyl-6-tert-butylphenol), 2,2'-methylenebis(4 - methyl-6-tert-butylphenol), 4,4'-butylidenebis(3-methyl-6-tertbutylphenol), 4,4'-thiobis(3-methyl-6-tert-butylphenol), butylated reaction products of p-cresol and dicyclopentadiene, and the like. Among these, 2,2'-methylenebis(4-ethyl-6-tertbutylphenol) is preferable.

Examples of polyphenolic antioxidants include 2,5-di-tert-butylhydroquinone, 2,5-di-tert-amylhydroquinone, and the like. Among these, 2,5-di-tert-butylhydroquinone is preferable.

Examples of benzimidazole antioxidants include 2-mercaptobenzimidazole, 2-mercaptomethylbenzimidazole, zinc salt of 2-mercaptobenzimidazole, and the like. Among these, 2-mercaptobenzimidazole is preferable.

Examples of dithiocarbamic acid salt antioxidants include nickel dibutyldithiocarbamate and the like.

Examples of thiourea antioxidants include 1,3-bis(dimethylaminopropyl)-2-thiourea, tributylthiourea, and the like. Among these, 1,3-bis(dimethylaminopropyl)-2-thiourea is preferable.

Examples of phosphorous acid antioxidants include tris(nonylphenyl)phosphite and the like.

Examples of organic thio acid antioxidants include dilauryl thiodipropionate and the like.

Examples of sulfide antioxidants include bis(3,5-di-tert-butyl-4-hydroxybenzyl)sulfide and the like.

Among the above antioxidants, N-phenyl-N'-(1,3-dimethylbutyl)-p-phenylenediamine, which is an aromatic secondary amine antioxidant, 2,2,4-trimethyl-1,2-dihydroquinoline polymers, which are amine-ketone antioxidants, 2,6-di-tert-butyl-4-methylphenol, which is a monophenolic antioxidant, 2-mercaptobenzimidazole, which is a benzimidazole antioxidant, are preferable; N-phenyl-N'-(1,3-dimethylbutyl)-p-phenylenediamine, which is an aromatic secondary amine antioxidant, and 2-mercaptobenzimidazole, which is a benzimidazole antioxidant, are more preferable; and N-phenyl-N'-(1,3-dimethylbutyl)-p-phenylenediamine, which is an aromatic secondary amine antioxidant, is particularly preferable.

The amount of the component (c4) is preferably 0.5 to 10 parts by mass, more preferably 0.7 to 7 parts by mass, and even more preferably 1 to 5 parts by mass, per 100 parts by mass of the component (a4) in the first rubber composition.

The first rubber composition of the present invention comprises a vulcanizing agent in an amount of 0 to 1 part by mass per 100 parts by mass of the component (a4). It is preferable that the first rubber composition of the present invention comprises a vulcanizing agent in an amount of 0 to 0.5 parts by mass per 100 parts by mass of the component (a4), and it is particularly preferable that the first rubber composition of the present invention comprises no vulcanizing agents at all. Although it is particularly preferable that the amount of a vulcanizing agent is 0 parts by mass per 100 parts by mass of the component (a4) (the composition comprises no vulcanizing agents at all) as described above, the first rubber composition of the present invention may comprise a vulcanizing agent in an amount of 1 part by mass or less, for example, 0.001 parts by mass to 1 part by mass, per 100 parts by mass of the component (a4).

If the first rubber composition comprises a vulcanizing agent in an amount of more than 1 part by mass per 100 parts by mass of the component (a4), the tear strength of a tire, which is a final target product, is insufficient, and the processability during tire production deteriorates.

The second rubber composition of the present invention is a rubber composition obtained by adding a vulcanizing agent to the first rubber composition, and can be suitably used as a rubber composition for the production of a tire.

The vulcanizing agent contained in the second rubber composition of the present invention is not particularly

limited. Examples include sulfur, peroxides, quinone dioxime compounds, nitrosobenzene compounds, thio compounds, bismaleimide compounds, and the like. Among these, sulfur and peroxides are preferable. As such vulcanizing agents, commercially available products can be suitably used.

As sulfur, an oil-treated product or the like obtained by incorporating 2 to 10 mass% of oil commonly used in the rubber industry, such as naphthenic oil, into sulfur may be used.

Examples of peroxides include dicumyl peroxide, 2,5-dimethyl-2,5-di(tert-butyl-peroxy)hexane and the like. Among these, dicumyl peroxide is preferable.

Examples of quinone dioxime compounds include p-quinone dioxime, o,o'-dibenzoyl-p-quinone dioxime, and the like. Among these, p-quinone dioxime is preferable.

Examples of nitrosobenzene compounds include poly-p-dinitrosobenzene and the like.

Examples of thio compounds include 4,4'-dithiomorpholine and the like.

Examples of bismaleimide compounds include N,N' -m-phenylenebismaleimide, N,N'-m-phenylene biscitraconimide, and the like. Among these, N,N'-m-phenylenebismaleimide is preferable.

The amount of the vulcanizing agent is preferably 0.01 to 10 parts by mass, more preferably 0.05 to 7 parts by mass, and even more preferably 0.1 to 5 parts by mass, per 100 parts by mass of the component (a4) in the first rubber composition.

It is also preferable that the rubber composition of the present invention comprises the following components (a5), (b5), and (c5). This rubber composition is excellent in durability. Moreover, this rubber composition can be used for tires, belts (conveyor belts), vibration-proof rubber, seismic isolation rubber, hoses, and the like. In particular, the rubber composition is preferably used for vibration-proof rubber. The composition may comprise the following components:

(a5) diene rubber;
(b5) a pyrazolone compound represented by the following formula (1) or (2) (hereinafter also referred to as "pyrazolone compound (1)" or "pyrazolone compound (2)");
(c5) carbon black; and
(d4) a cross-linking agent;

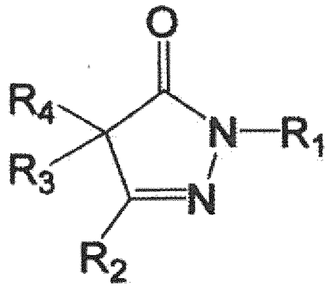
(1)

wherein $R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

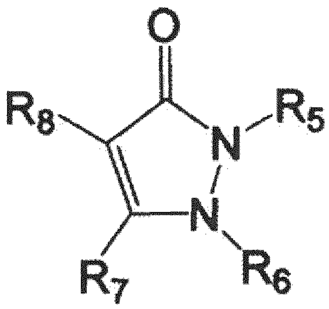
(2)

wherein $R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

Examples of the diene rubber include natural rubber (NR), synthetic diene rubber, a mixture of natural rubber and synthetic diene rubber, and the like. The natural rubber and synthetic diene rubber are as described above.

The pyrazolone compound (1) is the same as the compound (1) described above, and the pyrazolone compound (2) is the same as the compound (2) described above.

The definition of the component (c4) is the same as that of the carbon black described above.

The amount of the pyrazolone compound (1) and/or pyrazolone compound (2) is preferably 0.1 to 50 parts by mass, more preferably 0.1 to 20 parts by mass, and even more preferably 0.2 to 10 parts by mass, per 100 parts by mass of the component (a5) (diene rubber) in the rubber composition for vibration-proof rubber.

When the pyrazolone compound represented by the formula (1) and/or formula (2) is a powder, the volume mean diameter is not particularly limited. From the viewpoint of achieving a viscosity-reducing effect, the volume mean diameter is preferably 300 μm or less, more preferably 150 μm or less, and particularly preferably 75 μm or less.

The volume mean diameter can be determined as a particle diameter corresponding to 50% on the cumulative size distribution curve from the volume-based particle size distribution by using a particle size distribution analyzer based on laser optical diffraction or the like.

Further, from the viewpoint of the handling at the time of use and of reducing the ignition or explosion risk, the powder may be surface-treated with oil, resin, stearic acid, or the like, or the powder may be used by mixing with, for example, a filler, such as calcium carbonate, silica, and carbon black.

Adding the pyrazolone compound represented by the formula (1) or (2) to diene rubber enables the rubber composition to have vibration-proof performance and improves durability. Specifically, adding the pyrazolone compound represented by the formula (1) or (2) to diene rubber improves compression set while maintaining flex fatigue resistance of the rubber composition. Vibration-proof rubber manufactured (produced) from a rubber composition comprising such a pyrazolone compound (1) or pyrazolone compound (2) has vibration-proof performance and excellent durability.

The amount of the carbon black is preferably 2 to 100 parts by mass, more preferably 10 to 80 parts by mass, and even more preferably 10 to 50 parts by mass, per 100 parts by mass of the component (a5) (diene rubber).

Examples of the cross-linking agent include sulfur cross-linking agents, sulfur compound cross-linking agents, quinoid cross-linking agents, and maleimide cross-linking agents.

Examples of sulfur cross-linking agents include powdered sulfur, sublimed sulfur, highly dispersible sulfur, insoluble sulfur, precipitated sulfur, surface-treated sulfur, colloidal sulfur, sulfur chloride, sulfur monochloride, sulfur dichloride, and the like. These sulfur cross-linking agents may be used singly or in a combination of two or more. Among these, insoluble sulfur is preferable in terms of preventing bleeding.

Moreover, when a sulfur cross-linking agent is used, a cross-linking accelerator can also be used in combination.

Examples of cross-linking accelerators include hexamethylenetetramine, acetaldehyde ammonia, and like aldehyde ammonias; n-butylaldehyde-aniline condensation products, butylaldehyde-monobutylamine condensation products, heptaldehyde-aniline reaction products, tricrotonylidenetetramine, and like aldehyde amines; diphenylguanidine, di-o-tolylguanidine, 1-(o-tolyl)biguanide, di-ortho-tolylguanidine salt of dicatecholboric acid, and like guanidine salts; 2-mercaptoimidazoline and like imidazolines; 2-mercaptobenzothiazole, 2-mercaptothiazoline, dibenzothiazyl disulfide, zinc salt of 2-mercaptobenzothiazole, sodium salt of 2-mercaptobenzothiazole, cyclohexylamine salt of 2-mercaptobenzothiazole, 2-(2,4-dinitrophenylthio)benzothiazole, 2-(N,N-diethylthiocarbamoylthio)benzothiazole, 2-(4'-morpholinodithio)benzothiazole, 4-morphonyl-2-benzothiazyl disulfide, and like thiazoles; N-cyclohexyl-2-benzothiazyl sulfenamide, N,N-dicyclohexyl-2-benzothiazyl sulfenamide, N-oxydiethylene-2-benzothiazyl sulfenamide, N,N-diisopropyl-2-benzothiazyl sulfenamide, N-tert-butyl-2-benzothiazyl sulfonamide, and like sulfonamides; thiocarbamide, ethylene thiourea(2-mercaptoimidazoline), diethylthiourea, dibutylthiourea, mixed alkylthiourea, trimethylthiourea, dilaurylthiourea, and like thioureas; sodium dimethyldithiocarbamate, sodium diethyldithiocarbamate, sodium di-n-butylcarbamate, lead dimethyldithiocarbamate, lead diamyldithiocarbamate, zinc dimethyldithiocarbamate, zinc diamyldithiocarbamate, zinc diethyldithiocarbamate, zinc di-n-butyldithiocarbamate, zinc dibenzyldithiocarbamate, zinc N-pentamethylenedithiocarbamate, zinc ethylphenyldithiocarbamate, selenium dimethyldithiocarbamate, selenium diethyldithiocarbamate, tellurium diethyldithiocarbamate, cadmium diethyldithiocarbamate, copper dimethyldithiocarbamate, iron dimethyldithiocarbamate, bismuth dimethyldithiocarbamate, piperidine dimethyldithiocarbamate, pipecolin methylpentamethylenedithiocarbamate, activated dithiocarbamate, and like dithiocarbamic acid salts; tetramethylthiuram monosulfide, tetramethylthiuram disulfide, activated tetramethylthiuram disulfide, tetraethylthiuram disulfide, tetrabutylthiuram disulfide, N,N'-dimethyl-N,N'-diphenylthiuram disulfide, dipentamethylenethiuram disulfide, dipentamethylenethiuram tetrasulfide, mixed alkylthiuram disulfide, and like thiurams; sodium isopropylxanthate, zinc isopropylxanthate, zinc butylxanthate, and like xanthates; 4,4'-dithiodimorpholine, amino dialkyl dithiophosphate, zinc-o,o-n-butylphosphorodithioate, 3-mercaptoimidazoline-thione-2, thioglycolic acid ester, and the like. These vulcanization accelerators can be used singly or in a combination of two or more.

Examples of sulfur compound cross-linking agents include tetramethylthiuram monosulfide, tetramethylthiuram disulfide, tetraethylthiuram disulfide, tetrabutylthiuram disulfide, dipentamethylenethiuram disulfide, dipentamethylenethiuram tetrasulfide, dimorpholyl disulfide, 2-(4'-morpholinodithio)benzothiazole, and the like. Among these, tetramethylthiuram monosulfide is preferable in terms of the vulcanization rate. These sulfur compound cross-linking agents may be used singly or in a combination of two or more.

Examples of cross-linking accelerators used to improve the cross-linking rate for such a sulfur compound cross-linking agent include N-(tert-butyl)-2-benzothiazolesulfenamide, N-cyclohexyl-2-benzothiazyl sulfenamide, zinc dimethyldithiocarbamate, zinc dibutyldithiocarbamate, zinc diethyldithiocarbamate, cyclohexylamine salt of 2-mercaptobenzothiazole, and thiourea accelerators. These may be used singly or in a combination of two or more.

Examples of quinoid cross-linking agents include p-quinone dioxime, o,o'-dibenzoyl-p-quinone dioxime, tetrachloro-p-benzoquinone, poly-p-dinitrobenzene, and the like. Among these, o,o'-dibenzoyl-p-quinone dioxime is preferable in terms of the vulcanization rate. These quinoid cross-linking agents can be used singly or in a combination of two or more.

Examples of maleimide cross-linking agents include N,N'-1,3-phenylenebi smaleimide (another name: N,N'-m-phenylenebismaleimide), 1,3-bis(citraconimidomethyl)benzene, N,N'-1,2-phenylenebismaleimide, N,N'-1,4-phenylenebismaleimide, N,N'-(4,4'-diphenylmethane)bismaleimide, 2,2-bis [4-(4-maleimidophenoxy)phenyl]propane, bis(3-ethyl-5-methyl-4-maleimidophenyl]methane, and the like. Among these, N,N'-1,3-phenylenebismaleimide is preferable in terms of heat resistance. 1,3-Bis(citraconimidomethyl)benzene is also preferable in terms of preventing reversion. These maleimide cross-linking agents can be used singly or in a combination of two or more.

In the rubber composition for vibration-proof rubber of the present invention, as the component (d5) (cross-linking agent), sulfur cross-linking agents and sulfur compound cross-linking agents are preferable, and sulfur cross-linking agents are more preferable.

It is preferable that the rubber composition for vibration-proof rubber of the present invention comprises no organic peroxides as a cross-linking agent. When the composition comprises no organic peroxides as a cross-linking agent, good durability of the resulting vibration-proof rubber can be ensured.

The amount of the component (d5) (cross-linking agent) is preferably 0.1 to 20 parts by mass, more preferably 0.1 to 10 parts by mass, and even more preferably 0.1 to 5 parts by mass, per 100 parts by mass of the component (a4) (diene rubber) in the rubber composition for vibration-proof rubber.

Rubber Material

The present invention also includes an invention directed to a rubber material obtained by incorporating the compound (1) and/or compound (2) into the rubber component described above. In general, the compound (1) and compound (2) are both used as additives that can impart low heat build-up to the rubber material. When the compound (1) and/or compound (2) is incorporated into the rubber component, it is preferable that the compound (1) and/or compound (2) is uniformly incorporated into the rubber component.

Incorporating a compound into a rubber material to improve functionality generally decreases the processability of the rubber material. Thus, incorporating the compound (1) or (2) into the rubber component is also usually presumed to deteriorate the processability of the rubber component. However, the present inventor found that sufficient processability of the rubber composition can be ensured by preparing a rubber material by incorporating the compound (1) and/or compound (2) into the rubber component in advance, and preparing a rubber composition comprising the rubber material. Further, surprisingly, the inventor found that the rubber composition has extremely good low heat build-up.

Although it is unclear at this time what kind of phenomenon and/or reaction is specifically induced by incorporating the above compound into the rubber component so that they are both present, the compound is presumed to be bonded to the rubber component to produce some reaction product.

As a result, the rubber composition comprising the rubber material of the present invention has not only sufficient processability, but also extremely good low heat build-up.

The rubber component used in the rubber material of the present invention may be a rubber component that is the same as the rubber component described above. In particular, it is preferable to use diene rubber, and it is more preferable to use natural rubber.

The amount of the compound (1) or compound (2) is preferably 0.1 to 50 parts by mass, more preferably 0.1 to 20 parts by mass, and even more preferably 0.2 to 10 parts by mass, 100 parts by mass of the rubber component.

Master Batch

The present invention includes an invention directed to a master batch comprising a rubber component and at least one member selected from the group consisting of compounds represented by the formulas (1) and (2) described above and salts of the compounds.

The master batch of the present invention may be the rubber material of the present invention itself described above or may comprise other ingredients, such as antioxidants, silane coupling agents, zinc oxide, and vulcanization accelerators. However, it is preferable that the master batch does not comprise silica.

Rubber Compositions of Other Embodiments

As an embodiment other than the rubber compositions described above, the present invention also includes an invention directed to a rubber composition obtained by adding carbon black and/or an inorganic filler to a rubber material obtained by incorporating the compound (1) and/or compound (2) into the rubber component described above. Further, the present invention also includes an invention directed to a rubber composition comprising the master batch of the present invention and carbon black and/or an inorganic filler.

Incorporating the rubber material or master batch of the present invention in the rubber composition of the present invention ensures sufficient processability of the rubber composition, and also significantly improves the low heat build-up of the rubber composition.

The rubber composition of the present invention may comprise an additional rubber component, if necessary. The additional rubber component is not particularly limited as long as the objects of the present invention are not impaired, and may be the diene rubber described above or non-diene rubber. A wide variety of known non-diene rubber can be used.

Method for Producing Rubber Material

The rubber material of the present invention can be obtained by incorporating the compound (1) and/or compound (2) into the rubber component described above. More specifically, the rubber material of the present invention can be obtained by mixing (kneading) the rubber component and the compound (1) and/or compound (2).

When the rubber component and the compound (1) and/or compound (2) are mixed, the entire amount of each ingredient may be mixed at once, or it is also preferable that each ingredient is added in portions according to the intended purpose, such as viscosity adjustment, and mixed.

The mixing temperature is not particularly limited. Specifically, the mixing temperature is preferably 60 to 190°C, more preferably 70 to 160°C, and even more preferably 80 to 150°C. Setting the mixing temperature to 60°C or more improves workability during mixing, whereas setting the mixing temperature to 190°C or less prevents deterioration of the rubber component.

The time for mixing the rubber component and the compound (1) and/or compound (2) is preferably 10 seconds to 20 minutes, more preferably 30 seconds to 10 minutes, and even more preferably 60 seconds to 7 minutes. Setting the mixing time to 10 seconds or more enables the rubber component and the compound (1) and/or compound (2) to be uniformly mixed, whereas setting the mixing time to 20 minutes or less prevents a decrease in productivity.

The master batch of the present invention can also be produced by the same method as that for the rubber material of the present invention described above.

The rubber material, master batch, or rubber composition of the present invention can be used for tires (in particular, all-season tires and studless tires), hoses, belts (conveyor belts), vibration-proof rubber, seismic isolation rubber, and the like. The tires, hoses, belts (conveyor belts), vibration-proof rubber, and seismic isolation rubber can be produced by an ordinary method.

Method for Producing Rubber Composition

The method for producing the rubber composition of the present invention is not particularly limited. For example, the method for producing the rubber composition of the present invention comprises the steps of (A1) kneading raw material ingredients including a rubber component, the compound (1) or compound (2), and carbon black and/or an inorganic filler; and (B1) kneading the mixture obtained in step (A1) and a vulcanizing agent.

Step (A1)

Step (A1) is a step of kneading raw material ingredients including a rubber component, the compound (1) or compound (2), and carbon black and/or an inorganic filler. Step (A1) refers to the step before incorporating a vulcanizing agent.

In step (A1), other ingredients as mentioned above etc. can also be incorporated, if necessary.

The kneading method in step (A1) may be, for example, a method of kneading a composition comprising raw material ingredients including a rubber component, the compound (1) or compound (2), and carbon black and/or an inorganic filler. In this kneading method, the entire amount of each ingredient may be kneaded at once, or each ingredient may be added in portions according to the intended purpose, such as viscosity adjustment, and kneaded. Alternatively, after kneading a rubber component, and carbon black and/or an inorganic filler, the compound (1) or compound (2) may be added and kneaded; or, after kneading a rubber component and the compound (1) or compound (2), carbon black and/or an inorganic filler may be added and kneaded. To uniformly disperse each ingredient, the kneading may be performed repeatedly.

Another example of the kneading method in step (A1) is a two-step kneading method comprising the steps of (A1-1) kneading a rubber component, and the compound (1) or compound (2); and (Al-2) kneading the mixture obtained in step (A1-1) and raw material ingredients including carbon black and/or an inorganic filler.

The temperature of mixing the rubber composition in step (A1) is not particularly limited. For example, the upper limit of the temperature of the rubber composition is preferably 100 to 190°C, more preferably 110 to 175°C, even more preferably 120 to 170°C.

The mixing time in step (A1) is not particularly limited. For example, the mixing time is preferably 10 seconds to 20 minutes, more preferably 30 seconds to 10 minutes, and even more preferably 1 to 8 minutes.

In step (A1-1), the temperature of mixing the rubber component and the compound (1) or compound (2) is preferably 60 to 190°C, more preferably 70 to 160°C, and even more preferably 80 to 150°C. This is because a mixing temperature lower than 60°C does not allow the reaction to proceed, whereas a mixing temperature of more than 190°C accelerates deterioration of the rubber.

The mixing time in step (A1-1) is preferably 10 seconds to 20 minutes, more preferably 30 seconds to 10 minutes, and even more preferably 60 seconds to 7 minutes. When the mixing time is shorter than 10 seconds, the reaction does not proceed sufficiently, whereas a mixing time of more than 20 minutes lowers the productivity.

In step (A1-2), the temperature of mixing the mixture obtained in step (A1-1), and the carbon black and/or inorganic filler is not particularly limited. For example, the upper limit of the temperature of the mixture is preferably 100 to 190°C, more preferably 130 to 175°C, and even more preferably 110 to 170°C.

The mixing time in step (A1-2) is not particularly limited. For example, the mixing time is preferably 10 seconds to 20 minutes, more preferably 30 seconds to 10 minutes, and even more preferably 1 to 8 minutes.

In step (A1), the amount of the compound (1) or compound (2) is not particularly limited. For example, the amount of the compound (1) or compound (2) is 0.1 to 50 parts by mass, preferably 0.1 to 20 parts by mass, and more preferably 0.2 to 10 parts by mass, per 100 parts by mass of the rubber component.

Step (B1)

Step (B1) is a step of mixing the mixture obtained in step (A1) and a vulcanizing agent. Step (B1) refers to the final stage of kneading.

In step (B1), a vulcanization accelerator etc. can also be added, if necessary.

Step (B1) can be performed under heating conditions. The heating temperature in step (B1) is not particularly limited and is, for example, preferably 60 to 140°C, more preferably 80 to 120°C, and even more preferably 90 to 120°C.

The mixing (or kneading) time is not particularly limited. For example, the mixing (or kneading) time is preferably 10 seconds to 20 minutes, more preferably 30 seconds to 10 minutes, and even more preferably 60 seconds to 5 minutes.

Before proceeding with the process to step (B1) from step (A1), it is preferable to reduce the temperature by 30°C or more from the temperature after completion of the preceding step, and then allow the process to proceed to the following step (step (B1)).

In the method for producing the rubber composition of the present invention, various ingredients usually incorporated in the rubber composition, such as stearic acid, vulcanization accelerators such as zinc oxide, and antioxidants, may be added in step (A1) or (B1), if necessary.

The rubber composition of the present invention may be mixed or kneaded using a Banbury mixer, a roll, an intensive mixer, a kneader, a single-screw extruder, a twin-screw extruder, or the like. In an extrusion step, the resulting mixture is then extruded and processed to form, for example, a tread member or a sidewall member. Subsequently, the member is attached and molded in a usual manner using a tire molding machine to form a green tire. The green tire is heated under pressure in a vulcanizing machine to obtain a tire.

Method for Producing Rubber Composition of Another Embodiment

In the method for producing the rubber composition of another embodiment of the present invention, it is preferable to add and mix carbon black and/or an inorganic filler after mixing (kneading) a rubber component and the compound (1) and/or compound (2). A specific example of the method is a two-step production method comprising the steps of ($\alpha$) mixing (kneading) a rubber component and the compound (1) and/or compound (2), and ($\beta$) mixing (kneading) the mixture obtained in step (A) and raw material ingredients including carbon black and/or an inorganic filler.

The temperature of mixing the rubber component and the compound (1) and/or compound (2) in step ($\alpha$) is preferably 60 to 190°C, more preferably 70 to 160°C, and even more preferably 80 to 150°C. Setting the mixing temperature to 60°C or more allows for uniform mixing, whereas setting the mixing temperature to 190°C or less prevents deterioration of the rubber.

The mixing time in step ($\alpha$) is preferably 10 seconds to 20 minutes, more preferably 30 seconds to 10 minutes, and even more preferably 60 seconds to 7 minutes. Setting the mixing time to 10 seconds or more allows for uniform

mixing, whereas setting the mixing time to 20 minutes or less prevents a decrease in productivity.

In step (β), the temperature of mixing the mixture obtained in step (α), and the carbon black and/or inorganic filler is not particularly limited. For example, the upper limit of the temperature of the mixture is preferably 100 to 190°C, more preferably 130 to 175°C, and even more preferably 110 to 170°C.

The mixing time in step (β) is not particularly limited. For example, the mixing time is preferably 10 seconds to 20 minutes, more preferably 30 seconds to 10 minutes, and even more preferably 1 to 8 minutes.

The amount of the compound (1) and/or compound (2) is not particularly limited. For example, the amount of the compound (1) and/or compound (2) is 0.1 to 50 parts by mass, preferably 0.1 to 20 parts by mass, and more preferably 0.2 to 10 parts by mass, per 100 parts by mass of the rubber component.

Addition of Other Ingredients

In the method for producing the rubber composition of the present invention (including rubber compositions of other embodiments), various ingredients usually incorporated in the rubber composition, such as stearic acid, zinc oxide, and antioxidants, may be added in each step, if necessary.

The other ingredients may be added in any one of the steps or may be added in each of the steps.

Molding Method for Rubber Composition

The rubber composition of the present invention (including rubber compositions of other embodiments) may be mixed or kneaded using a Banbury mixer, a roll, an intensive mixer, a kneader, a single-screw extruder, a twin-screw extruder, or the like. In an extrusion step, the resulting mixture is then extruded and processed to form, for example, a tread member or a sidewall member. Subsequently, the member is attached and molded in a usual manner using a tire molding machine to form a green tire. The green tire is heated under pressure in a vulcanizing machine to obtain a tire.

Further, the methods for producing the first rubber composition and the second rubber composition are described below.

Method for Producing First Rubber Composition: Step (A4)

The method for producing the first rubber composition of the present invention comprises step (A4) of mixing the components (a4), (b4), and (c4) described above.

In the present specification, the term "mixing" includes not only the meaning of merely mixing, but also the meaning of kneading.

In this mixing method, the entire amount of each ingredient may be mixed at once, or each ingredient may be added in portions according to the intended purpose, such as viscosity adjustment, and mixed. Alternatively, after mixing the components (a4) and (b4), the component (c4) may be added and mixed; or after mixing the components (a4) and (c4), the component (b4) may be added and mixed. To uniformly disperse each ingredient, the mixing may be performed repeatedly. Moreover, filler master batch rubber in which a filler is added to rubber in advance by a wet method and/or a dry mixing method may be used.

The temperature of mixing the first rubber composition in step (A4) is not particularly limited. For example, the temperature of the first rubber composition is preferably 100 to 190°C, more preferably 110 to 175°C, and even more preferably 120 to 170°C.

The mixing time in step (A4) is not particularly limited. For example, the mixing time is preferably 10 seconds to 20 minutes, more preferably 30 seconds to 10 minutes, and even more preferably 1 to 8 minutes.

In the method for producing the first rubber composition of the present invention, other ingredients usually incorporated in the rubber composition, such as carbon black, inorganic fillers, stearic acid, and zinc oxide, may be added in step (A4), if necessary.

In particular, when carbon black and/or an inorganic filler is added to the first rubber composition of the present invention, and the mixture is mixed, the carbon black and/or inorganic filler may be mixed with the total amount of the components (a4), (b4), and (c4), or each ingredient may be added in portions according to the intended purpose, such as viscosity adjustment, and mixed.

Another example of the kneading method when carbon black and/or an inorganic filler is added to the first rubber composition of the present invention, and the mixture is mixed, is a two-step kneading method comprising the steps of (A4-1) kneading the components (a4), (b4), and (c4); and (A4-2) kneading the first rubber composition of the present invention obtained in step (A4-1) and raw material ingredients including carbon black and/or an inorganic filler.

The temperature of mixing the first rubber composition in step (A4-1) is not particularly limited. For example, the temperature of the first rubber composition is preferably 100 to 190°C, more preferably 110 to 175°C, and even more preferably 120 to 170°C.

The mixing time in step (A4-1) is not particularly limited. For example, the mixing time is preferably 10 seconds to 20 minutes, more preferably 30 seconds to 10 minutes, and even more preferably 1 to 8 minutes.
In step (A4-2), the temperature of mixing the first rubber composition of the present invention obtained in step (A4-1), and the carbon black and/or inorganic filler is not particularly limited. For example, the temperature of the mixture is preferably 100 to 190°C, more preferably 130 to 175°C, and even more preferably 110 to 170°C.
The mixing time in step (A4-2) is not particularly limited. For example, the mixing time is preferably 10 seconds to 20 minutes, more preferably 30 seconds to 10 minutes, and even more preferably 1 to 8 minutes.
Moreover, a master batch in which the components (a4) and (b4) are mixed in any proportion in advance may be used.
The amount of the component (b4) of the master batch of the first rubber composition is preferably 0.01 to 200 parts by mass, and more preferably 0.1 to 100 parts by mass, per 100 parts by mass of the component (a4).
The mixing method when the first rubber composition comprises 1 part by mass or less of a vulcanizing agent per 100 parts by mass of the component (a4) is not particularly limited.

Method for Mixing First Rubber Composition

The first rubber composition of the present invention is preferably mixed using a Banbury mixer, a roll, an intensive mixer, a kneader, a single-screw extruder, a twin-screw extruder, or the like.

Method for Producing Second Rubber Composition: Step (B4)

The method for producing the second rubber composition of the present invention comprises step (B4) of mixing the first rubber composition obtained in step (A4) and a vulcanizing agent. It is particularly preferable to incorporate a vulcanizing agent in step (B4) after producing a first rubber composition that comprises a rubber component, a specific pyrazolone compound, and an antioxidant, but does not comprise a vulcanizing agent.
Thus, the method for producing the second rubber composition of the present invention comprises the steps of (A4) mixing the components (a4), (b4), and (c4); and (B4) mixing the first rubber composition obtained in step (A4) and a vulcanizing agent.
In the method for producing the second rubber composition of the present invention, an antioxidant may also be added in step (B4).
In the method for producing the second rubber composition of the present invention, other ingredients usually incorporated in the rubber composition, such as vulcanization accelerators, stearic acid, and zinc oxide, may be added in step (B4), if necessary.
The other ingredients may be added in either step (A4) or step (B4), or may be added in step (A4) and step (B4).
Step (B4) can be performed under heating conditions. The heating temperature in step (B4) is not particularly limited and is, for example, preferably 60 to 140°C, more preferably 80 to 120°C, and even more preferably 90 to 120°C.
The mixing time is not particularly limited. For example, the mixing time is preferably 10 seconds to 20 minutes, more preferably 30 seconds to 10 minutes, and even more preferably 60 seconds to 5 minutes.
Before proceeding with the process to step (B4) from step (A4), it is preferable to reduce the temperature by 30°C or more from the temperature after completion of the preceding step, and then allow the process to proceed to the following step (step (B4)).

Mixing and Molding Methods for Second Rubber Composition

The second rubber composition of the present invention is preferably mixed using a Banbury mixer, a roll, an intensive mixer, a kneader, a single-screw extruder, a twin-screw extruder, or the like. In an extrusion step, the resulting mixture is then extruded and processed to form, for example, a tread member or a sidewall member. Subsequently, the member is preferably attached and molded in a usual manner using a tire molding machine to form a green tire. The green tire is heated under pressure in a vulcanizing machine to obtain a tire.

Tire

The tire of the present invention is a tire produced using the rubber composition of the present invention (including rubber compositions of other embodiments).
Among the rubber compositions of the present invention, the rubber compositions described above, the rubber composition comprising the components (a2), (b2), and (c2), the rubber composition comprising the components (a3), (b3), (c3), and (d3), wherein the composition comprises the component (d3) in an amount of 0.8 to 2.2 parts by mass per 100 parts by mass of the component (a3), and the rubber composition comprising the components (a4), (b4), and

(c4), wherein the composition comprises a vulcanizing agent in an amount of 0 to 1 part by mass per 100 parts by mass of the component (a4), are preferable for producing tires.

Examples of the tire of the present invention include pneumatic tires (such as radial-ply tires and bias tires), solid tires, and the like.

The use of the tire is not particularly limited. Examples include passenger car tires, heavy-duty tires, such as construction equipment tires and truck and bus tires, motorcycle tires, all-season tires, studless tires, and the like. Among these, the tire of the present invention is preferably used as passenger car tires.

The shape, structure, size, and material of the tire of the present invention are not particularly limited, and can be appropriately selected according to the purpose.

In the tire of the present invention, the above rubber composition is used particularly for at least one member selected from tread, sidewall, bead area, belt, carcass, and shoulder portions.

Among these, according to a most preferred embodiment, a tire tread portion of a pneumatic tire is formed using the rubber composition.

The "tread" is a portion that has a tread pattern and comes into direct contact with the road surface. The tread refers to a tire casing portion for protecting the carcass and preventing wear and flaws, and refers to a cap tread that constitutes the grounding part of a tire and/or to a base tread that is disposed inside the cap tread.

The "sidewall" refers to, for example, a portion from the lower side of a shoulder portion to a bead portion of a pneumatic radial-ply tire. Sidewall portions protect the carcass and are bent the most when the vehicle drives.

The "bead area" portions function to anchor both ends of the carcass cords and simultaneously hold the tire to the rim. Beads are composed of bundles of high-carbon steel.

The "belt" refers to a reinforcing band disposed between the carcass and the tread of a radial structure in the circumferential direction. The belt tightens the carcass like a hoop of a barrel to enhance the rigidity of the tread.

The "carcass" refers to a cord layer portion that forms the framework of the tire. The carcass plays a role in bearing the load, impact, and filled air pressure applied to the tire.

The "shoulder" refers to a shoulder portion of the tire. Shoulder portions play a role in protecting the carcass.

The tire of the present invention can be produced by methods known in the field of tires. The tire may be filled with ordinary air, or air having an adjusted oxygen partial pressure; or an inert gas, such as nitrogen, argon, or helium.

The tire of the present invention has low heat build-up and reduced rolling resistance, thus achieving lower fuel consumption in automobiles. Further, even the rubber composition highly filled with carbon black and/or an inorganic filler can have excellent low heat build-up, thus providing a fuel-efficient tire with high kinematic performance.

Vibration-proof Rubber

When the rubber composition for vibration-proof rubber of the present invention (including rubber compositions of other embodiments) is used for vibration-proof rubber, vibration-proof rubber can be produced by curing the rubber composition of the present invention. Curing of the rubber composition can be achieved, for example, by heating the rubber composition. The curing conditions (cross-linking conditions) for curing the rubber composition are not particularly limited. The temperature is preferably 140 to 180°C, and more preferably 150 to 170°C, and the curing time is preferably 1 to 120 minutes. The vibration-proof rubber obtained by curing the rubber composition of the present invention can be used as vibration-proof rubber used in an environment where durability is required, such as vibration-proof rubber used for engine mounts, strut mounts, body mounts, suspension bushes, etc. of automobiles.

Among the rubber compositions of the present invention, the rubber composition comprising the components (a2), (b2), and (c2), the rubber composition comprising the components (a4), (b4), and (c4), wherein the composition comprises a vulcanizing agent in an amount of 0 to 1 part by mass per 100 parts by mass of the component (a4), and the rubber composition comprising the components (a5), (b5), and (c5) are preferable for producing vibration-proof rubber.

Applications Other Than Tires and Vibration-proof Rubber

The rubber composition of the present invention (including rubber compositions of other embodiments) can be used for belts (conveyor belts), seismic isolation rubber, hoses, and the like in addition to the above applications, i.e., tires and vibration-proof rubber.

Additive for Imparting Low Heat Build-up to Rubber Component

The additive for imparting low heat build-up to a rubber component of the present invention (also referred to as the "low heat build-up agent") comprises the compound (1) or compound (2).

Adding the compound (1) or compound (2) to a rubber component can impart low heat build-up to the rubber component. Thus, a rubber composition comprising the compound (1) or compound (2) can exhibit excellent low heat

build-up. Further, a tire produced from a rubber composition comprising such a compound (1) or compound (2) has reduced rolling resistance, thus exhibiting excellent low fuel consumption performance.

Additive for Imparting Tear Strength to Rubber Component

The additive for imparting tear strength to a rubber component of the present invention (hereinafter also referred to as "the additive of the present invention") comprises a compound represented by the following formula (1) or a salt thereof (hereinafter also referred to as "the compound (1)"), or a compound represented by the following formula (2) or a salt thereof (hereinafter also referred to as "the compound (2)"). The additive is preferably the compound (1) and/or compound (2) (i.e., the compound (1) and/or compound (2) itself), but may also comprise other ingredients.

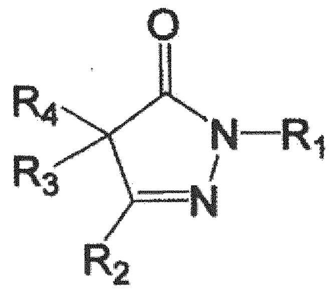
(1)

wherein $R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents;

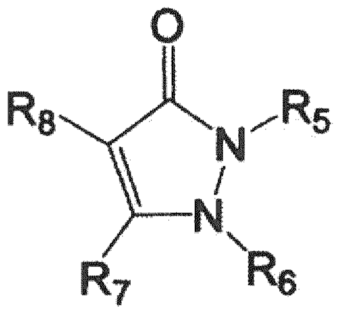
(2)

wherein $R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

The additive for imparting excellent tear strength to a rubber component can also be referred to as "tear strength imparting agent for rubber" or "tear strength enhancer for rubber."

Examples

The present invention is described below more specifically with reference to Production Examples and Examples. However, the following examples are only illustrative and are not intended to limit the present invention to these.

Production Example 1: Production of 3-(naphthalen-2-yl)-1H-pyrazol-5(4H)-one

8.5 g (52.1 mmol) of carbonyldiimidazole and 60 mL of dehydrated tetrahydrofuran were placed in a 100-mL four-necked recovery flask and stirred at room temperature. Subsequently, 7.5 g (43.6 mmol) of 2-naphthoic acid was added to this mixture and the resulting mixture was stirred overnight at room temperature. The obtained reaction mixture is hereinafter referred to as "reaction mixture 1."

14.8 g (87.0 mmol) of potassium monoethylmalate, 210 mL of dehydrated acetonitrile, and 18.3 mL (132.4 mmol) of triethylamine were placed in a 500-mL four-necked flask and stirred under ice cooling. Subsequently, 10.4 g (109.2 mmol) of magnesium chloride was then added to this mixture, and the resulting mixture was stirred at room

temperature for 4 hours. Reaction mixture 1 was added dropwise to the obtained reaction mixture and stirred at room temperature overnight.

After this reaction mixture was concentrated, 125 mL of toluene was added to the obtained residue. The resulting mixture was washed with 70 mL of 4M hydrochloric acid and 70 mL of water. The organic layer was dried over anhydrous magnesium sulfate and then concentrated under reduced pressure. This concentrate was purified by silica gel column chromatography (n-hexane: ethyl acetate = 4:1 (volume ratio)) to obtain a colorless and transparent liquid.

After 2.2 mL (44.8 mmol) of hydrazine monohydrate was added to this liquid, 2.0 mL (35.0 mmol) of acetic acid was added dropwise, and the resulting mixture was refluxed for 4 hours. This reaction mixture was returned to room temperature and 30 mL of diisopropyl ether was added. The precipitated crystals were filtered to obtain 8.22 g of 3-(naphthalen-2-yl)-1H-pyrazol-5(4H)-one as a white solid.
Melting point: 204°C
$^{1}$H-NMR (300 MHz, $d_6$-DMSO, $\delta$ ppm) :
11.94 (1H, br), 8.20-8.21 (1H, J=1.2 Hz, d), 7.82-7.96 (4H, m), 7.47-7.56 (2H, m), 6.02 (1H, s)
$^{13}$C-NMR (500 MHz, $d_6$-DMSO, $\delta$ ppm) :
168.13, 160.95, 143.83, 133.06, 132.39, 128.30, 127.90, 127.62, 126.55, 126.06, 123.28, 123.14, 87.10

Production Example 2: Production of 3-(furan-2-yl)-1H-pyrazol-5(4H)-one

8.7 g (53.5 mmol) of carbonyldiimidazole and 60 mL of dehydrated tetrahydrofuran were added to a 100-mL eggplant flask and the resulting mixture was stirred at room temperature. Subsequently, 5.0 g (45.0 mmol) of 2-furancarboxylic acid was added to this mixture and stirred overnight at room temperature. The obtained reaction mixture is hereinafter referred to as "reaction mixture 2."

14.8 g (87.0 mmol) of potassium monoethylmalate, 200 mL of dehydrated acetonitrile, and 18.3 mL (132.4 mmol) of triethylamine were placed in a 500-mL four-necked flask and stirred under ice cooling. Subsequently, 10.4 g (109.2 mmol) of magnesium chloride was added to this mixture, and the resulting mixture was stirred at room temperature for 4 hours. The reaction mixture 2 was added dropwise to this reaction mixture. The resulting mixture was stirred overnight at room temperature.

After the obtained reaction solution was concentrated, 120 mL of chloroform was added. The resulting mixture was washed with 70 mL of 4M hydrochloric acid and 70 mL of water. The organic layer was dried over anhydrous magnesium sulfate and then concentrated under reduced pressure to obtain a colorless transparent liquid.

2.5 mL (50.7 mmol) of hydrazine monohydrate was added to the obtained liquid, and 2.0 mL (35.0 mmol) of acetic acid was added dropwise. The resulting mixture was refluxed for 4 hours. The obtained reaction mixture was returned to room temperature, and 30 mL of diisopropyl ether was added. The precipitated crystals were filtered to obtain 47 g of 3-(furan-2-yl)-1H-pyrazol-5(4H)-one as a white solid.
Melting point: 231°C
$^{1}$H-NMR (300 MHz, $d_6$-DMSO, $\delta$ ppm) :
11.79 (2H, br), 7.68-7.69 (1H, m), 6.69 (1H, J=3.3 Hz, d), 6.55 (1H, J=1.8, 1.5, 1.8 Hz, dd), 5.69 (1H, s)
$^{13}$C-NMR (500 MHz, $d_6$-DMSO, $\delta$ ppm) :
160.23, 146.16, 142.24, 135.84, 111.50, 105.77, 85.88

Production Example 3: Production of 3-phenyl-1H-pyrazol-5(4H)-one

25 g (0.13 mol) of ethyl benzoylacetoacetate and 25 mL of ethanol were placed in a 100-mL four-necked flask and stirred. While the resulting mixture was cooled in a water bath, 6.5 mL (0.13 mol) of hydrazine monohydrate was added dropwise. After the resulting mixture was stirred at room temperature for 4 hours, the obtained white solid was separated by filtration, washed with 50 mL of a mixture of water to methanol of 1:1 (volume ratio), and dried to obtain 18.8 g of 3-phenyl-1H-pyrazol-5(4H)-one as a white solid.
Melting point: 236°C
$^{1}$H-NMR (500 MHz, $d_6$-DMSO, $\delta$ ppm) :
12.03 (1H, br), 9.69 (1H, br), 7.66 (2H, m), 7.39 (2H, m), 7.30 (1H, m), 5.88 (1H, s)
$^{13}$C-NMR (500 MHz, $d_6$-DMSO, $\delta$ ppm) :
161.00, 143.48, 130.48, 128.73, 127.71, 124.73, 86.87

Production Example 4: Production of 3-propyl-1H-pyrazol-5(4H)-one

24.8 g (0.16 mol) of methyl 3-oxohexanoate and 260 mL of ethanol were placed in a 500-mL four-necked flask and stirred. While the mixture was cooled in an ice bath, 8.6 mL (0.18 mol) of hydrazine monohydrate was added dropwise. The resulting mixture was stirred under ice cooling for 2 hours and then heated under reflux for 2 hours. The

reaction mixture was allowed to cool to room temperature. The obtained white solid was separated by filtration, washed with 50 mL of a mixture of water to methanol of 1:1 (volume ratio), and dried to obtain 13.2 g of 3-propyl-1H-pyrazol-5(4H)-one as a white solid.
Melting point: 205 to 206°C
$^{1}$H-NMR (500 MHz, $d_6$-DMSO, δ ppm) :
11.11 (1H, br), 9.42 (1H, br), 5.23 (1H, s), 2.41 (2H, J=7.5 Hz, t), 1.54 (2H, m), 0.88 (3H, J=7.3 Hz, t)
$^{13}$C-NMR (500 MHz, $d_6$-DMSO, δ ppm) :
160.87, 144.04, 87.93, 27.67, 21.93, 13.57

Production Example 5: Production of 4,4'-(phenylmethylene)bis(5-methyl-1H-pyrazol-3(2H)-one)

1 L of water, 10 g (0.10 mol) of 3-methyl-5-pyrazolone, 5.4 g (0.05 mol) of benzaldehyde, and 0.73 g (2.5 mmol) of sodium dodecyl sulfate were placed in a 2-L four-necked flask and stirred at room temperature for 30 minutes and then refluxed for 1 hour. This reaction mixture was stirred with cooling in an ice bath for 30 minutes. The obtained solid was then separated by filtration, washed with 500 mL of water, and dried to obtain 12.1 g of 4,4'-(phenylmethylen)bis(5-methyl-1H-pyrazol-3(2H)-one) as a pale yellow solid.
Melting point: 230-232°C
$^{1}$H-NMR (500 MHz, $d_6$-DMSO, δ ppm) :
11.31 (4H, br), 7.20 (2H, m), 7.12 (3H, m), 4.81 (1H, s), 2.07 (6H, s)
$^{13}$C-NMR (500 MHz, $d_6$-DMSO, δ ppm) :
161.24, 143.33, 139.73, 127.67, 127.47, 125.35, 104.21, 32.72, 10.34

Production Example 6: Production of 4,4'-(4-hydroxyphenylmethylene)bis(5-methyl-1H-pyrazol-3(2H)-one)

2.5 L of water, 25.0 g (0.26 mol) of 3-methyl-5-pyrazolone, 15.6 g (0.13 mol) of 4-hydroxybenzaldehyde, and 1.84 g of sodium dodecyl sulfate (6.4 mmol) were placed in a 3-L four-necked flask. The resulting mixture was stirred at room temperature for 30 minutes and then refluxed for 1 hour. This reaction mixture was stirred with cooling in an ice bath for 30 minutes. The obtained solid was then separated by filtration, washed with 2.5 L of water, and dried to obtain 33.5 g of 4,4'-(4-hydroxyphenylmethylene)bis(5-methyl-1H-pyrazol-3(2H)-one) as a pale yellow solid.
Melting point: 262-264°C
$^{1}$H-NMR (500 MHz, $d_6$-DMSO, δ ppm) :
11.22 (4H, br), 9.03 (1H, s), 6.91 (2H, J=8.5 Hz, d), 6.59 (2H, J=8.5 Hz, d), 4.71 (1H, s), 2.06 (6H, s)
$^{13}$C-NMR (500 MHz, $d_6$-DMSO, δ ppm) :
161.12, 155.03, 139.67, 133.44, 128.27, 114.42, 104.84, 31.91, 10.35

Production Example 7: Production of 4,4'-(4-nitrophenylmethylene)bis(5-methyl-1H-pyrazol-3(2H)-one)

2.5 L of water, 25.0 g (0.26 mol) of 3-methyl-5-pyrazolone, 19.3 g (0.13 mol) of 4-nitrobenzaldehyde, and 1.84 g (6.4 mmol) of sodium dodecyl sulfate were placed in a 3-L four-necked flask. The resulting mixture was stirred at room temperature for 30 minutes and then refluxed for 1 hour. This reaction mixture was stirred with cooling in an ice bath for 30 minutes. The obtained solid was then separated by filtration, washed with 500 mL of methanol, and dried to obtain 37.8 g of 4,4'-(4-nitrophenylmethylene)bis(5-methyl-1H-pyrazol-3(2H)-one) as a pale yellow solid.
Melting point: 300-302°C
$^{1}$H-NMR (500 MHz, $d_6$-DMSO, δ ppm) :
11.40 (4H, br), 8.12 (2H, J=8.8 Hz, d), 7.38 (2H, J=8.8 Hz, d), 4.98 (1H, s), 2.10 (6H, s)
$^{13}$C-NMR (500 MHz, $_{d6}$-DMSO, δ ppm) :
160.82, 151.71, 145.56, 139.71, 128.76, 122.94, 103.24, 32.95, 10.28

Production Example 8: Production of 4-[(dimethylamino)methylidene]-3-methyl-1H-pyrazol-5(4H)-one

300 mL of xylene, 14.7 g (0.15 mol) of 3-methyl-5-pyrazolone, and 21.6 g (0.18 mol) of N,N-dimethylformamide dimethylacetal were placed in a 500-mL four-necked flask and stirred at 110°C overnight. After the reaction mixture was cooled to room temperature, the obtained solid was separated by filtration, washed with 300 mL of toluene, and dried to obtain 20.3 g of 4-[(dimethylamino)methylidene]-3-methyl-1H-pyrazol-5(4H)-one as a yellow solid.
Melting point: 158 to 159°C
$^{1}$H-NMR (500 MHz, $d_6$-DMSO, δ ppm) :
10.30 (1H, br), 7.27 (1H, s), 3.75 (3H, s), 3.26 (3H, s), 1.97 (3H, s)
$^{13}$C-NMR (500 MHz, $d_6$-DMSO, δ ppm) :

164.77, 152.66, 149.66, 97.13, 46.74, 42.44, 13.35

Production Example 9: Production of 4-{[4-dimethylamino]phenyl}methylidene}-3-methyl-1H-pyrazol-5(4H)-one

2.7 L of ethanol, 27.0 g (0.28 mol) of 3-methyl-5-pyrazolone, 44.4 g (0.30 mol) of 4-dimethylaminobenzaldehyde, and 4.7 g (55 mmol) of piperidine were placed in a 3-L four-necked flask and refluxed for 3 hours. After this reaction mixture was stirred overnight at room temperature, the obtained solid was separated by filtration and dried to obtain 46.3 g of 4-{[4-dimethylaminolphenyl}methylidene}-3-methyl-1H-pyrazol-5(4H)-one as a pale yellow solid.
Melting point: 164°C
$^1$H-NMR (500 MHz, $d_7$-DMF, $\delta$ ppm) :
10.91 (1H, br), 8.70 (2H, J=9.3 Hz, d), 7.45 (1H, s), 6.83 (2H, J=9.3 Hz, d), 3.15 (6H, s), 2.18 (3H, s)
$^{13}$C-NMR (500 MHz, $d_7$-DMF, $\delta$ ppm) :
166.74, 153.84, 150.55, 146.23, 136.92, 122.49, 120.74, 111.43, 39.58, 12.98

Production Example 10: Production of 3-undecyl-1H-pyrazol-5(4H)-one

2.8 g (17.4 mmol) of carbonyldiimidazole and 20 mL of chloroform were placed in a 50-mL recovery flask and stirred at room temperature. Subsequently, 2.9 g (14.5 mmol) of lauric acid was added to this mixture and stirred at room temperature overnight. The obtained reaction mixture is hereinafter referred to as "reaction mixture 3."
4.9 g (30.0 mmol) of potassium monoethylmalonate, 70 mL of dehydrated acetonitrile, and 6.1 mL (44.1 mmol) of triethylamine were placed in a 200-mL four-necked flask. The resulting mixture was stirred under ice cooling. Subsequently, 3.5 g (36.4 mmol) of magnesium chloride was added to this mixture and the resulting mixture was stirred at room temperature for 4 hours. The reaction mixture 3 was added dropwise to this reaction mixture and stirred overnight at room temperature.
The obtained reaction mixture was concentrated, and 42 mL of chloroform was added. The resulting mixture was washed with 48 mL of 2M hydrochloric acid and 48 mL of water, and the organic layer was dried over anhydrous magnesium sulfate and then concentrated under reduced pressure.
20 mL of ethanol and 0.7 mL (14.9 mmol) of hydrazine monohydrate were added thereto and 0.7 mL (11.7 mmol) of acetic acid was added dropwise. The resulting mixture was refluxed for 4 hours. This reaction mixture was returned to room temperature and 10 mL of diisopropyl ether was added. The precipitated crystals were separated by filtration to obtain 2.83 g of 3-undecyl-1H-pyrazol-5(4H)-one as a white solid.
Melting point: 188°C
$^1$H-NMP, (300 MHz, $d_6$-DMSO, $\delta$ ppm) :
11.05 (1H, br), 9.45 (1H, br), 5.21 (1H, s), 2.39-2.44 (2H, J=7.5 Hz, t), 1.48-1.53 (2H, m), 1.30 (16H, s), 0.83-0.88 (3H, m) $^{13}$C-NMR (500 MHz, $d_6$-DMSO, $\delta$ ppm) :
160.85, 144.20, 87.84, 31.27, 29.01, 28.98, 28.95, 28.71, 28.68, 28.62, 28.57, 25.60, 22.06, 13.90

Production Example 11: Production of 4-(2-hydroxyethyl)-3-methyl-1H-pyrazol-5(4H)-one

24.3 g (0.19 mol) of $\alpha$-acetyl-$\gamma$-butyrolactone and 24 mL of ethanol were placed in a 100-mL four-necked flask and stirred. While this mixture was cooled in an ice bath, 9.7 mL (0.20 mol) of hydrazine monohydrate was added dropwise, and the resulting mixture was stirred at room temperature for 3 hours. The precipitated solid was separated by filtration, washed with 50 mL of isopropanol, and dried to obtain 23.8 g of 4-(2-hydroxyethyl)-3-methyl-1H-pyrazol-5(4H)-one as a white solid.
Melting point: 182°C
$^1$H-NMR (500 MHz, $d_7$-DMF, $\delta$ ppm) :
10.62 (2H, br), 3.78 (2H, J=7.0 Hz, t), 3.66 (1H, br), 2.69 (2H, J=7.0 Hz, t), 2.31 (3H, s)
$^{13}$C-NMR (500 MHz, $d_7$-DMF, $\delta$ ppm) :
160.88, 137.87, 98.61, 62.29, 26.18, 9.66

Production Example 12: Production of 4-benzyl-3-methyl-1H-pyrazol-5(4H)-one

24.5 g (0.11 mol) of ethyl 2-benzylacetoacetate and 25 mL of ethanol were placed in a 100-mL four-necked flask and stirred. While this mixture was cooled in an ice bath, 5.7 mL (0.12 mol) of hydrazine monohydrate was added dropwise. The resulting mixture was then stirred at room temperature for 3 hours. The precipitated solid was separated by filtration, washed with 50 mL of a mixture of water to methanol of 1:1 (volume ratio), and dried to obtain 15.6 g of 4-benzyl-3-methyl-1H-pyrazol-5(4H)-one as a white solid.
Melting point: 228 to 229°C

$^1$H-NMR (500 MHz, $d_6$-DMSO, δ ppm) :
11.08 (1H, br), 9.46 (1H, br), 7.24 (2H, m), 7.14 (3H, m), 3.55 (2H, s), 2.01 (3H, s)
$^{13}$C-NMR (500 MHz, $d_6$-DMSO, δ ppm) :
159.58, 141.90, 136.82, 128.06, 127.96, 125.37, 99.94, 27.28, 9.94

Production Example 13: Production of 1-phenyl-1H-pyrazol-3(2H)-one

10 g (62 mmol) of 1-phenyl-3-pyrazolidone and 150 mL of N,N-dimethylformamide were placed in a 1-L four-necked flask and stirred. 317 mg (3.2 mmol) of copper (I) chloride was added to this mixture, and the resulting mixture was stirred overnight in an open system. 750 mL of water was added to the reaction mixture, and the resulting mixture was stirred with cooling in an ice bath for 30 minutes. The obtained solid was then separated by filtration, washed with 750 mL of water, and dried to obtain 6.1 g of 1-phenyl-1H-pyrazol-3(2H)-one as a pale brown solid. Melting point: 152°C
$^1$H-NMR (500 MHz, $d_6$-DMSO, δ ppm) :
10.22 (1H, br), 8.22 (1H, s), 7.68 (2H, m), 7.42 (2H, m), 7.18 (1H, m), 5.80 (1H, s)
$^{13}$C-NMR (500 MHz, $d_6$-DMSO, δ ppm) :
162.64, 139.79, 129.29, 128.37, 124.59, 116.76, 94.47

Production Example 14: Production of 4-methyl-2,3-diazospiro[4.4]non-3-en-1-one

7.2 g (62.0 mmol) of methyl acetoacetate, 23.1 g (75 mmol) of 1,4-diiodobutane, 42.9 g (310 mmol) of potassium carbonate, and 220 mL of dimethyl sulfoxide were placed in a 500-mL four-necked flask and stirred at room temperature overnight. After 220 mL of water was added to the reaction mixture and stirred for 10 minutes, the organic layer obtained by extraction with 300 mL of isopropyl ether was washed twice with 200 mL of water. The organic layer was dried over sodium sulfate, then concentrated and column-purified (hexane:ethyl acetate = 50:1 (volume ratio)) to obtain 6.5 g (36 mmol) of an intermediate.

The obtained intermediate and 6 mL of ethanol were mixed. While the mixture was cooled in an ice bath, 1.8 mL (39 mmol) of hydrazine monohydrate was added. The resulting mixture was stirred overnight at 60°C. The solid obtained by concentrating the reaction mixture was washed with 40 mL of a mixture of hexane to ethyl acetate of 5:1 (volume ratio) to obtain 3.8 g of 4-methyl-2,3-diazospiro[4.4]non-3-en-1-one as a white solid.
Melting point: 83-84°C
$^1$H-NMR (500 MHz, $d_6$-DMSO, δ ppm) :
10.77 (1H, s), 1.92 (3H, s), 1.86-1.66 (8H, m)
$^{13}$C-NMR (500 MHz, $d_6$-DMSO, δ ppm) :
181.64, 163.37, 55.78, 33.53, 26.48, 13.34

Production Example 15: Production of 4,5,6,7-tetrahydro-2H-indazol-3(3aH)-one

24.5 g (0.14 mol) of ethyl 2-oxocyclohexanecarboxylate and 24 mL of ethanol were placed in a 100-mL four-necked flask and mixed. While cooling the resulting mixture in an ice bath, 7.3 mL (0.15 mol) of hydrazine monohydrate was added dropwise thereto. After the resulting mixture was stirred at 80°C for 3 hours, the mixture was stirred for 30 minutes with cooling in an ice bath. The reaction mixture was then filtered, washed with 50 mL of a mixture of water to methanol of 1:1 (volume ratio), and dried to obtain 17.3 g of 4,5,6,7-tetrahydro-2H-indazol-3(3aH)-one as a white solid.
Melting point: 286-288°C
$^1$H-NMR (500 MHz, $d_6$-DMSO, δ ppm) :
10.50 (1H, br), 9.66 (1H, br), 2.43 (2H, J=5.7 Hz, t), 2.22 (2H, J=5.7 Hz, t), 1.67-1.62 (4H, m)
$^{13}$C-NMR (500 MHz, $d_6$-DMSO, δ ppm) :
158.30, 139.62, 98.41, 22.86, 22.27, 21.26, 18.88

Production Example 16: Production of 1,3-diphenyl-1H-pyrazol-5 (4H) -one

50.0 g (0.26 mol) of ethyl benzoyl acetate, 28.1 g (0.26 mol) of phenylhydrazine, 4.5 mL (0.08 mol) of acetic acid, and 500 mL of water were placed in a 1-L four-necked flask and mixed. After this mixture was cooled at 100°C for 1 hour, the mixture was stirred for 30 minutes while cooling in an ice bath. The obtained solid was then separated by filtration, washed with 1 L of a mixture of water to methanol of 1:1 (volume ratio), and dried to obtain 58.0 g of 1,3-diphenyl-1H-pyrazol-5(4H)-one as a pale orange solid.
Melting point: 137-138°C
$^1$H-NMR (500 MHz, $d_6$-DMSO, δ ppm) :
11.81 (1H, br), 7.83 (4H, m), 7.49 (2H, m), 7.42 (2H, m), 7.35-7.28 (2H, m), 6.02 (1H, s)

$^{13}C$-NMR (500 MHz, $d_6$-DMSO, δ ppm) :
153.73, 149.52, 138.85, 133.41, 128.87, 128.51, 127.78, 125.65, 125.04, 121.11, 85.05

Examples 1-28 and Comparative Examples 1-8: Production of Rubber Compositions

The components shown in step (A1) of Tables 1 to 4 below were mixed in the proportions (parts by weight) shown therein and kneaded using a Banbury mixer. After the obtained mixture was allowed to rest until the temperature of the mixture was reduced to 60°C or less, the components shown in step (B1) of Tables 1 to 4 below were added in the proportions (parts by weight) shown therein and kneaded while controlling the maximum temperature of the mixture to not more than 70°C, thus producing a rubber composition.

Examples 29 to 31 and Comparative Examples 9 to 10: Production of Rubber Compositions

The components shown in step (A1-1) of Table 5 below were mixed in the proportions (part by mass) shown therein and kneaded for 3 minutes while adjusting the number of rotations of the mixer so that the mixture was maintained at a constant temperature of 100°C, thus producing a master batch. Subsequently, the components shown in step (A1-2) of Table 6 below were added in the proportions shown therein and kneaded with a Banbury mixer. After the obtained mixture was allowed to rest until the temperature of the mixture was reduced to 60°C or less, the components shown in step (B1) of Table 6 were added in the proportions (parts by weight) shown therein and kneaded while adjusting the number of rotations of the mixer so that the maximum temperature of the mixture was not more than 70°C, thus producing a rubber composition.

Examples 1-31 and Comparative Examples 1-10: Low Heat Build-up (tan δ index) Test

The tan δ of the rubber compositions obtained in the following Examples 1 to 31 and Comparative Examples 1 to 10 was measured using a viscoelasticity measuring instrument (produced by Metravib) at a temperature of 40°C, a dynamic strain of 5%, and a frequency of 15 Hz. For comparison, rubber compositions (Comparative Examples 1 to 10) were prepared using the same components and the same production methods as in the Examples except that no compound was added. The tan δ value of each of the Comparative Examples was defined as 100. The low heat build-up index was calculated according to the following formula. A smaller low heat build-up index value indicates lower heat build-up and smaller hysteresis loss.

Formula: Low heat build-up index = (Tan δ of the rubber composition of one of Examples 1 to 16) / (Tan δ of Comparative Example 1) x 100

Formula: Low heat build-up index = (Tan δ of the rubber composition of each of Examples 17 to 20) / (Tan δ of Comparative Example 2) x 100

Formula: Low heat build-up index = (Tan δ of the rubber composition of Example 21) / (Tan δ of Comparative Example 3) x 100

Formula: Low heat build-up index = (Tan δ of the rubber composition of Example 22) / (Tan δ of Comparative Example 4) x 100

Formula: Low heat build-up index = (Tan δ of the rubber composition of Example 23) / (Tan δ of Comparative Example 5) x 100

Formula: Low heat build-up index = (Tan δ of the rubber composition of Example 24) / (Tan δ of Comparative Example 6) x 100

Formula: Low heat build-up index = (Tan δ of the rubber compositions of Examples 25 and 26) / (Tan δ of Comparative Example 7) x 100

Formula: Low heat build-up index = (Tan δ of the rubber compositions of Examples 27 and 28) / (Tan δ of Comparative Example 8) x 100

Formula: Low heat build-up index = (Tan δ of the rubber compositions of Examples 29 and 30) / (Tan δ of Comparative Example 9) x 100

Formula: Low heat build-up index = (Tan δ of the rubber composition of Example 31) / (Tan δ of Comparative Example 10) x 100

Table 1

| Components of the rubber composition (parts by weight) | | Examples | | | | | | | | | | | | | | | | Comparative Examples |
|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 1 |
| Step A1 | NR[*1] | 100 | 100 | 100 | 100 | 100 | 100 | 100 | 100 | 100 | 100 | 100 | 100 | 100 | 100 | 100 | 100 | 100 |
| | CB[*2] | 55 | 55 | 55 | 55 | 55 | 55 | 55 | 55 | 55 | 55 | 55 | 55 | 55 | 55 | 55 | 55 | 55 |
| | Antioxidant[*3] | 2 | 2 | 2 | 2 | 2 | 2 | 2 | 2 | 2 | 2 | 2 | 2 | 2 | 2 | 2 | 2 | 2 |
| | Wax[*5] | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | Zinc oxide[*5] | 4 | 4 | 4 | 4 | 4 | 4 | 4 | 4 | 4 | 4 | 4 | 4 | 4 | 4 | 4 | 4 | 4 |
| | Stearic acid[*5] | 2 | 2 | 2 | 2 | 2 | 2 | 2 | 2 | 2 | 2 | 2 | 2 | 2 | 2 | 2 | 2 | 2 |
| | Compound A[*7] | 1 | | | | | | | | | | | | | | | | |
| | Compound B[*8] | | 1 | | | | | | | | | | | | | | | |
| | Compound C[*9] | | | 1 | | | | | | | | | | | | | | |
| | Compound D[*10] | | | | 1 | | | | | | | | | | | | | |
| | Compound E[*11] | | | | | 1 | | | | | | | | | | | | |
| | Compound F[*12] | | | | | | 1 | | | | | | | | | | | |
| | Compound G[*13] | | | | | | | 1 | | | | | | | | | | |
| | Compound H[*1A] | | | | | | | | 1 | | | | | | | | | |
| | Compound I[*15] | | | | | | | | | 1 | | | | | | | | |
| | Compound J[*16] | | | | | | | | | | 1 | | | | | | | |
| | Compound K[*17] | | | | | | | | | | | 1 | | | | | | |

(continued)

| Components of the rubber composition (parts by weight) | | Examples | | | | | | | | | | | | | | | | Comparative Examples |
|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 1 |
| | Compound L*18 | | | | | | | | | | | | 1 | | | | | |
| | Compound M*19 | | | | | | | | | | | | | 1 | | | | |
| | Compound N*20 | | | | | | | | | | | | | | 1 | | | |
| | Compound O*21 | | | | | | | | | | | | | | | 1 | | |
| | Compound P*22 | | | | | | | | | | | | | | | | 1 | |
| Step B1 | Vulcanization accelerator*23 | 1.2 | 1.2 | 1.2 | 1.2 | 1.2 | 1.2 | 1.2 | 1.2 | 1.2 | 1.2 | 1.2 | 1.2 | 1.2 | 1.2 | 1.2 | 1.2 | 1.2 |
| | Sulfur*24 | 1 | 1 | 1 | 1 | 1 | 1 | 1 | 1 | 1 | 1 | 1 | 1 | 1 | 1 | 1 | 1 | 1 |
| Low heat build-up index | | 74 | 81 | 81 | 82 | 83 | 82 | 84 | 92 | 91 | 89 | 90 | 90 | 85 | 90 | 92 | 92 | 100 |

Table 2

| Components of the rubber composition (parts by weight) | | Examples | | | | Comparative Examples |
|---|---|---|---|---|---|---|
| | | 17 | 18 | 19 | 20 | 2 |
| Step A1 | IR*25 | 100 | 100 | 100 | 100 | 100 |
| | CB*2 | 50 | 50 | 50 | 50 | 50 |
| | Antioxidant *3 | 2 | 2 | 2 | 2 | 2 |
| | Wax*4 | 1.5 | 1.5 | 1.5 | 1.5 | 1.5 |
| | Zinc oxide*5 | 3 | | 3 | | 3 |
| | Stearic acid*6 | 2 | 2 | 2 | 2 | 2 |
| | Compound B*8 | 1 | 1 | 1 | 1 | |
| Step B1 | Vulcanization accelerator*23 | 1.2 | 1.2 | 1.2 | 1.2 | 1.2 |
| | Sulfur*24 | 2 | 2 | 2 | 2 | 2 |
| | Zinc oxide*5 | | 3 | | 3 | |
| | Compound B*8 | | | 1 | 1 | |
| Low heat build-up index | | 91 | 87 | 89 | 87 | 100 |

Table 3

| Components of the rubber composition (parts by weight) | | Examples | | | | Comparative Examples | | | |
|---|---|---|---|---|---|---|---|---|---|
| | | 21 | 22 | 23 | 24 | 3 | 4 | 5 | 6 |
| Step A1 | SBR*26 | 20 | 50 | 80 | 100 | 20 | 50 | 80 | 100 |
| | NR*1 | 80 | 50 | 20 | | 80 | 50 | 20 | |
| | CB*2 | 50 | 50 | 50 | 50 | 50 | 50 | 50 | 50 |
| | Antioxidant *3 | 2 | 2 | 2 | 2 | 2 | 2 | 2 | 2 |
| | Wax*4 | 1.5 | 1.5 | 1.5 | 1.5 | 1.5 | 1.5 | 1.5 | 1.5 |
| | Zinc oxide*5 | 3 | 3 | 3 | 3 | 3 | 3 | 3 | 3 |
| | Stearic acid*6 | 2 | 2 | 2 | 2 | 2 | 2 | 2 | 2 |
| | Compound B*8 | 1 | 1 | 1 | 1 | | | | |
| Step B1 | Vulcanization accelerator*23 | 1.2 | 1.2 | 1.2 | 1.2 | 1.2 | 1.2 | 1.2 | 1.2 |
| | Sulfur*24 | 2 | 2 | 2 | 2 | 2 | 2 | 2 | 2 |
| Low heat build-up index | | 88 | 89 | 87 | 85 | 100 | 100 | 100 | 100 |

Table 4

| Components of the rubber composition (parts by weight) | | Examples | | | | Comparative Examples | |
|---|---|---|---|---|---|---|---|
| | | 25 | 26 | 27 | 28 | 7 | 8 |
| Step A1 | BR*26 | 60 | 60 | 40 | 40 | 60 | 40 |
| | NR*1 | 40 | 40 | 60 | 60 | 40 | 60 |
| | CB*2 | 50 | 50 | 50 | 50 | 50 | 50 |
| | Antioxidant *3 | 4 | 4 | 4 | 4 | 4 | 4 |

(continued)

| Components of the rubber composition (parts by weight) | | Examples | | | | Comparative Examples | |
|---|---|---|---|---|---|---|---|
| | | 25 | 26 | 27 | 28 | 7 | 8 |
| | Wax*4 | 2 | 2 | 2 | 2 | 2 | 2 |
| | Zinc oxide*5 | 3 | 1.5 | 3 | 1.5 | 3 | 3 |
| | Stearic acid*6 | 1.5 | 1.5 | 1.5 | 1.5 | 1.5 | 1.5 |
| | Compound B*8 | 1 | 1 | 1 | 1 | | |
| Step B1 | Vulcanization accelerator*23 | 1.2 | 1.2 | 1.2 | 1.2 | 1.2 | 1.2 |
| | Sulfur*24 | 2 | 2 | 2 | 2 | 2 | 2 |
| | Zinc oxide*5 | | 1.5 | | 1.5 | | |
| Low heat build-up index | | 94 | 95 | 88 | 89 | 100 | 100 |

Table 5

| Components of the rubber composition (parts by weight) | | Master batch No. | | | |
|---|---|---|---|---|---|
| | | 1 | 2 | 3 | 4 |
| Step A1-1 | NR*1 | 100 | 100 | 100 | 100 |
| | Zinc oxide*5 | | 3 | | 3 |
| | Compound B*8 | 1 | 1 | | |

Table 6

| Components of the rubber composition (parts by weight) | | Examples | | | Comparative Examples | |
|---|---|---|---|---|---|---|
| | | 29 | 30 | 31 | 9 | 10 |
| Step A1-2 | Master batch 1*28 | 101 | 101 | | | |
| | Master batch 2*29 | | | 101 | | |
| | Master batch 3*30 | | | | 101 | |
| | Master batch 4*31 | | | | | 101 |
| | CB*2 | 50 | 50 | 50 | 50 | 50 |
| | Antioxidant*3 | 2 | 2 | 2 | 2 | 2 |
| | Wax*4 | 1.5 | 1.5 | 1.5 | 1.5 | 1.5 |
| | Zinc oxide*5 | 3 | | | 3 | |
| | Stearic acid*6 | | | | | |
| Step B1 | Vulcanization accelerator*23 | 1.2 | 1.2 | 1.2 | 1.2 | 1.2 |
| | Sulfur*24 | 2 | 2 | 2 | 2 | 2 |
| | Zinc oxide*5 | | 3 | | | |

(continued)

| Components of the rubber composition (parts by weight) | Examples | | | Comparative Examples | |
|---|---|---|---|---|---|
| | 29 | 30 | 31 | 9 | 10 |
| Low heat build-up index | 80 | 65 | 74 | 100 | 110 |

*1: NR (natural rubber); produced by Guangken Rubber Co., Ltd., TSR-20
*2: CB (carbon black); produced by Cabot Co., Ltd., N234
*3: Antioxidant (N-phenyl-N'-(1,3-dimethylbutyl)-p-phenylenediamine); produced by Kemai Chemical Co., Ltd.
*4: Wax; produced by Rhein Chemie Rheinau GmbH, Antilux 111
*5: Zinc oxide; produced by Dalian Zinc Oxide Co., Ltd.
*6: Stearic acid; produced by Sichuan Tianyu Grease Chemical Co., Ltd.
*7: Compound A; produced by Tokyo Chemical Industry Co., Ltd., 5-pyrazolone
*8: Compound B; produced by Otsuka Chemical Co., Ltd., 3-methyl-5-pyrazolone
*9: Compound C; 3-(naphthalen-2-yl)-1H-pyrazol-5(4H)-one produced in Production Example 1
*10: Compound D; 3-(furan-2-yl)-1H-pyrazol-5(4H)-one produced in Production Example 2
*11: Compound E; 3-phenyl-1H-pyrazol-5(4H)-one produced in Production Example 3
*12: Compound F; 3-propyl-1H-pyrazole-5(4H)-one produced in Production Example 4
*13: Compound G; 4,4'-(phenylmethylene)bis(5-methyl-1H-pyrazol-3(2H)-one) produced in Production Example 5
*14: Compound H; 4,4'-(4-hydroxyphenylmethylene)bis(5-methyl-1H-pyrazol-3(2H)-one) produced in Production Example 6,
*15: Compound I; 4,4'-(4-nitrophenylmethylene)bis(5-methyl-1H-pyrazol-3(2H)-one) produced in Production Example 7)
*16: Compound J; 4-[(dimethylamino)methylidene]-3-methyl-1H-pyrazol-5(4H)-one produced in Production Example 8
*17: Compound K; 4-{[4-dimethylamino]phenyl}methylidene}-3-methyl-1H-pyrazol-5(4H)-one produced in Production Example 9
*18: Compound L; 3-undecyl-1H-pyrazol-5(4H)-one produced in Production Example 10
*19: Compound M; 4-(2-hydroxyethyl)-3-methyl-1H-pyrazol-5(4H)-one produced in Production Example 11
*20: Compound N; 4-benzyl-3-methyl-1H-pyrazol-5(4H)-one produced in Production Example 12
*21: Compound O; 1-phenyl-1H-pyrazol-3(2H)-one produced in Production Example 13
*22: Compound P; produced by Tokyo Chemical Industry Co., Ltd., 5-methyl-2-(4-nitrophenyl)-1H-pyrazol-3(2H)-one
*23: Vulcanization accelerator, N-(tert-butyl)-2-benzothiazole sulfenamide; produced by Sanshin Chemical Industry Co., Ltd., Sunseller NS-G
*24: Sulfur; produced by Shanghai Jinghai Chemical Co., Ltd.
*25: IR (isoprene rubber); produced by Nippon Zeon Corporation, Nipol IR2200
*26: SBR (styrene-butadiene rubber); produced by PetroChina Dushanzi Petrochemical Company, RC2557S
*27: BR (butadiene rubber); produced by Sinopec QiLu Petrochemical Co., Ltd., BR9000
*28: Masterbatch No. 1 shown in Table 5
*29: Masterbatch No. 2 shown in Table 5
*30: Masterbatch No. 3 shown in Table 5
*31: Masterbatch No. 4 shown in Table 5

Examples 32 to 61 and Comparative Examples 11 to 18: Production of Rubber Compositions

The components shown in step (A1) of Tables 7 to 9 below were mixed in the proportions (parts by weight) shown therein and kneaded using a Banbury mixer. After the obtained mixture was allowed to rest until the temperature of the mixture was reduced to 60°C or less, the components shown in step (B1) of Tables 7 to 9 below were added in the proportions (parts by weight) shown therein and kneaded while controlling the maximum temperature of the mixture to not more than 70°C, thus producing a rubber composition.

Tear Strength Test

The tear strength index of the rubber compositions obtained in Examples 32 to 61 and Comparative Examples 11 to 18 was determined in accordance with JIS K6252 by using crescent-shaped test pieces at room temperature and at a tensile speed of 500 mm/min. The tear strength of the rubber compositions of the Examples was calculated according to the following formula as indices relative to the obtained value of each of Comparative Examples 11 to 18, which was

defined as 100. A higher tear strength value indicates a better tear strength.

Formula: Tear strength = (Tear strength of the rubber composition of one of Examples 32 to 45) / (Tear strength of Comparative Example 11) x 100

Formula: Tear strength = (Tear strength of the rubber composition of one of Examples 46 to 53) / (Tear strength of Comparative Example 12) x 100

Formula: Tear strength = (Tear strength of the rubber composition of Example 54) / (Tear strength of Comparative Example 13) x 100

Formula: Tear strength = (Tear strength of the rubber composition of Example 55) / (Tear strength of Comparative Example 14) x 100

Formula: Tear strength = (Tear strength of the rubber composition of one of Examples 56 to 58) / (Tear strength of Comparative Example 15) x 100

Formula: Tear strength = (Tear strength of the rubber composition of Example 59) / (Tear strength of Comparative Example 16) x 100

Formula: Tear strength = (Tear strength of the rubber composition of Example 60) / (Tear strength of Comparative Example 17) x 100

Formula: Tear strength = (Tear strength of the rubber composition of Example 61) / (Tear strength of Comparative Example 18) x 100

Table 7

| | | Comparative Examples | Examples | | | | | | | | | | | | | |
|---|---|---|---|---|---|---|---|---|---|---|---|---|---|---|---|---|
| | | 11 | 32 | 33 | 34 | 35 | 36 | 37 | 38 | 39 | 40 | 41 | 42 | 43 | 44 | 45 |
| Step A1 | NR[*32] | 100 | 100 | 100 | 100 | 100 | 100 | 100 | 100 | 100 | 100 | 100 | 100 | 100 | 100 | 100 |
| | CB[*33] | 55 | 55 | 55 | 55 | 55 | 55 | 55 | 55 | 55 | 55 | 55 | 55 | 55 | 55 | 55 |
| | Antioxidant [*34] | 2 | 2 | 2 | 2 | 2 | 2 | 2 | 2 | 2 | 2 | 2 | 2 | 2 | 2 | 2 |
| | Wax[*35] | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 |
| | Zinc Oxide[*36] | 4 | 4 | 4 | 4 | 4 | 4 | 4 | 4 | 4 | 4 | 4 | 4 | 4 | | |
| | Stearic acid[*37] | 2 | 2 | 2 | 2 | 2 | 2 | 2 | 2 | 2 | 2 | 2 | 2 | 2 | 2 | 2 |
| | Compound 1[38] | | 1 | | | | | | | | | | | | 1 | |
| | Compound 2[*39] | | | 1 | | | | | | | | | | | | |
| | Compound 3[*40] | | | | 1 | | | | | | | | | | | |
| | Compound 4[*41] | | | | | | | | | | | | | | | 1 |
| | Compound 5[*42] | | | | | 1 | | | | | | | | | | |
| | Compound 6[*43] | | | | | | 1 | | | | | | | | | |
| | Compound 7[*44] | | | | | | | 1 | | | | | | | | |
| | Compound 8[*45] | | | | | | | | 1 | | | | | | | |
| | Compound 10[*47] | | | | | | | | | 1 | | | | | | |
| | Compound 12[*41] | | | | | | | | | | 1 | | | | | |
| | Compound 13[*50] | | | | | | | | | | | 1 | | | | |
| | Compound 14[*51] | | | | | | | | | | | | 1 | | | |
| | Compound 15[*52] | | | | | | | | | | | | | 1 | | |
| Step B1 | Zinc oxide[*36] | | | | | | | | | | | | | | 4 | 4 |
| | Stearic acid[*53] | 1.2 | 1.2 | 1.2 | 1.2 | 1.2 | 1.2 | 1.2 | 1.2 | 1.2 | 1.2 | 1.2 | 1.2 | 1.2 | 1.2 | 1.2 |
| | Sulfur[*54] | 1 | 1 | 1 | 1 | 1 | 1 | 1 | 1 | 1 | 1 | 1 | 1 | 1 | 1 | 1 |
| Tear strength | | 100 | 164 | 150 | 152 | 195 | 162 | 221 | 164 | 123 | 112 | 107 | 111 | 114 | 251 | 253 |

Table 8

| | | Comparative Examples | Examples | | | | | | | |
|---|---|---|---|---|---|---|---|---|---|---|
| | | 12 | 46 | 47 | 48 | 49 | 50 | 51 | 52 | 53 |
| Step A1 | NR*32 | 100 | 100 | 100 | 100 | 100 | 100 | 100 | 100 | 100 |
| | CB*33 | 55 | 55 | 55 | 55 | 55 | 55 | 55 | 55 | 55 |
| | Antioxidant *34 | 2 | 2 | 2 | 2 | 2 | 2 | 2 | 2 | 2 |
| | Wax*35 | 1.5 | 1.5 | 1.5 | 1.5 | 1.5 | 1.5 | 1.5 | 1.5 | 1.5 |
| | Zinc oxide*36 | 2 | 2 | 2 | 2 | 2 | 2 | 2 | 2 | 2 |
| | Stearic acid*37 | 2 | 2 | 2 | 2 | 2 | 2 | 2 | 2 | 2 |
| | Compound 1*38 | | 1 | | | | | | | |
| | Compound 2*39 | | | 1 | | | | | | |
| | Compound 3*40 | | | | 1 | | | | | |
| | Compound 4*41 | | | | | 1 | | | | |
| | Compound 5*42 | | | | | | 1 | | | |
| | Compound 8*45 | | | | | | | 1 | | |
| | Compound 9*46 | | | | | | | | 1 | |
| | Compound 11*48 | | | | | | | | | 1 |
| Step B1 | Zinc oxide*36 | 2 | 2 | 2 | 2 | 2 | 2 | 2 | 2 | 2 |
| | Stearic acid*53 | 1.2 | 1.2 | 1.2 | 1.2 | 1.2 | 1.2 | 1.2 | 1.2 | 1.2 |
| | Sulfur*54 | 1 | 1 | 1 | 1 | 1 | 1 | 1 | 1 | 1 |
| Tear strength | | 100 | 191 | 160 | 124 | 272 | 207 | 157 | 121 | 121 |

Table 9

| | | Comparative Examples | | | | | | Examples | | | | | | | |
|---|---|---|---|---|---|---|---|---|---|---|---|---|---|---|---|
| | | 13 | 14 | 15 | 16 | 17 | 18 | 54 | 55 | 56 | 57 | 58 | 59 | 60 | 61 |
| Step A1 | NR*[32] | 100 | 100 | 100 | 80 | 80 | 100 | 100 | 100 | 100 | 100 | 100 | 80 | 80 | 100 |
| | BR*[55] | | | | 20 | | | | | | | | 20 | | |
| | SBR*[56] | | | | | 20 | | | | | | | | 20 | |
| | CB*[33] | 30 | 60 | 45 | 45 | 45 | 45 | 30 | 60 | 45 | 45 | 45 | 45 | 45 | 45 |
| | Silica*[57] | | | | | | 10 | | | | | | | | 10 |
| | Antioxidant *[34] | 2 | 2 | 2 | 2 | 2 | 2 | 2 | 2 | 2 | 2 | 2 | 2 | 2 | 2 |
| | Wax*[35] | 1.5 | 1. 5 | 1.5 | 1.5 | 1.5 | 1.5 | 1.5 | 1.5 | 1.5 | 1.5 | 1.5 | 1.5 | 1.5 | 1.5 |
| | Zinc oxide*[36] | 4 | 4 | 4 | 4 | 4 | 4 | 4 | 4 | 4 | 2 | | 4 | 4 | 4 |
| | Stearic acid*[37] | 2 | 2 | 2 | 2 | 2 | 2 | 2 | 2 | 2 | 2 | 2 | 2 | 2 | 2 |
| | Compound 1*[38] | | | | | | | 1 | 1 | 1 | 1 | 1 | 1 | 1 | 1 |
| Step B1 | Zinc oxide*[36] | | | | | | | | | | 2 | 4 | | | |
| | Vulcanization accelerator*[53] | 1.2 | 1.2 | 1.2 | 1.2 | 1.2 | 1.2 | 1.2 | 1.2 | 1.2 | 1.2 | 1.2 | 1.2 | 1.2 | 1.2 |
| | Sulfur*[54] | 1 | 1 | 1 | 1 | 1 | 1 | 1 | 1 | 1 | 1 | 1 | 1 | 1 | 1 |

(continued)

| | Comparative Examples | | | | | | Examples | | | | | | | |
|---|---|---|---|---|---|---|---|---|---|---|---|---|---|---|
| | 13 | 14 | 15 | 16 | 17 | 18 | 54 | 55 | 56 | 57 | 58 | 59 | 60 | 61 |
| Tear strength | 100 | 100 | 100 | 100 | 100 | 100 | 209 | 238 | 183 | 195 | 162 | 219 | 240 | 149 |

*32: NR (natural rubber); Guangken Rubber Co., Ltd., TSR-20
*33: CB (carbon black); produced by Cabot Co., Ltd., N234
*34: Antioxydant (N-phenyl-N'-(1,3-dimethylbutyl)-p-phenylenediamine); produced by Kemai Chemical Co., Ltd.
*35: Wax; produced by Rhein Chemie Rheinau, Antilux 11,
*36: Zinc oxide; produced by Dalian Zinc Oxide Co., Ltd.
*37: Stearic acid; produced by Sichuan Tianyu Grease Chemical Co., Ltd.
*38: Compound 1; produced by Otsuka Chemical Co., Ltd., 3-methyl-5-pyrazolone
*39: Compound 2; 3-(naphthalen-2-yl)-1H-pyrazol-5(4H)-one produced in Production Example 1
*40: Compound 3; 3-(furan-2-yl)-1H-pyrazol-5(4H)-one produced in Production Example 2
*41: Compound 4; 3-phenyl-1H-pyrazol-5(4H)-one produced in Production Example 3
*42: Compound 5; 3-propyl-1H-pyrazol-5(4H)-one produced in Production Example 4
*43: Compound 6; 4-[(dimethylamino)methylidene]-3-methyl-1H-pyrazol-5(4H)-one produced in Production Example 8
*44: Compound 7; 4,5,6,7-tetrahydro-2H-indazol-3(3aH)-one produced in Production Example 15
*45: Compound 8; 4-{[4-dimethylamino]phenyl}methylidene}-3-methyl-1H-pyrazol-5(4H)-one produced in Production Example 9
*46: Compound 9; 3-undecyl-1H-pyrazol-5(4H)-one produced in Production Example 10
*47: Compound 10; 1-phenyl-1H-pyrazol-3(2H)-one produced in Production Example 13
*48: Compound 11; 4,4'-(phenylmethylene)bis(5-methyl-1H-pyrazol-3(2H)-one) produced in Production Example 5
*49: Compound 12; 4-(2-hydroxyethyl)-3-methyl-1H-pyrazol-5(4H)-one produced in Production Example 11
*50: Compound 13; 4,4'-(4-hydroxyphenylmethylene)bis(5-methyl-1H-pyrazol-3(2H)-one) produced in Production Example 6
*51: Compound 14; 1,3-diphenyl-1H-pyrazol-5(4H)-one produced in Production Example 16
*52: Compound 15; produced by Tokyo Chemical Industry Co., Ltd., 4-aminoantipyrine
*53: Vulcanization accelerator, N-(tert-butyl)-2-benzothiazole sulfenamide; produced by Sanshin Chemical Industry Co., Ltd., Sunseller NS-G
*54: Sulfur; produced by Shanghai Jinghai Chemical Co., Ltd.
*55: BR (butadiene rubber); produced by Sinopec QiLu Petrochemical Co., Ltd., BR9000
*56: SBR (styrene butadiene copolymer rubber); produced by Petro China Dushanzi Petrochemical Company, SBR2557S

Examples 62 to 64 and Comparative Examples 19 and 20: Production of Rubber Compositions

The components shown in step (A2) of Table 10 below were blended in the proportions (parts by weight) shown therein and mixed using a Banbury mixer. After the obtained mixture was allowed to rest until the temperature of the mixture was reduced to 60°C or less, the components shown in step (B2) of Table 10 were added in the proportions (parts by weight) shown therein and mixed while controlling the maximum temperature of the mixture to not more than 70°C, thus producing a rubber composition.

Table 10

| | | | Examples | | | Comparative Examples | |
|---|---|---|---|---|---|---|---|
| | | | 62 | 63 | 64 | 19 | 20 |
| Components of the rubber composition | Step A2 | NR*57 | 50 | 50 | 50 | 50 | 50 |
| | | SBR*56 | 30 | 30 | 30 | 30 | 30 |
| | | BR*59 | 20 | 20 | 20 | 20 | 20 |
| | | Carbon black*60 | 30 | 30 | 30 | 30 | 30 |
| | | Silica*61 | 30 | 30 | 30 | 30 | 30 |
| | | Silane coupling agent*62 (Si69) | 2.4 | 2.4 | 2.4 | 2.4 | 2.4 |
| | | Antioxidant*63 (6PPD) | 2 | 2 | 2 | 2 | 2 |
| | | Wax*64. | 1.5 | 1.5 | 1.5 | 1.5 | 1.5 |
| | | Zinc oxide*65 | 3 | 3 | 3 | 3 | 3 |
| | | Stearic acid*66 | 2 | 2 | 2 | 2 | 2 |
| | | Dicyclopentadiene resin*67 | | 10 | | | 10 |
| | | Phenolic resin*18 | | | 10 | | |
| | | Compound (b) -1*69 | 0.6 | 0.6 | 0.6 | | |
| | Step B2 | Vulcanization accelerator*70 | 2 | 2 | 2 | 2 | 2 |
| | | Vulcanization accelerator*71 | 0.6 | 0.6 | 0.6 | 0.6 | 0.6 |
| | | Sulfur*72 | 1.2 | 1.2 | 1.2 | 1.2 | 1.2 |

(continued)

| | Examples | | | Comparative Examples | |
|---|---|---|---|---|---|
| | 62 | 63 | 64 | 19 | 20 |
| Wet grip performance index | 128 | 136 | 112 | 100 | 109 |

The details of each component in Table 10 are as follows.
*57: NR (natural rubber); produced by Guangken Rubber Co., Ltd., TSR-20
*58: SBR (styrene-butadiene rubber); produced by JSR, SL563
*59: BR (butadiene rubber); produced by Ube Industries, Ltd. BR150B
*60: Carbon black; produced by Cabot Co., Ltd., N234
*61: Silica; produced by Tosoh Silica Corporation, Nipsil AQ
*62: Silane coupling agent; produced by Evonik Industries, Si69
*63: Antioxidant (N-Phenyl-N'-(1,3-dimethylbutyl)-p-phenylenediamine); produced by Keimai Chemical Co., Ltd.
*64: Wax; produced by Rhein Chemie Rheinau GmbH, Antilux 111
*65: Zinc oxide; produced by Dalian Zinc Oxide Co., Ltd.
*66: Stearic acid; produced by Sichuan Tianyu Grease Chemical Co., Ltd.
*67: Dicyclopentadiene resin: produced by Maruzen Petrochemical Co., Ltd., Marukarez M-890A
*68: Phenolic resin: produced by Asahi Yukizai Corporation, SP1006N
*69: Compound (b)-1; produced by Otsuka Chemical Co., Ltd., 3-methyl-5-pyrazolone
*70: Vulcanization accelerator: N-cyclohexyl-2-benzothiazolylsulfeneamide; produced by Ouchi Shinko Chemical Industrial Co., Ltd., Noxeller CZ-G
*71: Vulcanization accelerator: 1,3-diphenylguanidine; produced by Ouchi Shinko Chemical Industrial Co., Ltd., Noxeller D
*72: Sulfur; produced by Shanghai Jinghai Chemical Co., Ltd.

Examples 62 to 64 and Comparative Examples 19 to 20: Wet Grip Performance Test

The frictional resistance of the rubber compositions obtained in Examples 62 to 64 and Comparative Examples 19 to 20 below was measured with a BPST friction tester at room temperature by using abrasive paper #A200 soaked in water as a road surface. The wet grip performance index was calculated according to the following formula as an index relative to the obtained frictional resistance value of Comparative Example 19, which was defined as 100. A higher wet grip performance index value indicates better wet grip performance.

Formula: Wet grip performance index = (Frictional resistance of the rubber composition of one of Examples 62 to 64 or Comparative Example 20) / (Frictional resistance of Comparative Example 19) x 100

The results confirm that when the rubber compositions of the Examples are used, excellent wet grip performance can be obtained, as compared with the rubber composition of the Comparative Example. A comparison of Example 62 and Example 64 confirmed that the rubber composition of the present invention can provide better wet grip performance when no phenoric resin is used.

Examples 65 to 67 and Comparative Example 21: Production of Rubber Compositions

The components shown in step (A3) of Table 11 below were blended in the proportions (parts by weight) shown therein and mixed using a Banbury mixer. After the obtained mixture was allowed to rest until the temperature of the mixture was reduced to 60°C or less, the components shown in step (B3) of Table 11 were added in the proportions (parts by weight) shown therein and mixed while controlling the maximum temperature of the mixture to not more than 70°C, thus producing a rubber composition.

Table 11

| | | Examples | | | Comparative Examples |
|---|---|---|---|---|---|
| | | 65 | 66 | 67 | 21 |
| Step A3 | NR*73 | 100 | 100 | 100 | 100 |
| | Carbon black*74 | 55 | 55 | 55 | 55 |
| | Antioxidant *75 | 2 | 2 | 2 | 2 |
| | Wax*76 | 1.5 | 1. 5 | 1.5 | 1.5 |
| | Zinc oxide*77 | 3 | 3 | 3 | 3 |
| | Stearic acid*78 | 2 | 2 | 2 | 2 |
| | Compound (b) -1*79 | 0.6 | 0.6 | 0.6 | |
| Step B3 | Vulcanization accelerator*80 | 2 | 2 | 2 | 2 |
| | Sulfur*81 | 1.2 | 0.5 | 4 | 1.2 |
| Wet grip performance index | | 113 | 108 | 91 | 100 |
| Durability index | | 121 | 93 | 88 | 100 |

The details of each component in Table 11 are as follows.
*73: NR (natural rubber); produced by Guangken Rubber Co., Ltd., TSR-20
*74: CB (carbon black); produced by Cabot Co., Ltd., N234
*75: Antioxidant (N-phenyl-N'-(1,3-dimethylbutyl)-p-phenylenediamine); produced by Kemai Chemical Co., Ltd.
*76: Wax; produced by Rhein Chemie Rheinau GmbH, Antilux 111
*77: Zinc oxide; produced by Dalian Zinc Oxide Co., Ltd.
*78: Stearic acid; produced by Sichuan Tianyu Grease Chemical Co., Ltd.
*79: Compound (b-1); produced by Otsuka Chemical Co., Ltd., 3-methyl-5-pyrazolone
*80: Vulcanization accelerator: N-cyclohexyl-2-benzothiazolylsulfeneamide; produced by Ouchi Shinko Chemical Industrial Co., Ltd., Noxeller CZ-G
*81: Sulfur; produced by Shanghai Jinghai Chemical Co., Ltd.

Examples 65 to 67 and Comparative Example 21: Wet Grip Performance Test

The frictional resistance of the rubber compositions obtained in Examples 65 to 67 and Comparative Example 21 below was measured with a BPST friction tester at room temperature by using abrasive paper #A200 soaked in water as a road surface. The wet grip performance index was calculated according to the following formula as an index relative to the obtained frictional resistance value of Comparative Example 22, which was defined as 100. A higher value of the wet grip performance index indicates a better wet grip performance.

Formula: Wet grip performance index = (Frictional resistance of the rubber composition of each of Examples 65 to 67) / (Frictional resistance of Comparative Example 21) x 100

Examples 65 to 67 and Comparative Example 21: Durability Test

The tensile strength of the rubber compositions of Example **64 and Comparative Examples 22 to 24 below was measured using unnicked angle test pieces in accordance with JIS 6252. The durability index was calculated according to the following formula as an index relative to the obtained tensile strength value of Comparative Example 22,** which was defined as 100. A higher durability index value indicates a better durability.

Formula: Durability index= (Tensile strength of one of rubber compositions of Examples 65 to 67 or Comparative Example 21) / (Tensile strength of Comparative Example 21) x 100

As shown in Table 11, the compound of Example 65 containing 0.8 to 2.2 parts by mass of sulfur based on 100 parts by mass of the rubber component was confirmed to have excellent wet grip performance and durability.

Example 68 and Comparative Examples 22 to 26: Production of First Rubber Composition and Second Rubber Composition

The components shown in step (A4) of Table 12 below were blended in the proportions (parts by weight) shown therein and mixed using a Banbury mixer to produce a first rubber composition. After the obtained mixture was allowed to rest until the temperature of the mixture was reduced to 60°C or less, the components shown in step (B4) of Table 12 below were added in the proportions (parts by weight) shown therein and mixed to produce a second rubber composition. The composition was further mixed while controlling the maximum temperature of the mixture to not more than 70°C.

Table 12

| Components of the rubber composition | | Examples | Comparative Examples | | | | |
|---|---|---|---|---|---|---|---|
| | | 68 | 22 | 23 | 24 | 25 | 26 |
| Step (A4) (first rubber composition) | Natural rubber*82 | 100 | 100 | 100 | 100 | 100 | 100 |
| | Compound (b) -1*83 | 1 | | | 1 | | |
| | Antioxidant *84 | 2 | 2 | | | 2 | |
| | Carbon black*85 | 50 | 50 | 50 | 50 | 50 | 50 |
| | Wax*86 | 1.5 | 1.5 | 1.5 | 1.5 | 1.5 | 1.5 |
| | Stearic acid*87 | 2 | 2 | 2 | 2 | 2 | 2 |
| | Zinc oxide*88 | 3 | 3 | 3 | 3 | 3 | 3 |
| Step (B4) (second rubber composi tion) | vulcanization accelerator*89 | 1.2 | 1.2 | 1.2 | 1.2 | 1.2 | 1.2 |
| | Vulcanizing agent (sulfur) *90 | 2 | 2 | 2 | 2 | 2 | 2 |
| | Compound (b) -1*83 | | | | | 1 | 1 |
| | Antioxidant *84 | | | 2 | 2 | | 2 |
| Tear strength index | | 134 | 100 | 100 | 109 | 105 | 106 |
| Scorch time index | | 125 | 100 | 113 | 64 | 80 | 84 |

The details of each component in Table 12 are as follows.
*82: NR (natural rubher) ; produced by Guangken Rubber Co., Ltd., TS R-20
*83: Compound (b) ; produced by Otsuka Chemical Co., Ltd., 3-methyl-5-pyrazolone
*84: Antioxidant; N-phenyl-N'-(1,3-dimethylbutyl)-p-phenylene diamine, produced by Ouchi Shinko Chemical Industrial Co., Ltd., Nocrac 6C
*85: Carbon black; produced by Cabot Co., Ltd., N234
*86: Wax; produced by Ouchi Shinko Chemical Industrial Co., Ltd., Sunknock
*87: Stearic acid; produced by Sichuan Tianyu Grease Chemical Co., Ltd.
*88: Zinc oxide; produced by Dalian Zinc Oxide Co., Ltd.
*89: Vulcanization accelerator; N-(tert-butyl)-2-benzothiazole sulfenamide, produced by Sanshin Chemical Industry Co., Ltd., Sanceler NS-G
*90: Vulcanizing agent (sulfur); Shanghai Jimghai kemikaru Co., Ltd.

Example 68 and Comparative Examples 22 to 26: Tear Strength Performance Test

The tear strength of the rubber compositions obtained in Example 68 and Comparative Examples 22 to 26 was determined in accordance with JIS K6252 by using crescent-shaped test pieces at room temperature and at a tensile speed of 500 mm/min. The tear strength was calculated according to the following formula as an index relative to the obtained value of Comparative Example 22, which was defined as 100. A higher tear strength value indicates a better teat strength.

Formula: Tear strength performance index = (Tear strength of the rubber composition of Examples 68 or one of Comparative Examples 23 to 26) / (Tear strength of Comparative Example 22) x 100

Example 68 and Comparative Examples 23-26: Scorch Time Test

The scorch time of the rubber compositions of Example 68 and Comparative Examples 23 to 26 was measured according to JIS K 6300. The scorch time index was calculated according to the following formula as an index relative to the obtained value of Comparative Example 22, which was defined as 100. A higher scorch time index value indicates a better workability.

Formula: Scorch time index = (Scorch time of the rubber composition of Examples 68 or one of Comparative Examples 23 to 26)/(Scorch time of Comparative Example 22) x 100

Example 69 and Comparative Example 27: Production of Vibration-proof Rubber Compositions

The components shown in step (A5) of Table 13 below were mixed in the proportions (parts by weight) shown therein and kneaded using a Banbury mixer for 3 minutes while adjusting the number of rotations of the mixer so that the maximum temperature of the mixture was 160°C. After the obtained mixture was allowed to rest until the temperature of the mixture was reduced to 60°C or less, the components shown in step (B5) of Table 13 below were added in the proportions (parts by weight) shown therein and kneaded while controlling the maximum temperature of the mixture to not more than 110°C, thus producing each rubber composition.

Table 13

| | | | | Examples 69 | Comparative Example 27 |
|---|---|---|---|---|---|
| Components of the rubber composition | Step (A5) | Step (A5-1) | NR*91 | 100 | 100 |
| | | | Pyrazolone compound *92 | 1 | |
| | | Step (A5-2) | Carbon black*93 | 40 | 40 |
| | | | Antioxidant*94 | 2 | 2 |
| | | | Antioxidant*95 | 1.5 | 1.5 |
| | | | Wax*96 | 2 | 2 |
| | | | Zinc oxide*97 | 5 | 5 |
| | | | Stearic acid*98 | 1 | 1 |
| | Step (B5) | | Cross-linking accelerator*99 | 1 | 1 |
| | | | Crosslinking agent *100 | 1.88 | 1.88 |

(continued)

| | Examples 69 | Comparative Example 27 |
|---|---|---|
| Tensile strength index | 111 | 100 |
| Low heat build-up index | 98 | 100 |

Description of Symbols in Tables
The starting materials used in Table 13 are as follows.
*91: NR (natural rubber); produced by Guangken Rubber Co., Ltd., SVR3L
*92: Pyrazolone compound; produced by Otsuka Chemical Co., Ltd., 3-methyl-5-pyrazolone
*93: CB (carbon black); produced by Cabot Co., Ltd., N550
*94: Antioxidant, produced by Kemai Chemical Co., Ltd., N-phenyl-N'-(1,3-dimethylbutyl)-p-phenylenediamine
*95: Antioxidant; produced by Ouchi Shinko Kagaku Kogyo Co., Ltd., 2,2,4-trimethyl-1,2-dihydroquinoline polymer, produced by manufactured by Ouchi Shinko Chemical Industrial Co., Ltd.
*96: Wax; produced by Rhein Chemie Rheinau GmbH, Antilux 111
*97: Zinc oxide; produced by Dalian Zinc Oxide Co., Ltd.
*98: Stearic acid; produced by Sichuan Tianyu Grease Chemical Co., Ltd.
*99: Vulcanization accelerator; produced by Sanshin Chemical Industry Co., Ltd., N-(tert-butyl)-2-benzothiazole sulfenamide
*100: Sulfur; produced by Shanghai Jinghai Chemical Co., Ltd.

Example 69 and Comparative Example 27: Durability (Tear Strength Index) Test

The tear strength index of the rubber compositions obtained in Example 69 and Comparative Example 27 was determined in accordance with JIS K6252 using crescent-shaped test pieces at room temperature and at a tensile speed of 500 mm/min. The tear strength index was calculated according to the following formula as an index relative to the obtained tear strength value of Comparative Example 30, which was defined as 100. Table 13 shows the results. A higher tear strength value indicates a better tear strength.

Formula: Tear strength index = {(Tear strength of rubber composition of Example 69) / (Tear strength of Comparative Example 27)) x 100

Low Heat Build-up (Tan δ Index) Test

The tan δ of rubber compositions obtained in Example 69 and Comparative Example 27 was measured using a viscoelasticity measuring instrument (produced by Metravib) at a temperature of 40°C, a dynamic strain of 5%, and a frequency of 15 Hz. For comparison, the rubber composition (Comparative Example 27) was prepared using the same components and the same production method as in Example 69 except that no compound was added. The tan δ of the composition of Comparative Example 27 was defined as 100. The low heat build-up index was calculated according to the following formula. Table 13 shows the results. A smaller low heat build-up index value indicates smaller hysteresis loss and a greater vibration-proof effect.

Formula: Low heat build-up index = (Tan δ of the rubber composition of Example 69) / (Tan δ of Comparative Example 27) x 100

Production Example 17: Production of Rubber Material by Kneading

The rubber component and the compound shown in Table 14 were mixed in the proportions (parts by mass) shown therein and kneaded using a Banbury mixer. When the temperature of the mixture had reached 150°C, the mixture was kneaded for about 1 minute while maintaining the temperature by adjustment. The resulting mixture was then cooled

on a roll mill to produce a rubber material A.

The tan δ of the rubber compositions obtained in Example 70, Comparative Example 28, and Reference Example 1 shown in Table 15 below was measured using a viscoelasticity measuring instrument (produced by Metravib) at a temperature of 25°C, a dynamic strain of 5%, and a frequency of 15 Hz. The reciprocal of tan δ of Comparative Example 28 was defined as 100. The low heat build-up index was calculated according to the following formula. A smaller low heat build-up index value indicates a better low heat build-up.

Formula: Low heat build-up index = (Tan δ of the rubber composition of Example 70, Comparative Example 28, or Reference Example 1) / (Tan δ of Reference Example 1) x 100

Mooney Viscosity Measurement (Processability)

The Mooney viscosity was measured in accordance with JIS K6300-1 (Determination of viscosity and scorch time according to Mooney viscometer; ML1+4, 100°C). The reciprocal of tan δ of Comparative Example 1 was defined as 100. The processability index was calculated according to the following formula. A higher processability index value indicates better processability.

Formula: Processability index = (Mooney viscosity of Reference Example 1) / (Mooney viscosity of the rubber composition of Example 70, Comparative Example 28, or Reference Example 1) x 100

Table 14

| | Production Example 17 |
|---|---|
| Natural rubber*101 | 100 |
| Compound A*102 | 0.35 |

Table 15

| | | Examples 70 | Comparative Examples 28 | Reference Example 1 |
|---|---|---|---|---|
| Step (1) | Rubber material A*103 | 100.35 | | |
| | Natural rubber*101 | | 100 | 100 |
| | Compound A*102 | | 0.35 | |
| | Silica*104 | 50 | 50 | 50 |
| | Silane coupling agent*105 | 4 | 4 | 4 |
| | Antioxidant *106 | 2 | 2 | 2 |
| | WaX*107 | 1.5 | 1. 5 | 1.5 |
| | Stearic acid*108 | 2 | 2 | 2 |
| | Zinc oxide*109 | 3 | 3 | 3 |

(continued)

| | | Examples 70 | Comparative Examples 28 | Reference Example 1 |
|---|---|---|---|---|
| Step (2) | Vulcanization accelerator A*110 | 1.2 | 1.2 | 1.2 |
| | Vulcanization accelerator B*111 | 1 | 1 | 1 |
| | Sulfur*112 | 2 | 2 | 2 |
| | Low heat build-up index | 72 | 84 | 100 |
| | Processability index | 95 | 83 | 100 |

*101: Natural rubber; produced by Chupan Rubber Company Limited, RSS3
*102: Compound A; produced by Otsuka Chemical Co., Ltd., 3-methyl-5-pyrazolone
*103: Rubber material A; natural rubber master batch produced in Production Example 1
*104: Silica; produced by QueChen Silicon Chemical Co., Ltd., HD165MP
*105: Silane coupling agent; produced by Evonik Industries, Si69
*106: Antioxidant; produced by Keimai Chemical Co., Ltd., 6-PPD
*107: Wax; produced by Rhein Chemie Rheinau GmbH, Antilux 111
*108: Stearic acid; produced by Sichuan Tianyu Grease Co., Ltd.
*109: Zinc oxide; produced by Dalian Zinc Oxide Co., Ltd.
*110: Vulcanization accelerator A; produced by Kemai Chemical Co., Ltd., CBS
*111: Vulcanization accelerator B; produced by Kemai Chemical Co., Ltd., DPG
*112: Sulfur; produced by Shanghai Jinghai Chemical Co., Ltd.

Industrial Applicability

The rubber composition of the present invention, which contains the compound (1) or compound (2), has improved dispersibility of carbon black and/or an inorganic filler and is excellent in terms of low heat build-up, wet grip performance, durability, and processability. Accordingly, the rubber composition of the present invention can be used as a member of various pneumatic tires for various automobiles, in particular, as a tread member for pneumatic radial-ply tires.

Further, due to its excellent durability, the rubber composition of the present invention can be used to produce vibration-proof rubber having excellent durability.

Further, incorporation of the additive for imparting tear strength to a rubber component according to the present invention, which contains the compound (1) or compound (2), into a rubber composition improves the dispersibility of carbon black and/or an inorganic filler, thus providing a rubber composition with excellent tear strength. Accordingly, a tire having excellent tear strength can be obtained by using this rubber composition having excellent tear strength.

## Claims

**1.** A rubber composition comprising the following components (a1), (b1), and (c1):

component (a1): a rubber component;
component (b1): a compound represented by the following formula (1) or (2), or a salt of the compound; and
component (cl): carbon black and/or an inorganic filler;

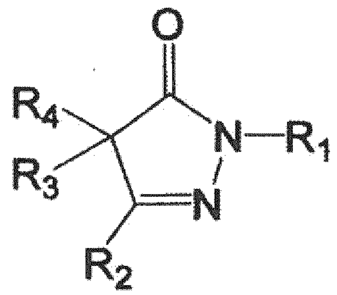

(1)

wherein
$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

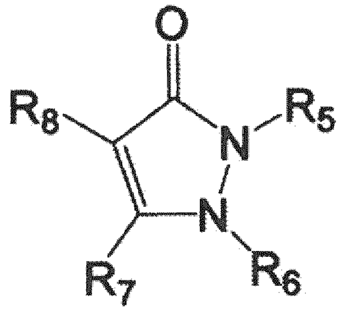
(2)

wherein
$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

**2.** The rubber composition according to claim 1, wherein the component (c1) is silica.

**3.** The rubber composition according to claim 2, further comprising a hydrocarbon polymer.

**4.** The rubber composition according to claim 1, which is used for tires, wherein the component (c1) is carbon black, and the rubber composition further comprises sulfur in an amount of 0.8 to 2.2 parts by mass, per 100 parts by mass of the rubber component.

**5.** The rubber composition according to claim 1, which is used for vibration-proof rubber, wherein the component (a1) is diene rubber, the component (c1) is carbon black, and the rubber composition further comprises a cross-linking agent.

**6.** The rubber composition for vibration-proof rubber according to claim 5, wherein the cross-linking agent is at least one cross-linking agent selected from the group consisting of sulfur cross-linking agents, sulfur compound cross-linking agents, quinoid cross-linking agents, and maleimide cross-linking agents.

**7.** A first rubber composition comprising:

a rubber component;
a compound represented by the following formula (1) or (2), or a salt of the compound;
an antioxidant; and
a vulcanizing agent in an amount of 0 to 1 part by mass, per 100 parts by mass of the rubber component;

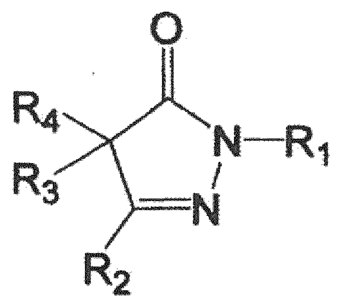
(1)

wherein
$R_1$, $R_2$, $R_3$, and $R_4$ are the same or different, and each represents a hydrogen atom, an alkyl group, an aralkyl group,

an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

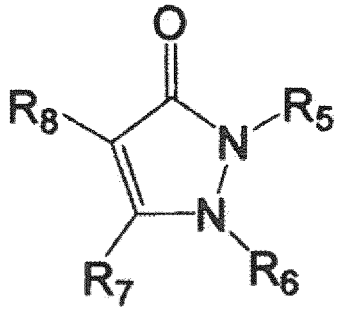

(2)

wherein

$R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

**8.** A second rubber composition obtained by adding a vulcanizing agent to the first rubber composition according to claim 7.

**9.** The rubber composition according to any one of claims 1 to 8, which is a compound in which in the formula (1), $R_1$, $R_3$, and $R_4$ all represent hydrogen atoms; and $R_2$ represents a hydrogen atom, a $C_{1-4}$ linear alkyl group, a phenyl group, a naphthyl group, or a furyl group.

**10.** A tire produced using the rubber composition according to any one of claims 1 to 4, 8, and 9.

**11.** Vibration-proof rubber produced using the rubber composition according to any one of claims 1 to 3, 5, 6, 8, and 9.

**12.** A hose produced using the rubber composition according to claims 1 to 3, 8, and 9.

**13.** A method for producing a rubber composition, comprising:

step (A1) of mixing raw material ingredients including a rubber component, a compound represented by the formula (1) or (2) or a salt of the compound, and carbon black and/or an inorganic filler; and
step (B1) of mixing the mixture obtained in step (A1) and a vulcanizing agent.

**14.** A method for producing a second rubber composition, comprising:

step (A4) of mixing the following components (a4), (b4), and
(c4); and
step (B4) of mixing the first rubber composition obtained in step (A4) and a vulcanizing agent:

(a4): a rubber component,
(b4): at least one compound selected from the group consisting of a compound represented by the formula (1), a compound represented by the formula (2), and salts of the compounds, and
(c4): an antixodiant;

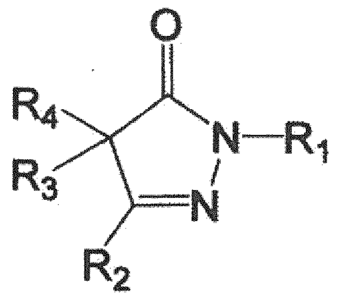

(1)

wherein

$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

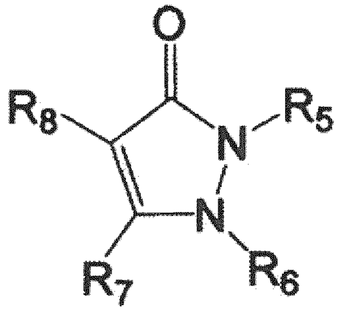

(2)

wherein

$R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

**15.** An additive for imparting low heat build-up to a rubber component, comprising a compound represented by the following formula (1) or (2), or a salt of the compound,

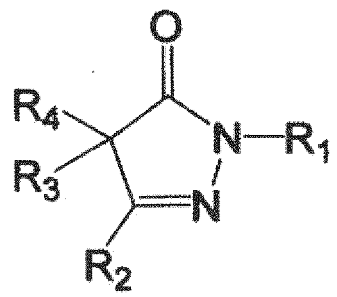

(1)

wherein $R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

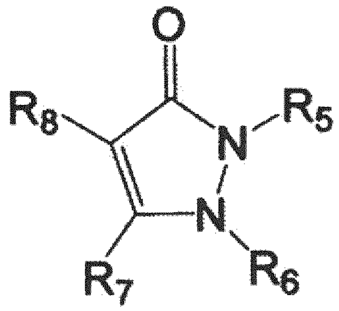

(2)

wherein $R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

**16.** A low heat build-up agent for rubber components, comprising a compound represented by the following formula (1) or (2), or a salt of the compound,

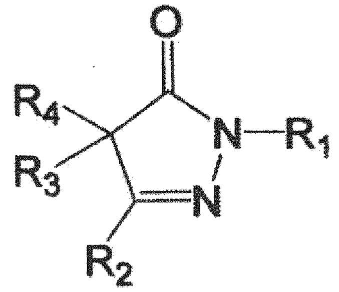

(1)

wherein
$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

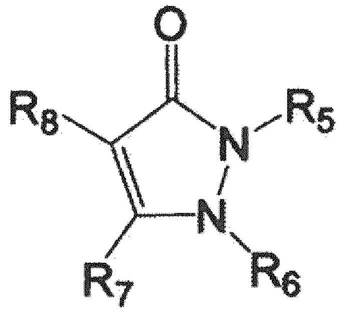

(2)

wherein
$R_3$, $R_7$, and $R_B$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

**17.** An additive for imparting tear strength to a rubber component, comprising a compound represented by the following formula (1) or (2), or a salt of the compound,

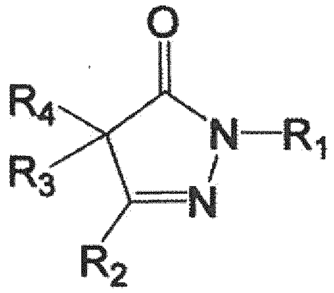 (1)

wherein

$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

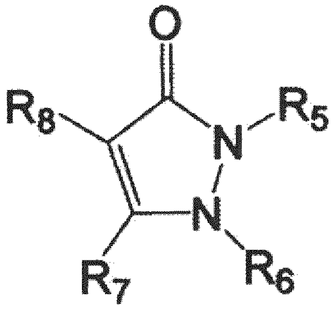 (2)

wherein

$R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

**18.** A tear strength imparting agent for rubber, comprising a compound represented by the following formula (1) or (2), or a salt of the compound,

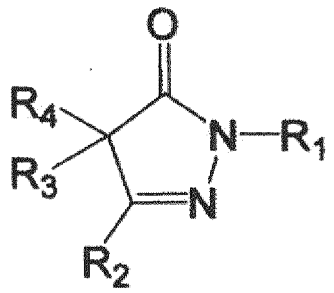 (1)

wherein

$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

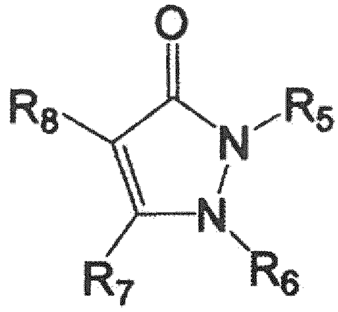

(2)

wherein

$R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

**19.** A rubber material obtained by incorporating, into a rubber component, at least one member selected from the group consisting of compounds represented by the following formulas (1) and (2), and salts of the compounds,

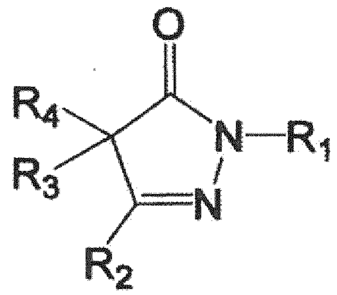

(1)

wherein

$R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

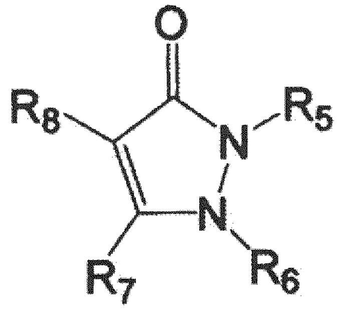

(2)

wherein $R_5$, $R_7$, and $R_8$ are the same or different, and each represents a hydrogen atom, an amino group, an alkyl group, an aralkyl group, an aryl group, or a heterocyclic group; $R_6$ represents an alkyl group, an aralkyl group, an aryl group, or a heterocyclic group; and each of these groups may have one or more substituents.

**20.** The material according to claim 18, which is a compound in which in the formula (1) , $R_1$, $R_3$, and $R_4$ are the same or different, and each represents a hydrogen atom, a $C_{1-4}$ linear or branched alkyl group, an aralkyl group, an aryl group, or a heterocyclic group; $R_2$ represents a hydrogen atom, a $C_{1-18}$ linear or branched alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; or

a compound in which in the formula (2), $R_5$ represents a hydrogen atom; $R_6$ represents a $C_{1-4}$ linear alkyl group,

an aralkyl group, or an aryl group; $R_7$ and $R_8$ are the same or different, and each represents a hydrogen atom, an amino group, an alkyl group, an aralkyl group, an aryl group, or a heterocyclic group; and each of these groups may have one or more substituents.

**21.** The material according to claim 18 or 19, wherein the rubber component is diene rubber.

**22.** The material according to claim 18 or 19, wherein the rubber component is natural rubber.

**23.** A master batch comprising the material according to any one of claims 18 to 21.

**24.** A rubber composition obtained by adding carbon black and/or an inorganic filler to the material according to any one of claims 18 to 21 or to the master batch according to claim 23.

**25.** The rubber composition according to claim 23, wherein the inorganic filler is silica.

**26.** A tire, a hose, vibration-proof rubber, or a belt produced using the rubber composition according to claim 23 or 24.

**27.** A method for producing a rubber material, comprising incorporating, into a rubber component, at least one member selected from the group consisting of compounds represented by the following formulas (1) and (2), and salts of the compounds,

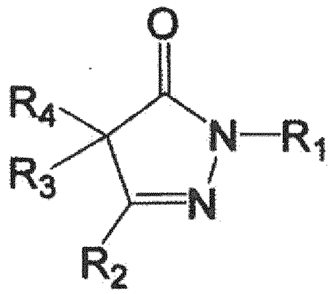

(1)

wherein $R_1$, $R_2$, $R_3$, and $R_4$ are the same or different, and each represents a hydrogen atom, an alkyl group, an aralkyl group, an aryl group, or a heterocyclic group; $R_3$ and $R_4$ may be taken together to form an alkylidene group, and any two of $R_2$, $R_3$, and $R_4$ may be taken together to form an alkylene group; and each of these groups may have one or more substituents; and

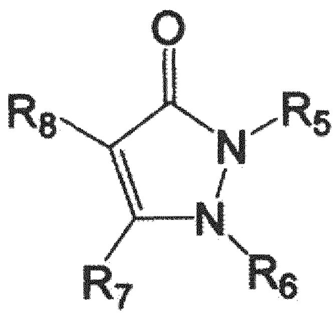

(2)

wherein $R_5$, $R_7$, and $R_8$ are the same or different, and each represents a hydrogen atom, an amino group, an alkyl group, an aralkyl group, an aryl group, or a heterocyclic group; and $R_6$ represents an alkyl group, an aralkyl group, an aryl group, or a heterocyclic group; and each of these groups may have one or more substituents.

## INTERNATIONAL SEARCH REPORT

International application No.
PCT/JP2019/033950

A. CLASSIFICATION OF SUBJECT MATTER
Int.Cl. C08L21/00(2006.01)i, B60C1/00(2006.01)i, C08K3/013(2018.01)i, C08K3/04(2006.01)i, C08K5/3445(2006.01)i, C08L9/00(2006.01)i
According to International Patent Classification (IPC) or to both national classification and IPC

B. FIELDS SEARCHED
Minimum documentation searched (classification system followed by classification symbols)
Int.Cl. C08L21/00, B60C1/00, C08K3/013, C08K3/04, C08K5/3445, C08L9/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2010-174127 A (SUMITOMO CHEMICAL CO., LTD.) 12 August 2010, claims 1, 2, 7, 8, paragraphs [0024], [0026], [0042], [0046], [0054], [0060] (Family: none) | 1-3, 5-9, 11-27<br>2-6, 10-12, 22, 25, 26 |
| X<br>Y | JP 2009-138046 A (SUMITOMO CHEMICAL CO., LTD.) 25 June 2009, claims 1, 4, 5, paragraphs [0028], [0029], [0038], [0043] & US 2009/0176916 A1, claims & CN 101451006 A | 1-3, 5-9, 11, 13-27<br>2-6, 10-12, 22, 25, 26 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>30 October 2019 (30.10.2019) | Date of mailing of the international search report<br>12 November 2019 (12.11.2019) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2019/033950

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | JP 2008-189911 A (BRIDGESTONE CORPORATION) 21 August 2008, claim 1, paragraphs [0017], [0019], [0022], examples 2, 4, 6 (Family: none) | 1, 4, 7-10, 13, 14, 19-24, 26, 27<br>2-6, 10-12, 22, 25, 26 |
| X<br><br>Y | JP 53-36539 A (DAINICHI-NIPPON CABLES, LTD.) 04 April 1978, claims, page 2, lower left column, line 16 to lower right column, line 15, page 3, upper left column, lines 12-14, examples 2-6 (Family: none) | 1, 7-9, 13-24, 27<br>2-6, 10-12, 22, 25, 26 |
| X<br><br>Y | JP 50-155549 A (DAINICHI-NIPPON CABLES, LTD.) 15 December 1975, claims, page 2, lower right column, lines 2-15, examples 17-21, 23, 24 (Family: none) | 1, 3, 7-9, 13-21, 23, 24, 27<br>2, 4-6, 10-12, 22, 25, 26 |
| X | US 6605658 B1 (GREAT LAKES CHEMICAL (EUROPE) GMBH) 12 August 2003, claims 1, 17, 18, column 3, line 35 to column 4, line 52, column 38, line 42 to column 39, line 4, column 40, lines 63-65, column 42, lines 50-51, column 49, lines 28-32 & EP 1110999 A2 & KR 10-2001-0060380 A | 1, 7, 9, 15-24, 27 |
| A | JP 49-24069 B1 (PLIVA) 20 June 1974, entire text & US 3736332 A, claims | 1-27 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015086318 A
- WO 2012031183 A
- JP 2018062629 A
- JP 2011052146 A
- JP 2017075245 A
- JP 2015028113 A
- JP 2015017160 A
- JP 2007131806 A